# EUROPEAN PATENT SPECIFICATION

(11) **EP 3 609 540 B1**
(45) Date of publication and mention of the grant of the patent: **11.01.2023**
(21) Application number: 18722830.9
(22) Date of filing: 16.04.2018
(51) Int. Cl.: A61K 47/55, A61K 47/68, A61P 35/00, C07K 16/28, C07K 16/30, C07K 16/32

(54) **IMMUNOCONJUGATE SYNTHESIS METHOD**
VERFAHREN ZUR IMMUNKONJUGATSYNTHESE
PROCÉDÉ DE SYNTHÈSE D'IMMUNOCONJUGUÉ

(30) Priority: 14.04.2017 US 201762485899 P; 28.06.2017 US 201762526347 P; 07.07.2017 US 201762530095 P; 07.07.2017 WO PCT/US2017/041268
(43) Date of publication of application: 19.02.2020
(73) Proprietor: Bolt Biotherapeutics, Inc., Redwood City, California 94063 (US)
(72) Inventor: JACKSON, David Y., Belmont, California 94002 (US); ALONSO, Michael N., Santa Clara, California 95051 (US); LEE, Arthur, San Jose, California 95128 (US); LAURA, Richard P., San Mateo, California 94403 (US)
(74) Representative: Huenges, Martin
(86) International application number: PCT/US2018/027771
(87) International publication number: WO 2018/191746

(56) References cited:
- WO-A1-2015/103987
- WO-A1-2015/103989
- WO-A1-2015/103990

## Description

Incorporated by reference in its entirety herein is a computer-readable nucleotide/amino acid sequence listing submitted concurrently herewith and identified as follows: One 803 Byte ASCII (Text) file named "SequenceListing.txt," created on April 16, 2018.

### BACKGROUND OF THE INVENTION

Cancer continues to be a major health concern. Unfortunately, the immune system is often not capable of controlling the growth and spread of cancer because the immune system cannot recognize the cancer cells as a target. Immune tolerance can be overcome by delivering neoantigens to dendritic cells via antibody-tumor immune complexes (Carmi et al., Nature, 521: 99-104 (2015)).

The simultaneous delivery of anti-tumor antibodies and adjuvants can be effective to treat tumors and to expand treatment options for cancer patients and other subjects. An effective way to simultaneously deliver tumor binding antibodies and immune adjuvants is by conjugating the antibodies and adjuvants to form immunoconjugates. There are several methods that can be used to produce such immunoconjugates.

Examples of methods that can be used to produce immunoconjugates are described in U.S. Patent No. 8,951,528. The synthesis methods described therein have many drawbacks. The synthesis methods are inefficient as they require several complex synthesis steps and substantial quantities of linker and adjuvants to create the immunoconjugates. The synthesis methods also have poor overall yields. In addition, the synthesis methods produce products that aggregate together. Most importantly, the immunoconjugates produced by the synthesis methods are less effective, e.g., less effectively activate myeloid cells for a given concentration.

Another method for preparing immunoconjugates involves modifying an antibody with an N-succinimidyl S-acetylthioacetate ("SATA") crosslinker. The SATA-modified antibody can then be conjugated to a modified adjuvant (for example, a succinimidyl 4-(N-maleimidomethyl)cyclohexane-1-carboxylate ["SMCC"] modified adjuvant which provides thiol-reactive maleimide groups) to form the immunoconjugates. While this method provides desirable immunoconjugates with satisfactory yields, this method has several drawbacks. The antibody itself must be modified before it can be conjugated, thereby requiring at least a two-step conjugation process. In the SATA method, the thiol groups must be added to antibodies' lysine side-chains. The addition of the thiol groups not only adds an additional step but also can decrease immunoconjugate stability and lead to undesirable aggregation of the immunoconjugates. Further, besides the desired immunoconjugates, this conjugation procedure provides the undesired "contaminants" (1) SATA-modified antibodies not linked to an adjuvant and (2) SMCC-modified adjuvants not linked to antibodies.

Thus, there remains a need for new methods for preparing immunoconjugates. The invention addresses this and other needs.

WO 2015/103989 A1 relates to compounds for targeted immunotherapy and the use of the compounds in the treatment of diseases such as cancer.

WO 2015/103990 A1 relates to compounds for targeted immunotherapy and the use of the compounds in the treatment of diseases such as EGFR expressing tumors.

WO 2015/103987 A1 relates to compounds for targeted immunotherapy and the use of the compounds in the treatment of diseases such as HER2 positive tumors.

### BRIEF SUMMARY OF THE INVENTION

The invention provides a method for producing an immunoconjugate of an adjuvant and an antibody. The method comprises combining one or more compounds of Formula I 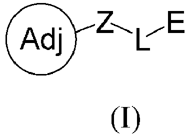 and an antibody of Formula II 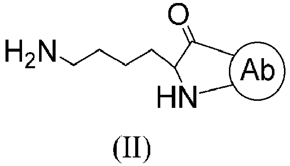 wherein Formula II is an antibody with residue 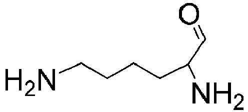 representing one or more lysine residues of the antibody, in an aqueous solution buffered at a pH of 7.5 to 9 until at least 33 mol% of the one or more compounds of Formula I is conjugated to the antibody of Formula II to provide the immunoconjugate of Formula III 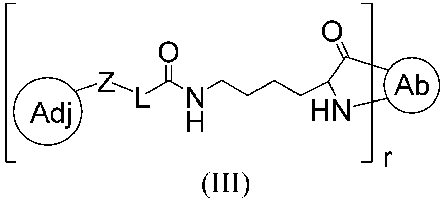 wherein Adj is an adjuvant, Z is -CH₂-, - C(O)NH-, -C(O)O-, or -C(O)-, L is a linker, E is an ester, and r is the average number of adjuvants attached to the antibody and is a positive number up to about 8, in a buffered aqueous solution.

### BRIEF DESCRIPTION OF THE SEVERAL VIEWS OF THE DRAWING(S)

Figure 1A shows a liquid chromatography-mass spectrometry analysis of immunoconjugate BB5 synthesized using the SATA method.
Figure 1B shows a liquid chromatography-mass spectrometry analysis of immunoconjugate BB5 synthesized using the ester method.
Figure 2A shows a size-exclusion chromatography analysis of immunoconjugate BB5 synthesized using the SATA method.
Figure 2B shows a size-exclusion chromatography analysis of immunoconjugate BB5 synthesized using the ester method.
Figure 3 shows a liquid chromatography-mass spectrometry analysis of immunoconjugate BB1 synthesized using the ester method.
Figure 4 shows a size-exclusion chromatography analysis of immunoconjugate BB1 synthesized using the ester method.
Figure 5 shows a liquid chromatography-mass spectrometry analysis of immunoconjugate BB4 synthesized using the ester method.
Figure 6 shows a size-exclusion chromatography analysis of immunoconjugate BB4 synthesized using the ester method.
Figure 7 shows a liquid chromatography-mass spectrometry analysis of immunoconjugate BB2 synthesized using the ester method.
Figure 8 shows a size-exclusion chromatography analysis of immunoconjugate BB2 synthesized using the ester method.
Figure 9A shows BB5 and BB2 synthesized using the ester method elicits myeloid activation as indicated by CD14 downregulation while the control does not. CD20 is the unconjugated monoclonal antibody used as a control.
Figure 9B shows BB5 and BB2 synthesized using the ester method elicits myeloid activation as indicated by CD16 downregulation while the control does not. CD20 is the unconjugated monoclonal antibody used as a control.
Figure 9C shows BB5 and BB2 synthesized using the ester method elicits myeloid activation as indicated by CD40 upregulation while the control does not. CD20 is the unconjugated monoclonal antibody used as a control.
Figure 9D shows BB5 and BB2 synthesized using the ester method elicits myeloid activation as indicated by CD86 upregulation while the control does not. CD20 is the unconjugated monoclonal antibody used as a control.
Figure 9E shows BB5 and BB2 synthesized using the ester method elicits myeloid activation as indicated by CD123 upregulation while the control does not. CD20 is the unconjugated monoclonal antibody used as a control.
Figure 9F shows BB5 and BB2 synthesized using the ester method elicits myeloid activation as indicated by Human Leukocyte Antigen-antigen D Related or "HLA-DR" while the control does not. CD20 is the unconjugated monoclonal antibody used as a control.
Figure 10A shows that BB5 elicits myeloid activation as indicated by CD14 downregulation while comparative IRM1 and IRM2 immunoconjugates do not. CD20 is the unconjugated monoclonal antibody used as a control.
Figure 10B shows that BB5 elicits myeloid activation as indicated by CD16 downregulation while comparative IRM1 and IRM2 immunoconjugates do not. CD20 is the unconjugated monoclonal antibody used as a control.
Figure 10C shows that BB5 elicits myeloid activation as indicated by CD40 upregulation while comparative IRM1 and IRM2 immunoconjugates do not. CD20 is the unconjugated monoclonal antibody used as a control.
Figure 10D shows that BB5 elicits myeloid activation as indicated by CD86 upregulation while comparative IRM1 and IRM2 immunoconjugates do not. CD20 is the unconjugated monoclonal antibody used as a control.
Figure 10E shows that BB5 elicits myeloid activation as indicated by CD123 upregulation while comparative IRM1 and IRM2 immunoconjugates do not. CD20 is the unconjugated monoclonal antibody used as a control.
Figure 10F shows that BB5 elicits myeloid activation as indicated by HLA-DR upregulation while comparative IRM1 and IRM2 immunoconjugates do not. CD20 is the unconjugated monoclonal antibody used as a control.
Figure 11A shows that BB5 elicits cytokine secretion (IL-1β) while comparative IRM1 and IRM2 immunoconjugates do not. CD20 is the unconjugated monoclonal antibody used as a control.
Figure 11B shows that BB5 elicits cytokine secretion (IL-6) while comparative IRM1 and IRM2 immunoconjugates do not. CD20 is the unconjugated monoclonal antibody used as a control.
Figure 11C shows that BB5 elicits cytokine secretion (TNFα) while comparative IRM1 and IRM2 immunoconjugates do not. CD20 is the unconjugated monoclonal antibody used as a control.
Figure 12A shows a size-exclusion chromatography analysis of immunoconjugate BB6 synthesized using the ester method.
Figure 12B shows a liquid chromatography-mass spectrometry analysis of immunoconjugate BB6 synthesized using the ester method.
Figure 13A shows a size-exclusion chromatography analysis of immunoconjugate BB7 synthesized using the ester method.
Figure 13B shows a liquid chromatography-mass spectrometry analysis of immunoconjugate BB7 synthesized using the ester method.
Figure 14A shows a size-exclusion chromatography analysis of immunoconjugate BB8 synthesized using the ester method.
Figure 14B shows a liquid chromatography-mass spectrometry analysis of immunoconjugate BB8 synthesized using the ester method.
Figure 15A shows a size-exclusion chromatography analysis of Comparative Conjugate IRM1.
Figure 15B shows a size-exclusion chromatography analysis of Comparative Conjugate IRM2.
Figure 16A shows a liquid chromatography-mass spectrometry analysis of IRM1 conjugate following overnight deglycosylation with PNGase F.
Figure 16B show a liquid chromatography-mass spectrometry analysis of BB5 conjugate following overnight deglycosylation with PNGase F.
Figure 17A shows a size-exclusion chromatography analysis of immunoconjugate BB9 synthesized using the ester method.
Figure 17B shows a liquid chromatography-mass spectrometry analysis of immunoconjugate BB9 synthesized using the ester method.
Figure 18A shows a size-exclusion chromatography analysis of immunoconjugate BB10 synthesized using the ester method.
Figure 18B shows a liquid chromatography-mass spectrometry analysis of immunoconjugate BB10 synthesized using the ester method.
Figure 19A shows a size-exclusion chromatography analysis of immunoconjugate BB11 synthesized using the ester method.
Figure 19B shows a liquid chromatography-mass spectrometry analysis of immunoconjugate BB11 synthesized using the ester method.
Figure 20A shows a size-exclusion chromatography analysis of immunoconjugate BB12 synthesized using the ester method.
Figure 20B shows a liquid chromatography-mass spectrometry analysis of immunoconjugate BB12 synthesized using the ester method.
Figure 21 shows a liquid chromatography-mass spectrometry analysis of immunoconjugate BB13 synthesized using the ester method.
Figure 22 shows a liquid chromatography-mass spectrometry analysis of immunoconjugate BB14 synthesized using the ester method.
Figure 23A shows that BB1 elicits myeloid activation as indicated by CD14 downregulation while the control does not. CD20 is the unconjugated monoclonal antibody used as a control.
Figure 23B shows that BB1 elicits myeloid activation as indicated by CD40 upregulation while the control does not. CD20 is the unconjugated monoclonal antibody used as a control.
Figure 23C shows that BB1 elicits myeloid activation as indicated by CD86 upregulation while the control does not. CD20 is the unconjugated monoclonal antibody used as a control.
Figure 23D shows that BB1 elicits myeloid activation as indicated by HLA-DR upregulation while the control does do not. CD20 is the unconjugated monoclonal antibody used as a control.
Figure 24A shows that BB4 elicits myeloid activation as indicated by CD14 downregulation while the control does not. CD20 is the unconjugated monoclonal antibody used as a control.
Figure 24B shows that BB4 elicits myeloid activation as indicated by CD40 upregulation while the control does not. CD20 is the unconjugated monoclonal antibody used as a control.
Figure 24C shows that BB4 elicits myeloid activation as indicated by CD86 upregulation while the control does not. CD20 is the unconjugated monoclonal antibody used as a control.
Figure 24D shows that BB7 elicits myeloid activation as indicated by HLA-DR upregulation while the control does do not. CD20 is the unconjugated monoclonal antibody used as a control.
Figure 25A shows that BB7 elicits myeloid activation as indicated by CD14 downregulation while the control does not. CD20 is the unconjugated monoclonal antibody used as a control.
Figure 25B shows that BB7 elicits myeloid activation as indicated by CD40 upregulation while the control does not. CD20 is the unconjugated monoclonal antibody used as a control.
Figure 25C shows that BB7 elicits myeloid activation as indicated by CD86 upregulation while the control does not. CD20 is the unconjugated monoclonal antibody used as a control.
Figure 25D shows that BB7 elicits myeloid activation as indicated by HLA-DR upregulation while the control does do not. CD20 is the unconjugated monoclonal antibody used as a control.
Figure 26A shows that BB9 elicits myeloid activation as indicated by CD14 downregulation while the control does not. CD20 is the unconjugated monoclonal antibody used as a control.
Figure 26B shows that BB9 elicits myeloid activation as indicated by CD40 upregulation while the control does not. CD20 is the unconjugated monoclonal antibody used as a control.
Figure 26C shows that BB9 elicits myeloid activation as indicated by CD86 upregulation while the control does not. CD20 is the unconjugated monoclonal antibody used as a control.
Figure 26D shows that BB9 elicits myeloid activation as indicated by HLA-DR upregulation while the control does do not. CD20 is the unconjugated monoclonal antibody used as a control.
Figure 27A shows that BB10 elicits myeloid activation as indicated by CD14 downregulation while the control does not. CD20 is the unconjugated monoclonal antibody used as a control.
Figure 27B shows that BB10 elicits myeloid activation as indicated by CD40 upregulation while the control does not. CD20 is the unconjugated monoclonal antibody used as a control.
Figure 27C shows that BB10 elicits myeloid activation as indicated by CD86 upregulation while the control does not. CD20 is the unconjugated monoclonal antibody used as a control.
Figure 27D shows that BB10 elicits myeloid activation as indicated by HLA-DR upregulation while the control does do not. CD20 is the unconjugated monoclonal antibody used as a control.
Figure 28 shows the drug to antibody ratio ("DAR") of an immunoconjugate synthesized using citrate buffered saline (pH 6.5) and incubated at 40 °C, as a function of time.
Figure 29 shows the DAR of an immunoconjugate synthesized using phosphate buffered saline (pH 7.4) and incubated at 40 °C, as a function of time.
Figure 30 shows the DAR of an immunoconjugate synthesized using borate buffered saline (pH 8.3) and incubated at 40 °C, as a function of time.
Figure 31 shows the free acid concentration of an immunoconjugate synthesized using citrate buffered saline (pH 6.5) and incubated at 40 °C, as a function of time.
Figure 32 shows the free acid concentration of an immunoconjugate synthesized using phosphate buffered saline (pH 7.4) and incubated at 40 °C, as a function of time.
Figure 33 shows the free acid concentration of an immunoconjugate synthesized using borate buffered saline (pH 8.3) and incubated at 40 °C, as a function of time.

### DETAILED DESCRIPTION OF THE INVENTION

The invention provides a method for producing an immunoconjugate of an adjuvant and an antibody. The method comprises combining one or more compounds of Formula I 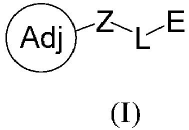 and an antibody of Formula II 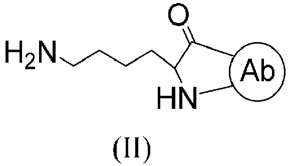 wherein Formula II is an antibody with residue 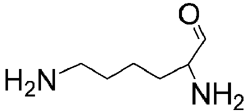 representing one or more lysine residues of the antibody, in an aqueous solution buffered at a pH of 7.5 to 9 until at least 33 mol% of the one or more compounds of Formula I is conjugated to the antibody of Formula II to provide the immunoconjugate of Formula III 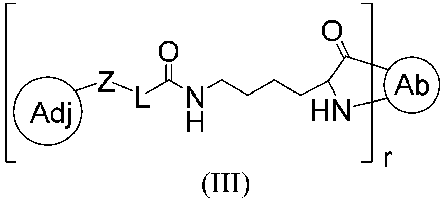 wherein Adj is an adjuvant, Z is -CH₂-, - C(O)NH-, -C(O)O-, or -C(O)-, L is a linker, E is an ester, and r is the average number of adjuvants attached to the antibody and is a positive number up to about 8, in a buffered aqueous solution.

The inventive production method provides for an improved synthesis of immunoconjugates by, for example, providing a simple one-step process for antibody-adjuvant conjugation. The streamlined method allows the modified adjuvant to conjugate directly to the lysine side chain of the antibody. The inventive production method can significantly increase the yield of the desired immunoconjugate (e.g., 80% yields compared to 50%-60% or less yields using previous methods). The inventive production method can decrease the amount of impurities that reduce the need for down-stream yield-reducing purification steps. The inventive production method can produce immunoconjugates that are less likely to aggregate and are more stable than immunoconjugates produced using previous methods. The inventive production method can allow for linkers to be used that retain more adjuvant activity following conjugation. The inventive method can provide an immunoconjugate with a desirable drug (adjuvant) to antibody ratio (DAR).

The present disclosure also provides particular immunoconjugates of an adjuvant and an antibody, including immunoconjugates prepared in accordance with the inventive production method, as well as compositions comprising such immunoconjugates. The present disclosure further provides an immunoconjugate according to the present disclosure for use in treating cancer.

### Definitions

As used herein, the term "immunoconjugate" refers to an antibody that is covalently bonded to an adjuvant moiety via a linker.

As used herein, the term "antibody" refers to a polypeptide comprising an antigen binding region (including the complementarity determining region (CDRs)) from an immunoglobulin gene or fragments thereof that specifically binds and recognizes an antigen. The recognized immunoglobulin genes include the kappa, lambda, alpha, gamma, delta, epsilon, and mu constant region genes, as well as numerous immunoglobulin variable region genes.

An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively. Light chains are classified as either kappa or lambda. Heavy chains are classified as gamma, mu, alpha, delta, or epsilon, which in turn define the immunoglobulin classes, IgG, IgM, IgA, IgD and IgE, respectively. IgG antibodies are large molecules of about 150 kDa composed of four peptide chains. IgG antibodies contain two identical class y heavy chains of about 50 kDa and two identical light chains of about 25 kDa, thus a tetrameric quaternary structure. The two heavy chains are linked to each other and to a light chain each by disulfide bonds. The resulting tetramer has two identical halves, which together form the Y-like shape. Each end of the fork contains an identical antigen binding site. There are four IgG subclasses (IgG1, 2, 3, and 4) in humans, named in order of their abundance in serum (IgG1 being the most abundant). Typically, the antigen-binding region of an antibody will be most critical in specificity and affinity of binding.

Antibodies exist, for example, as intact immunoglobulins or as a number of well-characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'2 dimer into a Fab' monomer. The Fab' monomer is essentially Fab with part of the hinge region (*see,* Fundamental Immunology (Paul ed., 7e ed. 2012). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries (see, e.g., McCafferty, et al., Nature, 348: 552-554 (1990)).

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity. "Antibody fragment," and all grammatical variants thereof, as used herein are defined as a portion of an intact antibody comprising the antigen binding site or variable region of the intact antibody, wherein the portion is free of the constant heavy chain domains (i.e. CH2, CH3, and CH4, depending on antibody isotype) of the Fc region of the intact antibody. Examples of antibody fragments include Fab, Fab', Fab'-SH, F(ab')₂, and Fv fragments; diabodies; camelid nanobodies (VHHs); any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv (scFv) molecules; (2) single chain polypeptides containing only one light chain variable domain, or a fragment thereof that contains the three CDRs of the light chain variable domain, without an associated heavy chain moiety; (3) single chain polypeptides containing only one heavy chain variable region, or a fragment thereof containing the three CDRs of the heavy chain variable region, without an associated light chain moiety; (4) nanobodies comprising single Ig domains from non-human species or other specific single-domain binding modules; and (5) multispecific or multivalent structures formed from antibody fragments. In an antibody fragment comprising one or more heavy chains, the heavy chain(s) can contain any constant domain sequence (e.g. CH1 in the IgG isotype) found in a non-Fc region of an intact antibody, and/or can contain any hinge region sequence found in an intact antibody, and/or can contain a leucine zipper sequence fused to or situated in the hinge region sequence or the constant domain sequence of the heavy chain(s).

As used herein, the term "epitope" means any antigenic determinant on an antigen to which the antigen-binding site, also referred to as the paratope, of an antibody binds. Epitopic determinants usually consist of chemically active surface groupings of molecules such as amino acids or sugar side chains and usually have specific three-dimensional structural characteristics, as well as specific charge characteristics.

The terms "polypeptide," "peptide," and "protein" are used interchangeably herein to refer to a polymer of amino acid residues. The terms also apply to amino acid polymers in which one or more amino acid residue is an artificial chemical mimetic of a corresponding naturally occurring amino acid, as well as to naturally occurring amino acid polymers and non-naturally occurring amino acid polymer.

As used herein, the term "adjuvant" refers to a substance capable of eliciting an immune response in a subject exposed to the adjuvant. The term "adjuvant moiety" refers to an adjuvant that is covalently bonded to an antibody as described herein. The adjuvant moiety can elicit the immune response while bonded to the antibody or after cleavage (e.g., enzymatic cleavage) from the antibody following administration of an immunoconjugate to the subject.

As used herein, the terms "Toll-like receptor" and "TLR" refer to any member of a family of highly-conserved mammalian proteins which recognize pathogen-associated molecular patterns and act as key signaling elements in innate immunity. TLR polypeptides share a characteristic structure that includes an extracellular domain that has leucine-rich repeats, a transmembrane domain, and an intracellular domain that is involved in TLR signaling.

The terms "Toll-like receptor 7" and "TLR7" refer to nucleic acids or polypeptides sharing at least 70%; at least 80%, at least 90%, at least 95%, at least 96%, at least 97%, at least 98%, at least 99%, or more sequence identity to a publicly-available TLR7 sequence, e.g., GenBank accession number AAZ99026 for human TLR7 polypeptide, or GenBank accession number AAK62676 for murine TLR7 polypeptide.

The terms "Toll-like receptor 8" and "TLR8" refer to nucleic acids or polypeptides sharing at least 70%; at least 80%, at least 90%, at least 95%, at least 96%, at least 97%, at least 98%, at least 99%, or more sequence identity to a publicly-available TLR7 sequence, e.g., GenBank accession number AAZ95441 for human TLR8 polypeptide, or GenBank accession number AAK62677 for murine TLR8 polypeptide.

A "TLR agonist" is a substance that binds, directly or indirectly, to a TLR (e.g., TLR7 and/or TLR8) to induce TLR signaling. Any detectable difference in TLR signaling can indicate that an agonist stimulates or activates a TLR. Signaling differences can be manifested, for example, as changes in the expression of target genes, in the phosphorylation of signal transduction components, in the intracellular localization of downstream elements such as NK-κB, in the association of certain components (such as IRAK) with other proteins or intracellular structures, or in the biochemical activity of components such as kinases (such as MAPK).

As used herein, the term "amino acid" refers to any monomeric unit that can be incorporated into a peptide, polypeptide, or protein. Amino acids include naturally-occurring α-amino acids and their stereoisomers, as well as unnatural (non-naturally occurring) amino acids and their stereoisomers. "Stereoisomers" of a given amino acid refer to isomers having the same molecular formula and intramolecular bonds but different three-dimensional arrangements of bonds and atoms (e.g., an L-amino acid and the corresponding D-amino acid).

Naturally-occurring amino acids are those encoded by the genetic code, as well as those amino acids that are later modified, e.g., hydroxyproline, γ-carboxyglutamate, and *O-*phosphoserine. Naturally-occurring α-amino acids include, without limitation, alanine (Ala), cysteine (Cys), aspartic acid (Asp), glutamic acid (Glu), phenylalanine (Phe), glycine (Gly), histidine (His), isoleucine (Ile), arginine (Arg), lysine (Lys), leucine (Leu), methionine (Met), asparagine (Asn), proline (Pro), glutamine (Gln), serine (Ser), threonine (Thr), valine (Val), tryptophan (Trp), tyrosine (Tyr), and combinations thereof. Stereoisomers of a naturally-occurring α-amino acids include, without limitation, D-alanine (D-Ala), D-cysteine (D-Cys), D-aspartic acid (D-Asp), D-glutamic acid (D-Glu), D-phenylalanine (D-Phe), D-histidine (D-His), D-isoleucine (D-Ile), D-arginine (D-Arg), D-lysine (D-Lys), D-leucine (D-Leu), D-methionine (D-Met), D-asparagine (D-Asn), D-proline (D-Pro), D-glutamine (D-Gln), D-serine (D-Ser), D-threonine (D-Thr), D-valine (D-Val), D-tryptophan (D-Trp), D-tyrosine (D-Tyr), and combinations thereof.

Unnatural (non-naturally occurring) amino acids include, without limitation, amino acid analogs, amino acid mimetics, synthetic amino acids, N-substituted glycines, and *N*-methyl amino acids in either the L- or D-configuration that function in a manner similar to the naturally-occurring amino acids. For example, "amino acid analogs" can be unnatural amino acids that have the same basic chemical structure as naturally-occurring amino acids (i.e., a carbon that is bonded to a hydrogen, a carboxyl group, an amino group) but have modified side-chain groups or modified peptide backbones, e.g., homoserine, norleucine, methionine sulfoxide, methionine methyl sulfonium. "Amino acid mimetics" refer to chemical compounds that have a structure that is different from the general chemical structure of an amino acid, but that functions in a manner similar to a naturally-occurring amino acid. Amino acids may be referred to herein by either the commonly known three letter symbols or by the one-letter symbols recommended by the IUPAC-IUB Biochemical Nomenclature Commission.

As used herein, the term "alkyl" refers to a straight or branched, saturated, aliphatic radical having the number of carbon atoms indicated. Alkyl can include any number of carbons, such as C₁₋₂, C₁₋₃, C₁₋₄, C₁₋₅, C₁₋₆, C₁₋₇, C₁₋₈, C₁₋₉, C₁₋₁₀, C₂₋₃, C₂₋₄, C₂₋₅, C₂₋₆, C₃₋₄, C₃₋₅, C₃₋₆, C₄₋₅, C₄₋₆ and C₅₋₆. For example, C₁₋₆ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 30 carbons atoms, such as, but not limited to heptyl, octyl, nonyl, decyl, etc. Alkyl groups can be substituted or unsubstituted. "Substituted alkyl" groups can be substituted with one or more groups selected from halo, hydroxy, amino, oxo (=O), alkylamino, amido, acyl, nitro, cyano, and alkoxy. The term "alkylene" refers to a divalent alkyl radical.

As used herein, the term "heteroalkyl" refers to an alkyl group as described herein, wherein one or more carbon atoms are optionally and independently replaced with heteroatom selected from N, O, and S. The term "heteroalkylene" refers to a divalent heteroalkyl radical.

As used herein, the term "carboalkyl" refers to a saturated or partially unsaturated, monocyclic, fused bicyclic, or bridged polycyclic ring assembly containing from 3 to 12 ring atoms, or the number of atoms indicated. Carboalkyl can include any number of carbons, such as C₃₋₆, C₄₋₆, C₅₋₆, C₃₋₈, C₄₋₈, C₅₋₈, C₆₋₈, C₃₋₉, C₃₋₁₀, C₃₋₁₁, and C₃₋₁₂. Saturated monocyclic carbocyclic rings include, for example, cyclopropyl, cyclobutyl, cyclopentyl, cyclohexyl, and cyclooctyl. Saturated bicyclic and polycyclic carbocyclic rings include, for example, norbornane, [2.2.2] bicyclooctane, decahydronaphthalene and adamantane. Carbocyclic groups can also be partially unsaturated, having one or more double or triple bonds in the ring. Representative carbocyclic groups that are partially unsaturated include, but are not limited to, cyclobutene, cyclopentene, cyclohexene, cyclohexadiene (1,3- and 1,4-isomers), cycloheptene, cycloheptadiene, cyclooctene, cyclooctadiene (1,3-, 1,4- and 1,5-isomers), norbornene, and norbornadiene.

Unsaturated carbocyclic groups also include aryl groups. The term "aryl" refers to an aromatic ring system having any suitable number of ring atoms and any suitable number of rings. Aryl groups can include any suitable number of ring atoms, such as, 6, 7, 8, 9, 10, 11, 12, 13, 14, 15 or 16 ring atoms, as well as from 6 to 10, 6 to 12, or 6 to 14 ring members. Aryl groups can be monocyclic, fused to form bicyclic or tricyclic groups, or linked by a bond to form a biaryl group. Representative aryl groups include phenyl, naphthyl and biphenyl. Other aryl groups include benzyl, having a methylene linking group. Some aryl groups have from 6 to 12 ring members, such as phenyl, naphthyl or biphenyl. Other aryl groups have from 6 to 10 ring members, such as phenyl or naphthyl.

A "divalent" carboalkyl refers to a carbocyclic group having two points of attachment for covalently linking two moieties in a molecule or material. Carboalkyls can be substituted or unsubstituted. "Substituted carboalkyl" groups can be substituted with one or more groups selected from halo, hydroxy, amino, alkylamino, amido, acyl, nitro, cyano, and alkoxy.

As used herein, the term "heterocycle" refers to heterocycloalkyl groups and heteroaryl groups. "Heteroaryl," by itself or as part of another substituent, refers to a monocyclic or fused bicyclic or tricyclic aromatic ring assembly containing 5 to 16 ring atoms, where from 1 to 5 of the ring atoms are a heteroatom such as N, O or S. Additional heteroatoms can also be useful, including, but not limited to, B, Al, Si and P. The heteroatoms can be oxidized to form moieties such as, but not limited to, -S(O)- and -S(O)₂-. Heteroaryl groups can include any number of ring atoms, such as 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heteroaryl groups, such as 1, 2, 3, 4, or 5, or 1 to 2, 1 to 3, 1 to 4, 1 to 5, 2 to 3, 2 to 4, 2 to 5, 3 to 4, or 3 to 5. The heteroaryl group can include groups such as pyrrole, pyridine, imidazole, pyrazole, triazole, tetrazole, pyrazine, pyrimidine, pyridazine, triazine (1,2,3-, 1,2,4- and 1,3,5-isomers), thiophene, furan, thiazole, isothiazole, oxazole, and isoxazole. The heteroaryl groups can also be fused to aromatic ring systems, such as a phenyl ring, to form members including, but not limited to, benzopyrroles such as indole and isoindole, benzopyridines such as quinoline and isoquinoline, benzopyrazine (quinoxaline), benzopyrimidine (quinazoline), benzopyridazines such as phthalazine and cinnoline, benzothiophene, and benzofuran. Other heteroaryl groups include heteroaryl rings linked by a bond, such as bipyridine. Heteroaryl groups can be substituted or unsubstituted. "Substituted heteroaryl" groups can be substituted with one or more groups selected from halo, hydroxy, amino, oxo (=O), alkylamino, amido, acyl, nitro, cyano, and alkoxy.

Heteroaryl groups can be linked via any position on the ring. For example, pyrrole includes 1-, 2- and 3-pyrrole, pyridine includes 2-, 3- and 4-pyridine, imidazole includes 1-, 2-, 4- and 5-imidazole, pyrazole includes 1-, 3-, 4- and 5-pyrazole, triazole includes 1-, 4- and 5-triazole, tetrazole includes 1- and 5-tetrazole, pyrimidine includes 2-, 4-, 5- and 6- pyrimidine, pyridazine includes 3- and 4-pyridazine, 1,2,3-triazine includes 4- and 5-triazine, 1,2,4-triazine includes 3-, 5- and 6-triazine, 1,3,5-triazine includes 2-triazine, thiophene includes 2- and 3-thiophene, furan includes 2- and 3-furan, thiazole includes 2-, 4- and 5-thiazole, isothiazole includes 3-, 4- and 5-isothiazole, oxazole includes 2-, 4- and 5-oxazole, isoxazole includes 3-, 4- and 5-isoxazole, indole includes 1-, 2- and 3-indole, isoindole includes 1- and 2-isoindole, quinoline includes 2-, 3- and 4-quinoline, isoquinoline includes 1-, 3- and 4-isoquinoline, quinazoline includes 2- and 4-quinoazoline, cinnoline includes 3- and 4-cinnoline, benzothiophene includes 2- and 3-benzothiophene, and benzofuran includes 2- and 3-benzofuran.

"Heterocycloalkyl," by itself or as part of another substituent, refers to a saturated ring system having from 3 to 12 ring members and from 1 to 4 heteroatoms of N, O and S. Additional heteroatoms can also be useful, including, but not limited to, B, Al, Si and P. The heteroatoms can be oxidized to form moieties such as, but not limited to, -S(O)- and -S(O)₂-. Heterocycloalkyl groups can include any number of ring atoms, such as, 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heterocycloalkyl groups, such as 1, 2, 3, or 4, or 1 to 2, 1 to 3, 1 to 4, 2 to 3, 2 to 4, or 3 to 4. The heterocycloalkyl group can include groups such as aziridine, azetidine, pyrrolidine, piperidine, azepane, azocane, quinuclidine, pyrazolidine, imidazolidine, piperazine (1,2-, 1,3- and 1,4-isomers), oxirane, oxetane, tetrahydrofuran, oxane (tetrahydropyran), oxepane, thiirane, thietane, thiolane (tetrahydrothiophene), thiane (tetrahydrothiopyran), oxazolidine, isoxazolidine, thiazolidine, isothiazolidine, dioxolane, dithiolane, morpholine, thiomorpholine, dioxane, or dithiane. The heterocycloalkyl groups can also be fused to aromatic or non-aromatic ring systems to form members including, but not limited to, indoline. Heterocycloalkyl groups can be unsubstituted or substituted. "Substituted heterocycloalkyl" groups can be substituted with one or more groups selected from halo, hydroxy, amino, oxo (=O), alkylamino, amido, acyl, nitro, cyano, and alkoxy.

Heterocycloalkyl groups can be linked via any position on the ring. For example, aziridine can be 1- or 2-aziridine, azetidine can be 1- or 2- azetidine, pyrrolidine can be 1-, 2- or 3-pyrrolidine, piperidine can be 1-, 2-, 3- or 4-piperidine, pyrazolidine can be 1-, 2-, 3-, or 4-pyrazolidine, imidazolidine can be 1-, 2-, 3- or 4-imidazolidine, piperazine can be 1-, 2-, 3- or 4-piperazine, tetrahydrofuran can be 1- or 2-tetrahydrofuran, oxazolidine can be 2-, 3-, 4- or 5-oxazolidine, isoxazolidine can be 2-, 3-, 4- or 5-isoxazolidine, thiazolidine can be 2-, 3-, 4- or 5-thiazolidine, isothiazolidine can be 2-, 3-, 4- or 5- isothiazolidine, and morpholine can be 2-, 3- or 4-morpholine.

As used herein, the terms "halo" and "halogen," by themselves or as part of another substituent, refer to a fluorine, chlorine, bromine, or iodine atom.

As used herein, the term "carbonyl," by itself or as part of another substituent, refers to -C(O)-, i.e., a carbon atom double-bonded to oxygen and bound to two other groups in the moiety having the carbonyl.

As used herein, the term "amino" refers to a moiety -NR₃, wherein each R group is H or alkyl. An amino moiety can be ionized to form the corresponding ammonium cation.

As used herein, the term "hydroxy" refers to the moiety -OH.

As used herein, the term "cyano" refers to a carbon atom triple-bonded to a nitrogen atom (i.e., the moiety -C≡N).

As used herein, the term "carboxy" refers to the moiety -C(O)OH. A carboxy moiety can be ionized to form the corresponding carboxylate anion.

As used herein, the term "amido" refers to a moiety -NRC(O)R or -C(O)NR₂, wherein each R group is H or alkyl.

As used herein, the term "nitro" refers to the moiety -NO₂.

As used herein, the term "oxo" refers to an oxygen atom that is double-bonded to a compound (i.e., O=).

As used herein, the symbol " 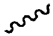 " defines the location at which the designated structure is bound (e.g., designates the location of the bond made between the adjuvant and Z, Z and the linker ("L"), or the linker and the ester ("E").

As used herein, when the term "optionally present" is used to refer to a chemical structure (e.g., "R"), if that chemical structure is not present, the bond originally made to the chemical structure is made directly to the adjacent atom.

As used herein, the term "linker" refers to a functional group that covalently bonds two or more moieties in a compound or material. For example, the linking moiety can serve to covalently bond an adjuvant moiety to an antibody in an immunoconjugate.

As used herein, the terms "treat," "treatment," and "treating" refer to any indicia of success in the treatment or amelioration of an injury, pathology, condition, or symptom (e.g., cognitive impairment), including any objective or subjective parameter such as abatement; remission; diminishing of symptoms or making the symptom, injury, pathology or condition more tolerable to the patient; reduction in the rate of symptom progression; decreasing the frequency or duration of the symptom or condition; or, in some situations, preventing the onset of the symptom. The treatment or amelioration of symptoms can be based on any objective or subjective parameter; including, e.g., the result of a physical examination.

The terms "cancer," "neoplasm," and "tumor" are used herein to refer to cells which exhibit autonomous, unregulated growth, such that they exhibit an aberrant growth phenotype characterized by a significant loss of control over cell proliferation. Cells of interest for detection, analysis, and/or treatment in the present disclosure include cancer cells (e.g., cancer cells from an individual with cancer), malignant cancer cells, pre-metastatic cancer cells, metastatic cancer cells, and non-metastatic cancer cells. Cancers of virtually every tissue are known. The phrase "cancer burden" refers to the quantum of cancer cells or cancer volume in a subject. Reducing cancer burden accordingly refers to reducing the number of cancer cells or the cancer volume in a subject. The term "cancer cell" as used herein refers to any cell that is a cancer cell (e.g., from any of the cancers for which an individual can be treated, e.g., isolated from an individual having cancer) or is derived from a cancer cell e.g. clone of a cancer cell. For example, a cancer cell can be from an established cancer cell line, can be a primary cell isolated from an individual with cancer, can be a progeny cell from a primary cell isolated from an individual with cancer, and the like. In some instances, the term can also refer to a portion of a cancer cell, such as a sub-cellular portion, a cell membrane portion, or a cell lysate of a cancer cell. Many types of cancers are known to those of skill in the art, including solid tumors such as carcinomas, sarcomas, glioblastomas, melanomas, lymphomas, myelomas, etc., and circulating cancers such as leukemias.

As used herein "cancer" includes any form of cancer, including but not limited to solid tumor cancers (e.g., lung, prostate, breast, bladder, colon, ovarian, pancreas, kidney, liver, glioblastoma, medulloblastoma, leiomyosarcoma, head & neck squamous cell carcinomas, melanomas, and neuroendocrine) and liquid cancers (e.g., hematological cancers); carcinomas; soft tissue tumors; sarcomas; teratomas; melanomas; leukemias; lymphomas; and brain cancers, including minimal residual disease, and including both primary and metastatic tumors. Any cancer is a suitable cancer to be treated by the subject methods and compositions.

Carcinomas are malignancies that originate in the epithelial tissues. Epithelial cells cover the external surface of the body, line the internal cavities, and form the lining of glandular tissues. Examples of carcinomas include, but are not limited to adenocarcinoma (cancer that begins in glandular (secretory) cells), e.g., cancers of the breast, pancreas, lung, prostate, and colon can be adenocarcinomas; adrenocortical carcinoma; hepatocellular carcinoma; renal cell carcinoma; ovarian carcinoma; carcinoma in situ; ductal carcinoma; carcinoma of the breast; basal cell carcinoma; squamous cell carcinoma; transitional cell carcinoma; colon carcinoma; nasopharyngeal carcinoma; multilocular cystic renal cell carcinoma; oat cell carcinoma; large cell lung carcinoma; small cell lung carcinoma; non-small cell lung carcinoma; and the like. Carcinomas may be found in prostrate, pancreas, colon, brain (usually as secondary metastases), lung, breast, and skin.

Soft tissue tumors are a highly diverse group of rare tumors that are derived from connective tissue. Examples of soft tissue tumors include, but are not limited to alveolar soft part sarcoma; angiomatoid fibrous histiocytoma; chondromyoxid fibroma; skeletal chondrosarcoma; extraskeletal myxoid chondrosarcoma; clear cell sarcoma; desmoplastic small round-cell tumor; dermatofibrosarcoma protuberans; endometrial stromal tumor; Ewing's sarcoma; fibromatosis (Desmoid); fibrosarcoma, infantile; gastrointestinal stromal tumor; bone giant cell tumor; tenosynovial giant cell tumor; inflammatory myofibroblastic tumor; uterine leiomyoma; leiomyosarcoma; lipoblastoma; typical lipoma; spindle cell or pleomorphic lipoma; atypical lipoma; chondroid lipoma; well-differentiated liposarcoma; myxoid/round cell liposarcoma; pleomorphic liposarcoma; myxoid malignant fibrous histiocytoma; high-grade malignant fibrous histiocytoma; myxofibrosarcoma; malignant peripheral nerve sheath tumor; mesothelioma; neuroblastoma; osteochondroma; osteosarcoma; primitive neuroectodermal tumor; alveolar rhabdomyosarcoma; embryonal rhabdomyosarcoma; benign or malignant schwannoma; synovial sarcoma; Evan's tumor; nodular fasciitis; desmoid-type fibromatosis; solitary fibrous tumor; dermatofibrosarcoma protuberans (DFSP); angiosarcoma; epithelioid hemangioendothelioma; tenosynovial giant cell tumor (TGCT); pigmented villonodular synovitis (PVNS); fibrous dysplasia; myxofibrosarcoma; fibrosarcoma; synovial sarcoma; malignant peripheral nerve sheath tumor; neurofibroma; and pleomorphic adenoma of soft tissue; and neoplasias derived from fibroblasts, myofibroblasts, histiocytes, vascular cells/endothelial cells and nerve sheath cells.

A sarcoma is a rare type of cancer that arises in cells of mesenchymal origin, e.g., in bone or in the soft tissues of the body, including cartilage, fat, muscle, blood vessels, fibrous tissue, or other connective or supportive tissue. Different types of sarcoma are based on where the cancer forms. For example, osteosarcoma forms in bone, liposarcoma forms in fat, and rhabdomyosarcoma forms in muscle. Examples of sarcomas include, but are not limited to askin's tumor; sarcoma botryoides; chondrosarcoma; ewing's sarcoma; malignant hemangioendothelioma; malignant schwannoma; osteosarcoma; and soft tissue sarcomas (e.g., alveolar soft part sarcoma; angiosarcoma; cystosarcoma phyllodesdermatofibrosarcoma protuberans (DFSP); desmoid tumor; desmoplastic small round cell tumor; epithelioid sarcoma; extraskeletal chondrosarcoma; extraskeletal osteosarcoma; fibrosarcoma; gastrointestinal stromal tumor (GIST); hemangiopericytoma; hemangiosarcoma (more commonly referred to as "angiosarcoma"); kaposi's sarcoma; leiomyosarcoma; liposarcoma; lymphangiosarcoma; malignant peripheral nerve sheath tumor (MPNST); neurofibrosarcoma; synovial sarcoma; and undifferentiated pleomorphic sarcoma).

A teratoma is a type of germ cell tumor that may contain several different types of tissue (e.g., can include tissues derived from any and/or all of the three germ layers: endoderm, mesoderm, and ectoderm), including for example, hair, muscle, and bone. Teratomas occur most often in the ovaries in women, the testicles in men, and the tailbone in children.

Melanoma is a form of cancer that begins in melanocytes (cells that make the pigment melanin). It may begin in a mole (skin melanoma), but can also begin in other pigmented tissues, such as in the eye or in the intestines.

Leukemias are cancers that start in blood-forming tissue, such as the bone marrow, and causes large numbers of abnormal blood cells to be produced and enter the bloodstream. For example, leukemias can originate in bone marrow-derived cells that normally mature in the bloodstream. Leukemias are named for how quickly the disease develops and progresses (e.g., acute versus chronic) and for the type of white blood cell that is affected (e.g., myeloid versus lymphoid). Myeloid leukemias are also called myelogenous or myeloblastic leukemias. Lymphoid leukemias are also called lymphoblastic or lymphocytic leukemia. Lymphoid leukemia cells may collect in the lymph nodes, which can become swollen. Examples of leukemias include, but are not limited to Acute myeloid leukemia (AML), Acute lymphoblastic leukemia (ALL), Chronic myeloid leukemia (CML), and Chronic lymphocytic leukemia (CLL).

Lymphomas are cancers that begin in cells of the immune system. For example, lymphomas can originate in bone marrow-derived cells that normally mature in the lymphatic system. There are two basic categories of lymphomas. One kind is Hodgkin lymphoma (HL), which is marked by the presence of a type of cell called the Reed-Sternberg cell. There are currently 6 recognized types of HL. Examples of Hodgkin lymphomas include: nodular sclerosis classical Hodgkin lymphoma (CHL), mixed cellularity CHL, lymphocyte-depletion CHL, lymphocyte-rich CHL, and nodular lymphocyte predominant HL.

The other category of lymphoma is non-Hodgkin lymphomas (NHL), which includes a large, diverse group of cancers of immune system cells. Non-Hodgkin lymphomas can be further divided into cancers that have an indolent (slow-growing) course and those that have an aggressive (fast-growing) course. There are currently 61 recognized types of NHL Examples of non-Hodgkin lymphomas include, but are not limited to AIDS-related Lymphomas, anaplastic large-cell lymphoma, angioimmunoblastic lymphoma, blastic NK-cell lymphoma, Burkitt's lymphoma, Burkitt-like lymphoma (small non-cleaved cell lymphoma), chronic lymphocytic leukemia/small lymphocytic lymphoma, cutaneous T-Cell lymphoma, diffuse large B-Cell lymphoma, enteropathy-type T-Cell lymphoma, follicular lymphoma, hepatosplenic gamma-delta T-Cell lymphomas, T-Cell leukemias, lymphoblastic lymphoma, mantle cell lymphoma, marginal zone lymphoma, nasal T-Cell lymphoma, pediatric lymphoma, peripheral T-Cell lymphomas, primary central nervous system lymphoma, transformed lymphomas, treatment-related T-Cell lymphomas, and Waldenstrom's macroglobulinemia.

Brain cancers include any cancer of the brain tissues. Examples of brain cancers include, but are not limited to gliomas (e.g., glioblastomas, astrocytomas, oligodendrogliomas, ependymomas, and the like), meningiomas, pituitary adenomas, and vestibular schwannomas, primitive neuroectodermal tumors (medulloblastomas).

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, and invasion of surrounding or distant tissues or organs, such as lymph nodes.

As used herein, the terms "cancer recurrence" and "tumor recurrence," and grammatical variants thereof, refer to further growth of neoplastic or cancerous cells after diagnosis of cancer. Particularly, recurrence may occur when further cancerous cell growth occurs in the cancerous tissue. "Tumor spread," similarly, occurs when the cells of a tumor disseminate into local or distant tissues and organs. Therefore, tumor spread encompasses tumor metastasis. "Tumor invasion" occurs when the tumor growth spread out locally to compromise the function of involved tissues by compression, destruction, or prevention of normal organ function.

As used herein, the term "metastasis" refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part that is not directly connected to the organ of the original cancerous tumor. Metastasis can also be defined as several steps of a process, such as the departure of cancer cells from an original tumor site, and migration and/or invasion of cancer cells to other parts of the body.

As used herein the terms "effective amount" and "therapeutically effective amount" refer to a dose of a substance such as an immunoconjugate that produces therapeutic effects for which it is administered. The exact dose will depend on the purpose of the treatment, and will be ascertainable by one skilled in the art using known techniques (*see,* e.g., Lieberman, Pharmaceutical Dosage Forms (vols. 1-3, 1992); Lloyd, The Art, Science and Technology of Pharmaceutical Compounding (1999); Pickar, Dosage Calculations (1999); Goodman & Gilman's The Pharmacological Basis of Therapeutics, 11th Edition, 2006, Brunton, Ed., McGraw-Hill; and Remington: The Science and Practice of Pharmacy, 21sf Edition, 2005, Hendrickson, Ed., Lippincott, Williams & Wilkins).

As used herein, the terms "recipient," "individual," "subject," "host," and "patient," are used interchangeably herein and refer to any mammalian subject for whom diagnosis, treatment, or therapy is desired (e.g., humans). "Mammal" for purposes of treatment refers to any animal classified as a mammal, including humans, domestic and farm animals, and zoo, sports, or pet animals, such as dogs, horses, cats, cows, sheep, goats, pigs, camels, etc. In certain embodiments, the mammal is human.

The term "synergistic adjuvant" or "synergistic combination" in the context of this invention includes the combination of two immune modulators such as a receptor agonist, cytokine, adjuvant polypeptide, that in combination elicit a synergistic effect on immunity relative to either administered alone. Particularly, this application discloses immunoconjugates that comprise synergistic combinations that comprise an adjuvant that is a TLR agonist and an antibody. These synergistic combinations upon administration together elicit a greater effect on immunity, e.g., relative to when the antibody or adjuvant are administered in the absence of the other moiety. Further, a decreased amount of the immunoconjugate may be administered (as measured by number of antibodies or number of adjuvants total administered as part of the immunoconjugate) compared to when either the antibody or adjuvant is administered alone.

As used herein, the term "administering" refers to parenteral, intravenous, intraperitoneal, intramuscular, intratumoral, intralesional, intranasal or subcutaneous administration, oral administration, administration as a suppository, topical contact, intrathecal administration, or the implantation of a slow-release device, e.g., a mini-osmotic pump, to the subject.

Immunoconjugates as described herein can provide an unexpectedly increased activation response of an antigen presenting cell ("APC"). This increased activation can be detected *in vitro* or *in vivo.* In some instances, increased APC activation can be detected in the form of a reduced time to achieve a specified level of APC activation. For example, in an *in vitro* assay, % APC activation can be achieved at an equivalent dose with an immunoconjugate within 1%, 10%, 20%, 30%, 40%, or 50% of the time required to receive the same or similar percentage of APC activation with a mixture of unconjugated antibody and TLR agonist. In some instances, an immunoconjugate can activate APCs (e.g., dendritic cells, and/or NK cells) in a reduced amount of time. For example, in some embodiments, an antibody TLR agonist mixture can activate APCs (e.g., dendritic cells, and/or NK cells) and/or induce dendritic cell differentiation after incubation with the mixture for 2, 3, 4, 5, 1-5, 2-5, 3-5, or 4-7 days; while, in contrast immunoconjugates described herein can activate and/or induce differentiation within 4 hours, 8 hours, 12 hours, 16 hours, or 1 day. Alternatively, the increased APC activation can be detected in the form of a reduced concentration of immunoconjugate required to achieve an amount (e.g., percent APCs), level (e.g., as measured by a level of upregulation of a suitable marker), or rate (e.g., as detected by a time of incubation required to activate) of APC activation.

The terms "about" and "around," as used herein to modify a numerical value, indicate a close range surrounding that explicit value. If "X" were the value, "about X" or "around X" would indicate a value from 0.9X to 1.1X, e.g., from 0.95X to 1.05X or from 0.99X to 1.01X. Any reference to "about X" or "around X" specifically indicates at least the values X, 0.95X, 0.96X, 0.97X, 0.98X, 0.99X, 1.01X, 1.02X, 1.03X, 1.04X, and 1.05X. Thus, "about X" and "around X" are intended to teach and provide written description support for a claim limitation of, e.g., "0.98X."

### Adjuvant

The adjuvant can be any suitable adjuvant. In some embodiments, the adjuvant is a compound that elicits an immune response. In some embodiments, the adjuvant moiety is a pattern recognition receptor ("PRR") agonist. Any adjuvant capable of activating a PRR can be installed in the immunoconjugates of the invention. As used herein, the terms "Pattern recognition receptor" and "PRR" refer to any member of a class of conserved mammalian proteins which recognize pathogen-associated molecular patterns ("PAMPs") or damage-associated molecular patterns ("DAMPs"), and act as key signaling elements in innate immunity. PRRs are divided into membrane-bound PRRs, cytoplasmic PRRs, and secreted PRRs. Examples of membrane-bound PRRs include Toll-like receptors ("TLRs") and C-type lectin receptors ("CLRs"). Examples of cytoplasmic PRRs include NOD-like receptors ("NLRs") and Rig-I-like receptors ("RLRs"). In some embodiments, the immunoconjugate can have more than one distinct PRR adjuvant moiety.

In certain embodiments, the adjuvant moiety in an immunoconjugate of the invention is a TLR agonist. Suitable TLR agonists include TLR1, TLR2, TLR3, TLR4, TLR5, TLR6, TLR7, TLR8, TLR9, TLR10, TLR11, or any combination thereof (e.g., TLR7/8 agonists). Any adjuvant capable of activating a TLR can be installed in the immunoconjugates of the invention. TLRs are type-I transmembrane proteins that are responsible for initiation of innate immune responses in vertebrates. TLRs recognize a variety of pathogen-associated molecular patterns from bacteria, viruses, and fungi and act as a first line of defense against invading pathogens. TLRs elicit overlapping yet distinct biological responses due to differences in cellular expression and in the signaling pathways that they initiate. Once engaged (e.g., by a natural stimulus or a synthetic TLR agonist) TLRs initiate a signal transduction cascade leading to activation of NF-κB via the adapter protein myeloid differentiation primary response gene 88 (MyD88) and recruitment of the IL-1 receptor associated kinase (IRAK). Phosphorylation of IRAK then leads to recruitment of TNF-receptor associated factor 6 (TRAF6), which results in the phosphorylation of the NF-κB inhibitor I-κB. As a result, NF-κB enters the cell nucleus and initiates transcription of genes whose promoters contain NF-κB binding sites, such as cytokines. Additional modes of regulation for TLR signaling include TIR-domain containing adapter-inducing interferon-β (TRIF)-dependent induction of TRAF6 and activation of MyD88 independent pathways via TRIF and TRAF3, leading to the phosphorylation of interferon response factor three (IRF3). Similarly, the MyD88 dependent pathway also activates several IRF family members, including IRF5 and IRF7 whereas the TRIF dependent pathway also activates the NF-κB pathway.

Examples of TLR3 agonists include Polyinosine-polycytidylic acid (poly (I:C)), Polyadenylic-polyuridylic acid (poly (A:U), and poly(I)-poly(C12U).

Examples of TLR4 agonists include Lipopolysaccharide (LPS) and Monophosphoryl lipid A (MPLA).

An example of a TLR5 agonist is Flagellin.

Examples of TLR9 agonists include single strand CpG oligodeoxynucleotides (CpG ODN). Three major classes of stimulatory CpG ODNs have been identified based on structural characteristics and activity on human peripheral blood mononuclear cells (PBMCs), in particular B cells and plasmacytoid dendritic cells (pDCs). These three classes are Class A (Type D), Class B (Type K), and Class C.

Examples of Nod Like Receptor (NLR) agonists include acylated derivative of iE-DAP, D-gamma-Glu-mDAP, L-Ala-gamma-D-Glu-mDAP, Muramyldipeptide with a C18 fatty acid chain, Muramyldipeptide, muramyl tripeptide, and N-glycolylated muramyldipeptide.

Examples of RIG-I-Like receptor (RLR) agonists include 5'ppp-dsrna (5'-pppGCAUGCGACCUCUGUUUGA -3' (SEQ ID NO: 1): 3'-CGUACGCUGGAGACAAACU -5' (SEQ ID NO: 2)), and Poly(deoxyadenylic-deoxythymidylic) acid (Poly(dA:dT)).

Additional immune-stimulatory compounds, such as cytosolic DNA and unique bacterial nucleic acids called cyclic dinucleotides, can be recognized by stimulator of interferon genes ("STING"), which can act a cytosolic DNA sensor. ADU-S100 can be a STING agonist. Non-limiting examples of STING agonists include: Cyclic [G(2',5')pA(2',5')p] (2'2'-cGAMP), cyclic [G(2',5')pA(3',5')p] (2'3'-cGAMP), cyclic [G(3',5')pA(3',5')p] (3'3'-cGAMP), Cyclic di-adenylate monophosphate (c-di-AMP), 2',5'-3',5'-c-diAMP (2'3'-c-di-AMP), Cyclic di-guanylate monophosphate (c-di-GMP), 2',5'-3',5'-c-diGMP (2'3'-c-di-GMP), Cyclic di-inosine monophosphate (c-di-IMP), Cyclic diuridine monophosphate (c-di-UMP), KIN700, KIN1148, KIN600, KIN500, KIN100, KIN101, KIN400, KIN2000, or SB-9200 can be recognized.

Any adjuvant capable of activating TLR7 and/or TLR8 can be installed in the immunoconjugates of the invention. Examples of TLR7 agonists and TLR8 agonists are described, e.g., by Vacchelli, et al. (Oncolmmunology, 2: 8, e25238, DOI: 10.4161/onci.25238 (2013)) and Carson et al. (U.S. Patent Application Publication 2013/0165455). TLR7 and TLR8 are both expressed in monocytes and dendritic cells. In humans, TLR7 is also expressed in plasmacytoid dendritic cells (pDCs) and B cells. TLR8 is expressed mostly in cells of myeloid origin, i.e., monocytes, granulocytes, and myeloid dendritic cells. TLR7 and TLR8 are capable of detecting the presence of "foreign" single-stranded RNA within a cell, as a means to respond to viral invasion. Treatment of TLR8-expressing cells, with TLR8 agonists can result in production of high levels of IL-12, IFN-γ, IL-1, TNF-α, IL-6, and other inflammatory cytokines. Similarly, stimulation of TLR7-expressing cells, such as pDCs, with TLR7 agonists can result in production of high levels of IFN-α and other inflammatory cytokines. TLR7/TLR8 engagement and resulting cytokine production can activate dendritic cells and other antigen-presenting cells, driving diverse innate and acquired immune response mechanisms leading to tumor destruction.

Examples of TLR7, TLR8 or TLR7/8 agonists include but are not limited to Gardiquimod (1-(4-amino-2-ethylaminomethylimidazo[4,5-c]quinolin-1-yl)-2-methylpropan-2-ol), Imiquimod (R837) (agonist for TLR7), loxoribine (agonist for TLR7), IRM1 (1-(2-amino-2-methylpropyl)-2-(ethoxymethyl)-1H-imidazo-[4,5-c]quinolin-4-amine), IRM2 (2-methyl-1-[2-(3-pyridin-3-ylpropoxy)ethyl]-1H-imidazo[4,5-c]quinolin-4-amine) (agonist for TLR8), IRM3 (N-(2-[2-[4-amino-2-(2-methoxyethyl)-1H-imidazo[4,5-c]quinolin-1-yl]ethoxy]ethyl)-N-methylcyclohexanecarboxamide) (agonist for TLR8), CL097 (2-(ethoxymethyl)-1H-imidazo[4,5-c]quinolin-4-amine) (agonist for TLR7/8), CL307 (agonist for TLR7), CL264 (agonist for TLR7), Resiquimod (agonist for TLR7/8), 3M-052/MEDI9197 (agonist for TLR7/8), SD-101 (N-[(4S)-2,5-dioxo-4-imidazolidinyl]-urea) (agonist for TLR7/8), motolimod (2-amino-N,N-dipropyl-8-[4-(pyrrolidine-1-carbonyl)phenyl]-3H-1-benzazepine-4-carboxamide) (agonist for TLR8), CL075 (3M002, 2-propylthiazolo[4,5-c]quinolin-4-amine) (agonist for TLR7/8), and TL8-506 (3H-1-benzazepine-4-carboxylic acid, 2-amino-8-(3-cyanophenyl)-, ethyl ester) (agonist for TLR8).

Examples of TLR2 agonists include but are not limited to an agent comprising N-α-palmitoyl-*S*-[2,3-bis(palmitoyloxy)-(2*RS*)-propyl]-L-cysteine, palmitoyl-Cys((*RS*)-2,3-di(palmitoyloxy)-propyl) ("Pam3Cys"), e.g., Pam3Cys, Pam3Cys-Ser-(Lys)4 (also known as "Pam3Cys-SKKKK" and "Pam₃CSK₄"), Triacyl lipid A ("OM-174"), Lipoteichoic acid ("LTA"), peptidoglycan, and CL419 (S-(2,3-bis(palmitoyloxy)-(2RS)propyl)-(R)-cysteinyl spermine).

An example of a TLR2/6 agonist is Pam₂CSK₄ (S-[2,3-bis(palmitoyloxy)-(2RS)-propyl]-[R]-cysteinyl-[S]-seryl-[S]-lysyl-[S]-lysyl-[S]-lysyl-[S]-lysine x 3 CF3COOH).

Examples of TLR2/7 agonist include CL572 (S-(2-myristoyloxy ethyl)-(R)-cysteinyl 4-((6-amino-2-(butylamino)-8-hydroxy-9H-purin-9-yl)methyl) aniline), CL413 (S-(2,3-bis(palmitoyloxy)-(2RS)propyl)-(R)-cysteinyl-(S)-seryl-(S)-lysyl-(S)-lysyl-(S)-lysyl-(S)-lysyl 4-((6-amino-2-(butylamino)-8-hydroxy-9H-purin-9-yl)methyl)aniline), and CL401 (S-(2,3-bis(palmitoyloxy)-(2RS)propyl)-(R)-cysteinyl 4-((6-amino-2(butyl amino)-8-hydroxy-9H-purin-9-yl)methyl) aniline).

In some embodiments, the adjuvant ("Adj") is of formula: 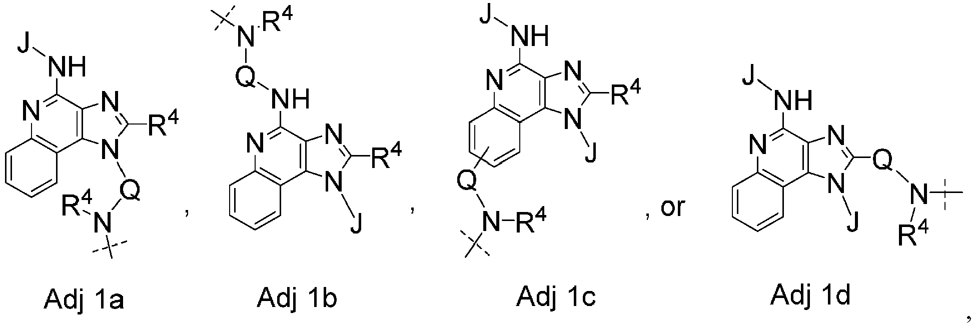 wherein each J independently is hydrogen, OR⁴, or R⁴; each R⁴ independently is hydrogen, or an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; Q is optionally present and is an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; and the dashed line (" 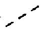 ") represents the point of attachment of the adjuvant. In certain embodiments, Q is present. In certain embodiments, the adjuvant ("Adj") is of formula: 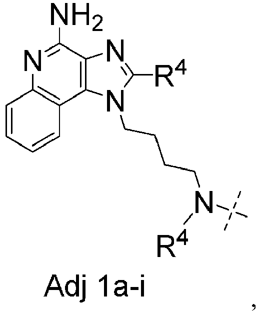 wherein each R⁴ independently is selected from the group consisting of hydrogen, or alkyl, heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units and the dashed line (" 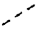 ") represents the point of attachment of the adjuvant.

In some embodiments, the adjuvant ("Adj") is of formula: 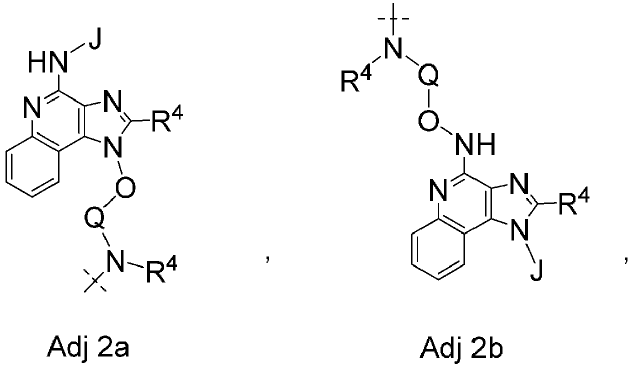 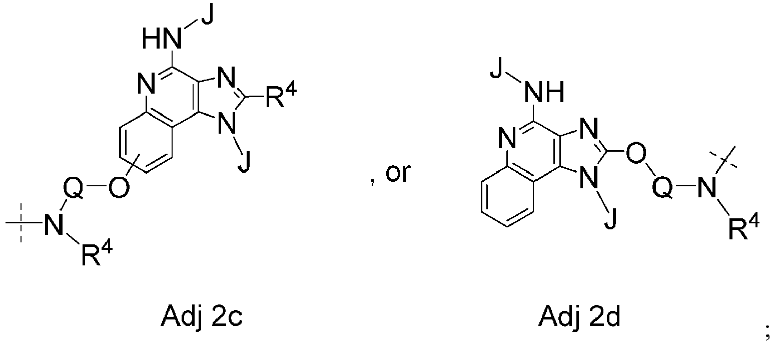 wherein J is hydrogen, OR⁴, or R⁴; each R⁴ independently is hydrogen, or alkyl, heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units; Q is selected from the group consisting of alkyl, or heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units; and the dashed line (" 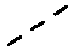 ") represents the point of attachment of the adjuvant. In certain embodiments, the adjuvant ("Adj") is of formula: 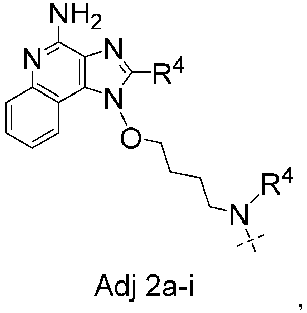 wherein each R⁴ independently is selected from the group consisting of hydrogen, or alkyl, heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units and the dashed line (" 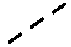 ") represents the point of attachment of the adjuvant.

In some embodiments, the adjuvant ("Adj") is of formula: 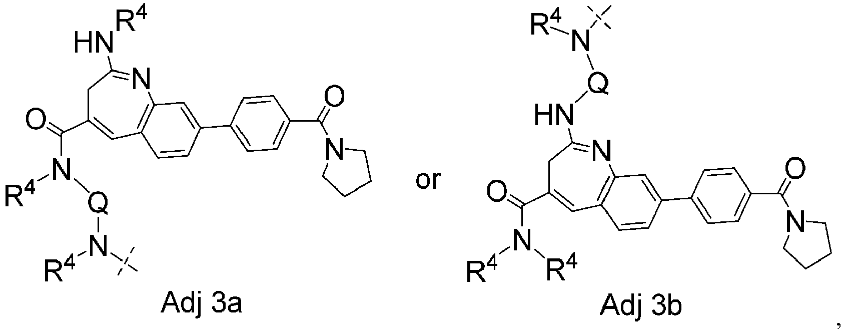 wherein each R⁴ independently is hydrogen, or alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; Q is alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; and the dashed line (" 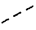 ") represents the point of attachment of the adjuvant.

In some embodiments, the adjuvant ("Adj") is of formula: 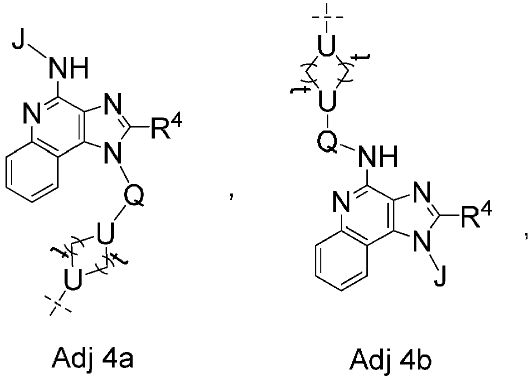 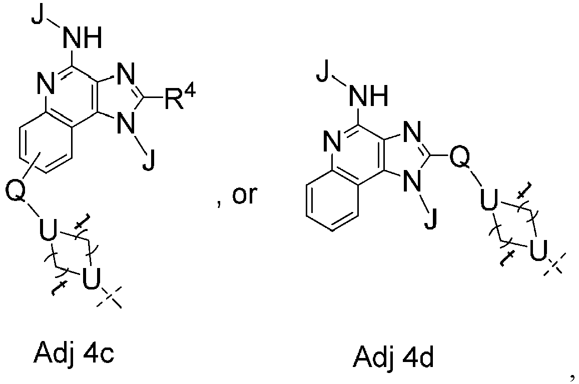 wherein each J independently is hydrogen, OR⁴, or R⁴; each R⁴ independently is hydrogen, or an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; each U independently is CH or N wherein at least one U is N; each subscript t independently is an integer from 1 to 3 (i.e., 1, 2, or 3); Q is optionally present and is an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; and the dashed line (" 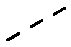 ") represents the point of attachment of the adjuvant. In certain embodiments, Q is present. In certain embodiments, the adjuvant ("Adj") is of formula: 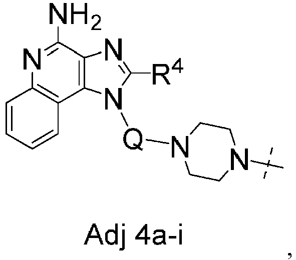 wherein R⁴ is selected from the group consisting of hydrogen, or alkyl, heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units Q is an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; and the dashed line (" 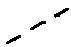 ") represents the point of attachment of the adjuvant.

In some embodiments, the adjuvant ("Adj") is of formula: 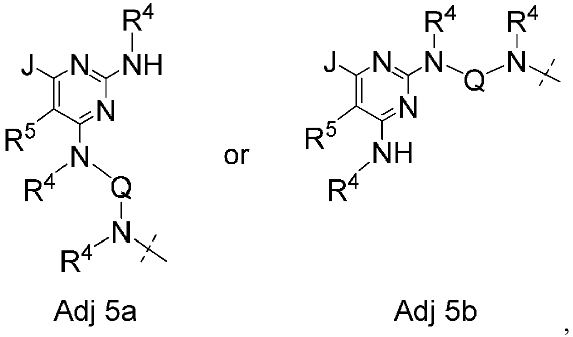 wherein J is hydrogen, OR⁴, or R⁴; each R⁴ independently is hydrogen, or an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; R³ is hydrogen, or an alkyl, heteroalkyl, cycloalkyl, heterocycloalkyl, aryl, heteroaryl, arylalkyl, or heteroarylalkyl group comprising from 1 to 10 (i.e., 1, 2, 3, 4, 5, 6, 7, 8, 9, or 10) carbon units; Q is an alkyl, heteroalkyl, cycloalkyl, heterocycloalkyl, aryl, heteroaryl, arylalkyl, or heteroarylalkyl group comprising from 1 to 8 (i.e., 1, 2, 3, 4, 5, 6, 7, or 8) carbon units; and the dashed line (" 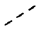 ") represents the point of attachment of the adjuvant. In certain embodiments, the adjuvant ("Adj") is of formula: 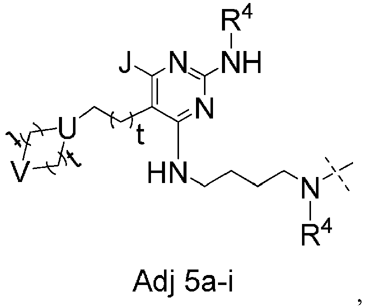 wherein J is hydrogen, OR⁴, or R⁴; each R⁴ independently is selected from the group consisting of hydrogen, or alkyl, heteroalkyl, cycloalkyl, heterocycloalkyl, aryl, heteroaryl, arylalkyl, and heteroarylalkyl group comprising from 1 to 8 (i.e., 1, 2, 3, 4, 5, 6, 7, or 8) carbon units; U is CH or N; V is CH₂, O, or NH; each subscript t independently is an integer from 1 to 3 (i.e., 1, 2, or 3); and the dashed line (" 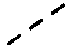 ") represents the point of attachment of the adjuvant.

In some embodiments, the adjuvant ("Adj") is of formula: 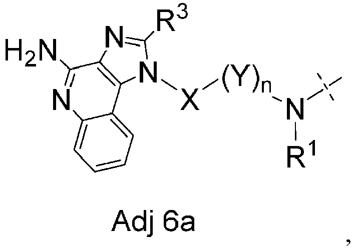 wherein R¹ is selected from H and C₁₋₄ alkyl; R³ is selected from C₁₋₆ alkyl and 2-to 6-membered heteroalkyl, each of which is optionally substituted with one or more members selected from the group consisting of halo, hydroxy, amino, oxo (=O), alkylamino, amido, acyl, nitro, cyano, and alkoxy; X is selected from O and CH₂; each Y is independently CHR², wherein R² is selected from H, OH, and NH₂, subscript n is an integer from 1 to 12 (i.e., 1, 2, 3, 4, 5, 6, 7, 8, 9, 10, 11, or 12); and the dashed line (" 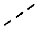 ") represents the point of attachment of the adjuvant. Alternatively, R¹ and the nitrogen atom to which it is attached can form a linking moiety comprising a 5-to 8-membered heterocycle. In some embodiments, subscript n is an integer from 1 to 6 (i.e., 1, 2, 3, 4, 5, or 6). In certain embodiments, subscript n is an integer from 1 to 3 (i.e., 1, 2, or 3).

In some embodiments, the adjuvant ("Adj") is of formula: 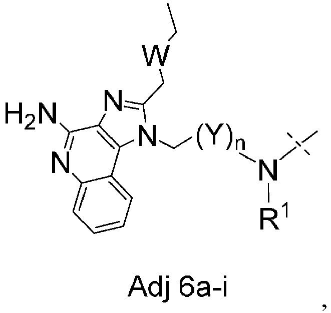 wherein W is selected from the group consisting of O and CH₂; R¹ is selected from H and C₁₋₄ alkyl; each Y is independently CHR², wherein R² is selected from H, OH, and NH₂; subscript n is an integer from 1 to 12 (i.e., 1, 2, 3, 4, 5, 6, 7, 8, 9, 10, 11, or 12); and the dashed line (" 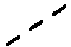 ") represents the point of attachment of the adjuvant. Alternatively, R¹ and the nitrogen atom to which it is attached can form a linking moiety comprising a 5-to 8-membered heterocycle. In some embodiments, subscript n is an integer from 1 to 6 (i.e., 1, 2, 3, 4, 5, or 6). In certain embodiments, subscript n is an integer from 1 to 3 (i.e., 1, 2, or 3).

In some embodiments, the adjuvant ("Adj") is of formula: 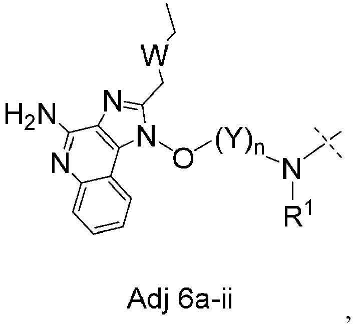 wherein W is selected from the group consisting of O and CH₂; R¹ is selected from H and C₁₋₄ alkyl; each Y is independently CHR², wherein R² is selected from H, OH, and NH₂; subscript n is an integer from 1 to 12 (i.e., 1, 2, 3, 4, 5, 6, 7, 8, 9, 10, 11, or 12); and the dashed line (" 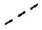 ") represents the point of attachment of the adjuvant. Alternatively, R¹ and the nitrogen atom to which it is attached can form a linking moiety comprising a 5-to 8-membered heterocycle. In some embodiments, subscript n is an integer from 1 to 6 (i.e., 1, 2, 3, 4, 5, or 6). In certain embodiments, subscript n is an integer from 1 to 3 (i.e., 1, 2, or 3).

In some embodiments, the adjuvant ("Adj") is of formula: 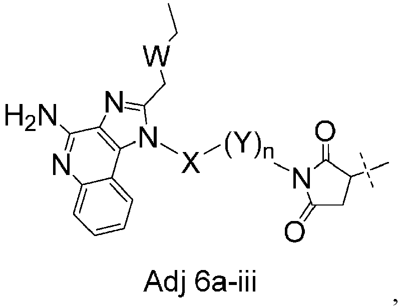 wherein W is selected from the group consisting of O and CH₂; X is selected from O and CH₂; each Y is independently CHR², wherein R² is selected from H, OH, and NH₂; subscript n is an integer from 1 to 12 (i.e., 1, 2, 3, 4, 5, 6, 7, 8, 9, 10, 11, or 12); and the dashed line (" 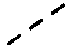 ") represents the point of attachment of the adjuvant. In some embodiments, subscript n is an integer from 1 to 6 (i.e., 1, 2, 3, 4, 5, or 6). In certain embodiments, subscript n is an integer from 1 to 3 (i.e., 1, 2, or 3).

In some embodiments, the adjuvant ("Adj") is of formula: 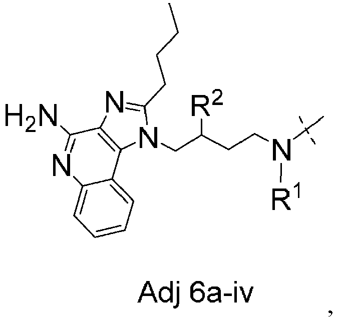 wherein R¹ is selected from H and C₁₋₄ alkyl; R² is selected from H, OH, and NH₂; and the dashed line (" 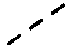 ") represents the point of attachment of the adjuvant.

In some embodiments, the adjuvant ("Adj") is of formula: 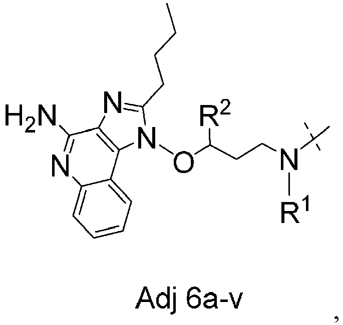 wherein R¹ is selected from H and C₁₋₄ alkyl; R² is selected from H, OH, and NH₂; and the dashed line (" 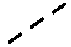 ") represents the point of attachment of the adjuvant.

In certain embodiments, the adjuvant ("Adj") is: 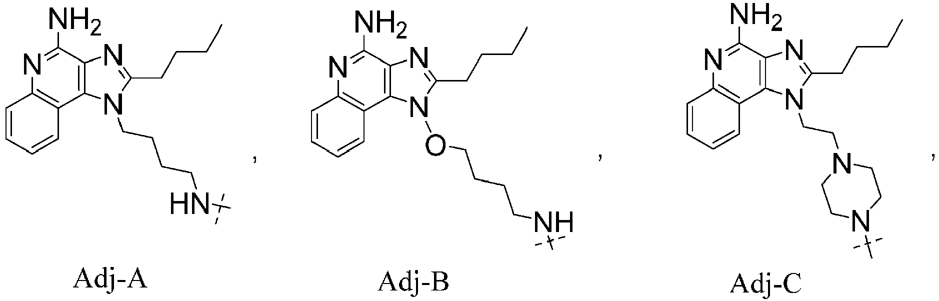 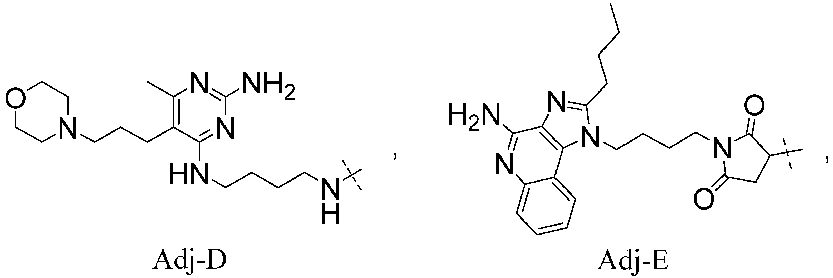 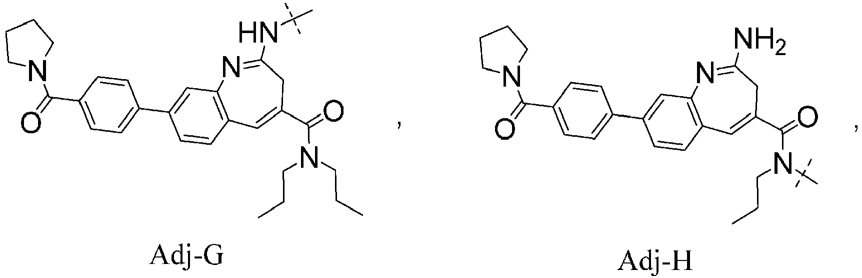 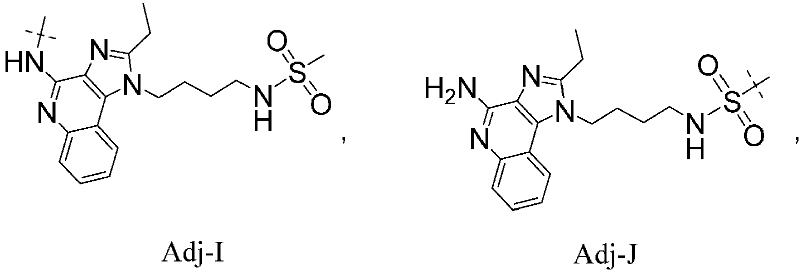 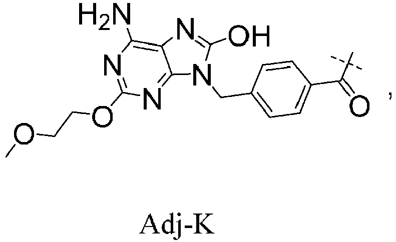 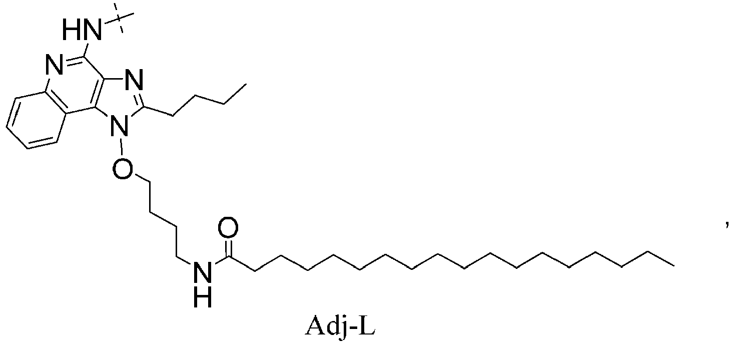 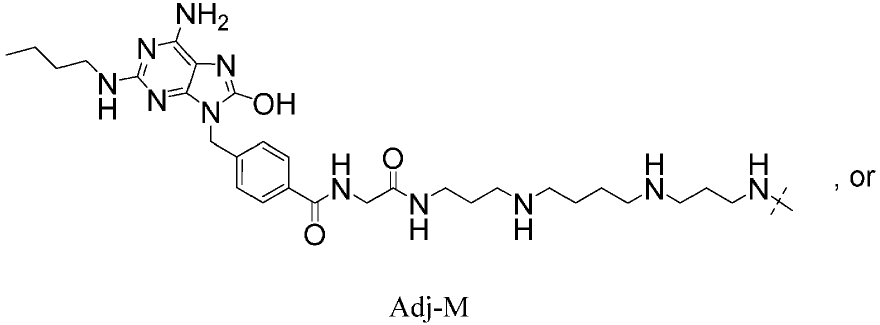 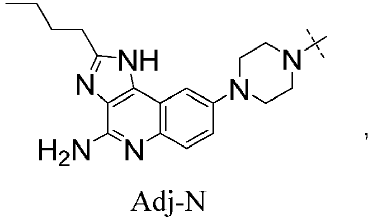 wherein the dashed line (" 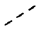 ") represents the point of attachment of the adjuvant.

In some embodiments, the adjuvant is not a fluorophore. In some embodiments, the adjuvant is not a radiodiagnostic compound. In some embodiments, the adjuvant is not a radiotherapuetic compound. In some embodiments, the adjuvant is not a tubulin inhibitor. In some embodiments, the adjuvant is not a DNA crosslinker/alkylator. In some embodiments, the adjuvant is not a topoisomerase inhibitor.

### Antibody

The antibody in the immunoconjugate can be any suitable antibody. The antibody in the immunoconjugates typically is an allogeneic antibody. The terms "allogeneic antibody" or "alloantibody" refer to an antibody that is not from the individual in question (e.g., an individual with a tumor and seeking treatment), but is from the same species, or is from a different species, but has been engineered to reduce, mitigate, or avoid recognition as a xeno-antibody (e.g., non-self). For example, the "allogeneic antibody" can be a humanized antibody. One skilled in the art is knowledgeable regarding how to engineer a non-human antibody to avoid recognition as a xeno-antibody. Unless specifically stated otherwise, "antibody" and "allogeneic antibody" as used herein refer to immunoglobulin G (IgG) or immunoglobulin A (IgA).

If a cancer cell of a human individual is contacted with an antibody that was not generated by that same person (e.g., the antibody was generated by a second human individual, the antibody was generated by another species such as a mouse, the antibody is a humanized antibody that was generated by another species, etc.), then the antibody is considered to be allogeneic (relative to the first individual). A humanized mouse monoclonal antibody that recognizes a human antigen (e.g., a cancer-specific antigen, an antigen that is enriched in and/or on cancer cells, etc.) is considered to be an "alloantibody" (an allogeneic antibody).

The antibody can be a polyclonal allogeneic IgG antibody. The antibody can be present in a mixture of polyclonal IgG antibodies with a plurality of binding specificities. The antibodies of the mixture can specifically bind to different target molecules, and the antibodies of the mixture can specifically bind to different epitopes of the same target molecule. Thus, a mixture of antibodies can include more than one immunoconjugate of the invention (e.g., adjuvant moieties can be covalently bonded to antibodies of a mixture, e.g., a mixture of polyclonal IgG antibodies, resulting in a mixture of antibody-adjuvant conjugates of the invention). A mixture of antibodies can be pooled from 2 or more individuals (e.g., 3 or more individuals, 4 or more individuals, 5 or more individuals, 6 or more individuals, 7 or more individuals, 8 or more individuals, 9 or more individuals, 10 or more individuals, etc.). In some embodiments, pooled serum is used as a source of alloantibody, where the serum can come from any number of individuals, none of whom are the first individual (e.g., the serum can be pooled from 2 or more individuals, 3 or more individuals, 4 or more individuals, 5 or more individuals, 6 or more individuals, 7 or more individuals, 8 or more individuals, 9 or more individuals, 10 or more individuals, etc.). The antibodies can be isolated or purified from serum prior to use. The purification can be conducted before or after pooling the antibodies from different individuals.

In some embodiments where the antibodies in the immunoconjugates comprise IgGs from serum, the target antigens for some (e.g., greater than 0% but less than 50%), half, most (greater than 50% but less than 100%), or even all of the antibodies (i.e., IgGs from the serum) will be unknown. However, the chances are high that at least one antibody in the mixture will recognize the target antigen of interest because such a mixture contains a wide variety of antibodies specific for a wide variety of target antigens.

In some embodiments, the antibody is a polyclonal allogeneic IgA antibody. The antibody can be present in a mixture of polyclonal IgA antibodies with a plurality of binding specificities. The antibodies of the mixture can specifically bind to different target molecules, and the antibodies of the mixture can specifically bind to different epitopes of the same target molecule. Thus, a mixture of antibodies can include more than one immunoconjugate of the invention (e.g., adjuvant moieties can be covalently bonded to antibodies of a mixture, e.g., a mixture of polyclonal IgA antibodies, resulting in a mixture of antibody-adjuvant conjugates of the invention). A mixture of antibodies can be pooled from 2 or more individuals (e.g., 3 or more individuals, 4 or more individuals, 5 or more individuals, 6 or more individuals, 7 or more individuals, 8 or more individuals, 9 or more individuals, 10 or more individuals, etc.). In some embodiments, pooled serum is used as a source of alloantibody, where the serum can come from any number of individuals, none of whom are the first individual (e.g., the serum can be pooled from 2 or more individuals, 3 or more individuals, 4 or more individuals, 5 or more individuals, 6 or more individuals, 7 or more individuals, 8 or more individuals, 9 or more individuals, 10 or more individuals, etc.). The antibodies can be isolated or purified from serum prior to use. The purification can be conducted before or after pooling the antibodies from different individuals.

In some embodiments where the antibodies in the immunoconjugates comprise IgAs from serum, the target antigens for some (e.g., greater than 0% but less than 50%), half, most (greater than 50% but less than 100%), or even all of the antibodies (i.e., IgAs from the serum) will be unknown. However, the chances are high that at least one antibody in the mixture will recognize the target antigen of interest because such a mixture contains a wide variety of antibodies specific for a wide variety of target antigens.

In some embodiments, the antibody in the immunoconjugates includes intravenous immunoglobulin (IVIG) and/or antibodies from (e.g., enriched from, purified from, e.g., affinity purified from) IVIG. IVIG is a blood product that contains IgG (immunoglobulin G) pooled from the plasma (e.g., in some embodiments without any other proteins) from many (e.g., sometimes over 1,000 to 60,000) normal and healthy blood donors. IVIG is commercially available. IVIG contains a high percentage of native human monomeric IVIG and has low IgA content. When administered intravenously, IVIG ameliorates several disease conditions. Therefore, the United States Food and Drug Administration (FDA) has approved the use of IVIG for a number of diseases including: (1) Kawasaki disease; (2) immune-mediated thrombocytopenia; (3) primary immunodeficiencies; (4) hematopoietic stem cell transplantation (for those older than 20 years); (5) chronic B-cell lymphocytic leukemia; and (6) pediatric HIV type 1 infection. In 2004, the FDA approved the Cedars-Sinai IVIG Protocol for kidney transplant recipients so that such recipients could accept a living donor kidney from any healthy donor, regardless of blood type (ABO incompatible) or tissue match. These and other aspects of IVIG are described, for example, in U.S. Patent Application Publications 2010/0150942; 2004/0101909; 2013/0177574; 2013/0108619; and 2013/0011388.

In some embodiments, the antibody is a monoclonal antibody of a defined subclass (e.g., IgG₁, IgG₂, IgG₃, IgG₄, IgA₁, or IgA₂). If combinations of antibodies are used, the antibodies can be from the same subclass or from different subclasses. For example, the antibodies can be IgG₁ antibodies. Various combinations of different subclasses, in different relative proportions, can be obtained by those of skill in the art. In some embodiments, a specific subclass, or a specific combination of different subclasses can be particularly effective at cancer treatment or tumor size reduction. Accordingly, some embodiments of the invention provide immunoconjugates wherein the antibody is a monoclonal antibody. In some embodiments, the monoclonal antibody is humanized.

In some embodiments, the antibody binds to an antigen of a cancer cell. For example, the antibody can bind to a target antigen that is present at an amount of at least 10; 100; 1,000; 10,000; 100,000; 1,000,000; 2.5 × 10⁶; 5 × 10⁶; or 1 × 10⁷ copies or more on the surface of a cancer cell.

In some embodiments, the antibody binds to an antigen on a cancer or immune cell at a higher affinity than a corresponding antigen on a non-cancer cell. For example, the antibody may preferentially recognize an antigen containing a polymorphism that is found on a cancer or immune cell as compared to recognition of a corresponding wild-type antigen on the non-cancer or non-immune cell. In some instances, the antibody binds a cancer or immune cell with greater avidity than a non-cancer or non-immune cell. For example, the cancer or immune cell can express a higher density of an antigen, thus providing for a higher affinity binding of a multivalent antibody to the cancer or immune cell.

In some embodiments, the antibody does not significantly bind non-cancer antigens (e.g., the antibody binds one or more non-cancer antigens with at least 10; 100; 1,000; 10,000; 100,000; or 1,000,000-fold lower affinity (higher Kd) than the target cancer antigen). In some embodiments, the target cancer antigen to which the antibody binds is enriched on the cancer cell. For example, the target cancer antigen can be present on the surface of the cancer cell at a level that is at least 2, 5, 10, 100, 1,000, 10,000, 100,000, or 1,000,000-fold higher than a corresponding non-cancer cell. In some embodiments, the corresponding non-cancer cell is a cell of the same tissue or origin that is not hyperproliferative or otherwise cancerous. In general, a subject IgG antibody that specifically binds to an antigen (a target antigen) of a cancer cell preferentially binds to that particular antigen relative to other available antigens. However, the target antigen need not be specific to the cancer cell or even enriched in cancer cells relative to other cells (e.g., the target antigen can be expressed by other cells). Thus, in the phrase "an antibody that specifically binds to an antigen of a cancer cell," the term "specifically" refers to the specificity of the antibody and not to the uniqueness of the antigen in that particular cell type.

### Modified Fc Region

In some embodiments, the antibodies in the immunoconjugates contain a modified Fc region, wherein the modification modulates the binding of the Fc region to one or more Fc receptors.

The terms "Fc receptor" or "FcR" refer to a receptor that binds to the Fc region of an antibody. There are three main classes of Fc receptors: FcγR which bind to IgG, FcaR which binds to IgA, and FcεR which binds to IgE. The FcγR family includes several members, such as FcγI (CD64), FcγRIIA (CD32A), FcyRIIB (CD32B), FcγRIIIA (CD16A), and FcγRIIIB (CD16B). The Fcγ receptors differ in their affinity for IgG and also have different affinities for the IgG subclasses (e.g., IgG1, IgG2, IgG3, IgG4).

In some embodiments, the antibodies in the immunoconjugates (e.g., antibodies conjugated to a TLR agonist such as a TLR7/8 agonist via a linker) contain one or more modifications (e.g., amino acid insertion, deletion, and/or substitution) in the Fc region that results in modulated binding (e.g., increased binding or decreased binding) to one or more Fc receptors (e.g., FcγRI (CD64), FcγRIIA (CD32A), FcγRIIB (CD32B), FcγRIIIA (CD16a), and/or FcγRIIIB (CD16b)) as compared to the native antibody lacking the mutation in the Fc region. In some embodiments, the antibodies in the immunoconjugates contain one or more modifications (e.g., amino acid insertion, deletion, and/or substitution) in the Fc region that reduce the binding of the Fc region of the antibody to FcγRIIB. In some embodiments, the antibodies in the immunoconjugates contain one or more modifications (e.g., amino acid insertion, deletion, and/or substitution) in the Fc region of the antibody that reduce the binding of the antibody to FcγRIIB while maintaining the same binding or having increased binding to FcγRI (CD64), FcγRIIA (CD32A), and/or FcRγIIIA (CD16a) as compared to the native antibody lacking the mutation in the Fc region. In some embodiments, the antibodies in the immunoconjugates contain one of more modifications in the Fc region that increase the binding of the Fc region of the antibody to FcγRIIB.

In some embodiments, the modulated binding is provided by mutations in the Fc region of the antibody relative to the native Fc region of the antibody. The mutations can be in a CH2 domain, a CH3 domain, or a combination thereof. A "native Fc region" is synonymous with a "wild-type Fc region" and comprises an amino acid sequence that is identical to the amino acid sequence of an Fc region found in nature or identical to the amino acid sequence of the Fc region found in the native antibody (e.g., rituximab). Native sequence human Fc regions include a native sequence human IgG1 Fc region; native sequence human IgG2 Fc region; native sequence human IgG3 Fc region; and native sequence human IgG4 Fc region as well as naturally occurring variants thereof. Native sequence Fc includes the various allotypes of Fcs (see, e.g., Jefferis et al., mAbs, 1(4): 332-338 (2009)).

In some embodiments, the mutations in the Fc region that result in modulated binding to one or more Fc receptors can include one or more of the following mutations: SD (S239D), SDIE (S239D/I332E), SE (S267E), SELF (S267E/L328F), SDIE (S239D/I332E), SDIEAL (S239D/I332E/A330L), GA (G236A), ALIE (A330L/I332E), GASDALIE (G236A/S239D/A330L/I332E), V9 (G237D/P238D/P271G/A330R), and V11 (G237D/P238D/H268D/P271G/A330R) and/or one or more mutations at the following amino acids: E233, G237, P238, H268, P271, L328 and A330. Additional Fc region modifications for modulating Fc receptor binding are described, e.g., in U.S. Patent Application Publication 2016/0145350, and U.S. Patents 7,416,726 and 5,624,821.

In some embodiments, the Fc region of the antibodies of the immunoconjugates are modified to have an altered glycosylation pattern of the Fc region compared to the native non-modified Fc region.

Human immunoglobulin is glycosylated at the Asn297 residue in the Cy2 domain of each heavy chain. This *N*-linked oligosaccharide is composed of a core heptasaccharide, N-acetylglucosamine4Mannose3 (GlcNAc4Man3). Removal of the heptasaccharide with endoglycosidase or PNGase F is known to lead to conformational changes in the antibody Fc region, which can significantly reduce antibody-binding affinity to activating FcγR and lead to decreased effector function. The core heptasaccharide is often decorated with galactose, bisecting GlcNAc, fucose or sialic acid, which differentially impacts Fc binding to activating and inhibitory FcγR. Additionally, it has been demonstrated that α2,6-sialyation enhances anti-inflammatory activity *in vivo* while defucosylation leads to improved FcγRIIIa binding and a 10-fold increase in antibody-dependent cellular cytotoxicity and antibody-dependent phagocytosis. Specific glycosylation patterns can therefore be used to control inflammatory effector functions.

In some embodiments, the modification to alter the glycosylation pattern is a mutation. For example, a substitution at Asn297. In some embodiments, Asn297 is mutated to glutamine (N297Q). Methods for controlling immune response with antibodies that modulate FcγR-regulated signaling are described, for example, in U.S. Patent 7,416,726, as well as U.S. Patent Application Publications 2007/0014795 and 2008/0286819.

In some embodiments, the antibodies of the immunoconjugates are modified to contain an engineered Fab region with a non-naturally occurring glycosylation pattern. For example, hybridomas can be genetically engineered to secrete afucosylated mAb, desialylated mAb or deglycosylated Fc with specific mutations that enable increased FcRγIIIa binding and effector function. In some embodiments, the antibodies of the immunoconjugates are engineered to be afucosylated (e.g., afucosylated rituximab, available from Invivogen, hcd20-mab13).

In some embodiments, the entire Fc region of an antibody in the immunoconjugates is exchanged with a different Fc region, so that the Fab region of the antibody is conjugated to a non-native Fc region. For example, the Fab region of rituximab, which normally comprises an IgG1 Fc region, can be conjugated to IgG2, IgG3, IgG4, or IgA, or the Fab region of nivolumab, which normally comprises an IgG4 Fc region, can be conjugated to IgG1, IgG2, IgG3, IgA1 or IgG2. In some embodiments, the Fc modified antibody with a non-native Fc domain also comprises one or more amino acid modification, such as the S228P mutation within the IgG4 Fc, that modulate the stability of the Fc domain described. In some embodiments, the Fc modified antibody with a non-native Fc domain also comprises one or more amino acid modifications described herein that modulate Fc binding to FcR.

In some embodiments, the modifications that modulate the binding of the Fc region to FcR do not alter the binding of the Fab region of the antibody to its antigen when compared to the native non-modified antibody. In other embodiments, the modifications that modulate the binding of the Fc region to FcR also increase the binding of the Fab region of the antibody to its antigen when compared to the native non-modified antibody.

### Antibody Target

The antibody target can be any suitable antibody target. In some embodiments, the antibody is capable of binding one or more targets selected from (e.g., specifically binds to a target selected from) 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOCl, AR, aromatase, ASPH, ATX, AX1, AXL, AZGP1 (zinc-a-glycoprotein), B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BIyS, BMP1, BMP2, BMP3B (GDFIO), BMP4, BMP6, BMP8, BMP10, BMPR1A, BMPR1B, BMPR2, BPAG1 (plectin), BRCA1, C19orflO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22(MDC/STC-I), CCL23 (MPIF-I), CCL24 (MPIF-2/eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD3, CD33, CD35, CD37, CD38, CD3E, CD3G, CD3Z, CD4, CD40, CD40L, CD44, CD45RB, CD47, CD52, CD69, CD70, CD72, CD74, CD79A, CD79B, CD8, CD80, CD81, CD83, CD86, CD125, CD137, CD147, CD179b, CD223, CD279, CD152, CD274, CDH6, CDH1 (E-cadherin), CDH1O, CDH12, CDH13, CDH18, CDH19, CDH2O, CDH3, CDH5, CDH6, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin-7), CLDN18, CLEC5A, CLEC6A, CLEC11A, CLEC14A, CLN3, CLU (clusterin), CMKLR1, CMKOR1 (RDC1), CNR1, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, Estrogen receptor, Earl, ESR2, F3 (TF), FADD, FAP, farnesyltransferase, FasL, FASNf, FCER1A, FCER2, FCGR3A, FGF, FGF1 (aFGF), FGF10, FGF11, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, histamine and histamine receptors, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFNgamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), ILlR2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, IL1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (glycoprotein 130), ILK, INHA, INHBA, INSL3, INSL4, IRAK1, IRAK2, ITGA1, ITGA2, ITGA3, ITGA6 (α6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (Keratin 19), KRT2A, KRTHB6(hair-specific type II keratin), L1CAM, LAG3, LAMA5, LAMP1, LEP (leptin), Lewis Y antigen ("LeY"), LILRB1, Lingo-p75, Lingo-Troy, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (metallothionectin-UI), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P-cadherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, phosphacan, PIAS2, PI3 Kinase, PIK3CG, PLAU (uPA), PLG, PLXDCI, PKC, PKC-beta, PPBP (CXCL7), PPID, PR1, PRAME, PRKCQ, PRKD1, PRL, PROC, PROK2, PSAP, PSCA, PSMA, PTAFR, PTEN, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (lipophilin B), SCGB2A1 (mammaglobin 2), SCGB2A2 (mammaglobin 1), SCYE1 (endothelial Monocyte-activating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF10 (TRAIL), TNFRSF10A, TNFRSF10B, TNFRSF12A, TNFRSF17, TNFSF11 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, versican, VHL C5, VLA-4, WT1, Wnt-1, XCL1 (lymphotactin), XCL2 (SCM-Ib), XCRI (GPR5/CCXCR1), YY1, ZFPM2, CLEC4C (BDCA-2, DLEC, CD303, CEA, CDH6, CLECSF7), CLEC4D (MCL, CLECSF8), CLEC4E (Mincle), CLEC6A (Dectin-2), CLEC5A (MDL-1, CLECSF5), CLEC1B (CLEC-2), CLEC9A (DNGR-1), CLEC7A (Dectin-1), CLEC11A, GFRA1, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), P-cadherin, PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, and KLRF1 (NKp80).

In certain embodiments, the antigen binding domain binds to an antigen selected from the group consisting of CDH1, CD19, CD20, CD29, CD30, CD40, CD47, EpCAM, SLAMF7, PDGFRa, gp75, MSLN, CA6, CA9, Caprin-1, CDH6, CEA, CTAG1B/NY-ESO-1, LAMP1, LeY, MAGEA3/A6, P-cadherin, BCMA, CD38, HLA-DR, ROR1, WT1, GFRA1, FR-alpha, L1-CAM, LRRC15, MUC1, MUC16, PSMA, SLC34A2, TROP2, GPC3, CCR8, and VEGF.

In some embodiments, the antibody is capable of binding one or more targets selected from (e.g., specifically binds to a target selected from): ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), and CH10 (Q64433). In the preceding list, accession numbers are shown in parentheses.

In some embodiments, the antibody binds an antigen selected from CDH1, CD19, CD20, CD29, CD30, CD47, CD179b, CAPRIN-1, EpCAM, MUC1, MUC16, EGFR, HER2, and gp75. In some embodiments, the antigen is selected from CD19, CD20, CD47, CD179b, CAPRIN-1, EpCAM, MUC1, MUC16, EGFR, and HER2. In some embodiments, the antibody binds an antigen selected from CD20 and CAPRIN-1. In some embodiments, the antibody binds an antigen selected from EGFR and HER2. In some embodiments, the antibody binds antigen HER2.

In some embodiments, the antibody is an anti-Cofilin-1 antibody, an anti-APOA2 antibody, or an anti-COTL-1 antibody.

In some embodiments, the antibody is an anti-CD19 antibody, anti-CD20 antibody, anti-CD22 antibody, anti-CD24 antibody, anti-CD25 antibody, anti-CD30 antibody, anti-CD33 antibody, anti-CD38 antibody, anti-CD44 antibody, anti-CD47 antibody, anti-CD52 antibody, anti-CD56 antibody, anti-CD70 antibody, anti-CD96 antibody, anti-CD97 antibody, anti-CD99 antibody, anti-CD117 antibody, anti-CD123 antibody, anti-CD179b antibody, anti-CD223, anti-CD279 (PD-1) antibody, anti-CD274 (PD-L1) antibody, anti-EpCam antibody, anti-EGFR antibody, anti-VEGF, anti-VEGFB, anti-VEGFC, anti-17-1A antibody, anti-CTLA4 antibody, anti-HER2 antibody, anti-C-Met antibody, anti-PTHR2 antibody, anti-HAVCR2 (TIM3) antibody, anti-CAPRIN-1 antibody, anti-Dectin-2 antibody, anti-CLEC5A antibody, and anti-SIRPA antibody. In some embodiments, the antibody is selected from the group consisting of an anti-HER2 antibody and an anti-EGFR antibody

In addition to antibodies, alternative protein scaffolds may be used as part of the immunoconjugates. The term "alternative protein scaffold" refers to a non-immunoglobulin derived protein or peptide. Such proteins and peptides are generally amenable to engineering and can be designed to confer monospecificity against a given antigen, bispecificity, or multispecificity. Engineering of an alternative protein scaffold can be conducted using several approaches. A loop grafting approach can be used where sequences of known specificity are grafted onto a variable loop of a scaffold. Sequence randomization and mutagenesis can be used to develop a library of mutants, which can be screened using various display platforms (e.g., phage display) to identify a novel binder. Site-specific mutagenesis can also be used as part of a similar approach. Alternative protein scaffolds exist in a variety of sizes, ranging from small peptides with minimal secondary structure to large proteins of similar size to a full-sized antibody. Examples of scaffolds include, but are not limited to, cystine knotted miniproteins (also known as knottins), cyclic cystine knotted miniproteins (also known as cyclotides), avimers, affibodies, the tenth type III domain of human fibronectin, DARPins (designed ankyrin repeats), and anticalins (also known as lipocalins). Naturally occurring ligands with known specificity can also be engineered to confer novel specificity against a given target. Examples of naturally occurring ligands that may be engineered include the EGF ligand and VEGF ligand. Engineered proteins can either be produced as monomeric proteins or as multimers, depending on the desired binding strategy and specificities. Protein engineering strategies can be used to fuse alternative protein scaffolds to Fc domains.

In some embodiments, the antibody binds to an FcRy-coupled receptor. In some embodiments, the FcRy- coupled receptor is selected from the group consisting of GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, and TARM1.

In some embodiments, the antibody binds to a DAP12-coupled receptor. In some embodiments, the DAP12-coupled receptor is selected from the group consisting of CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPB1 (CD172B), TREM1 (CD354), and TREM2.

In some embodiments, the antibody binds to a hemITAM-bearing receptor. In some embodiments, the hemITAM-bearing receptor is KLRF1 (NKp80).

In some embodiments, the antibody is capable of binding one or more targets selected from CLEC4C (BDCA-2, DLEC, CD303, CLECSF7), CLEC4D (MCL, CLECSF8), CLEC4E (Mincle), CLEC6A (Dectin-2), CLEC5A (MDL-1, CLECSF5), CLEC1B (CLEC-2), CLEC9A (DNGR-1), and CLEC7A (Dectin-1). In some embodiments, the antibody is capable of binding CLEC6A (Dectin-2) or CLEC5A. In some embodiments, the antibody is capable of binding CLEC6A (Dectin-2).

In some embodiments, the antibody is capable of binding one or more targets selected from (e.g., specifically binds to a target selected from): ATP5I (Q06185), OAT (P29758), AIFM1 (Q9ZOX1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), and CH10 (Q64433). In the preceding list, accession numbers are shown in parentheses.

In some embodiments, the antibody binds to an antigen selected from CCR8, CDH1, CD19, CD20, CD29, CD30, CD38, CD40, CD47, EpCAM, MUC1, MUC16, EGFR, HER2, SLAMF7, and gp75. In some embodiments, the antigen is selected from CCR8, CD19, CD20, CD47, EpCAM, MUC1, MUC16, EGFR, and HER2. In some embodiments, the antibody binds to an antigen selected from the Tn antigen and the Thomsen-Friedenreich antigen. In some embodiments, the antibody binds to an antigen selected from EGFR, CCR8, and HER2. In certain embodiments, the antibody binds to HER2.

In some embodiments, the antibody is selected from: abagovomab, abatacept (also known as ORENCIA^{™}), abciximab (also known as REOPRO^{™}, c7E3 Fab), adalimumab (also known as HUMIRA^{™}), adecatumumab, alemtuzumab (also known as CAMPATH^{™}, MabCampath or Campath-1H), altumomab, afelimomab, anatumomab mafenatox, anetumumab, anrukizumab, apolizumab, arcitumomab, aselizumab, atlizumab, atorolimumab, bapineuzumab, basiliximab (also known as SIMULECT^{™}), bavituximab, bectumomab (also known as LYMPHOSCAN^{™}), belimumab (also known as LYMPHO-STAT-B^{™}), bertilimumab, besilesomab, bevacizumab (also known as AVASTIN^{™}), biciromab brallobarbital, bivatuzumab mertansine, campath, canakinumab (also known as ACZ885), cantuzumab mertansine, capromab (also known as PROSTASCINT^{™}), catumaxomab (also known as REMOVAB^{™}), cedelizumab (also known as CIMZIA^{™}), certolizumab pegol, cetuximab (also known as ERBITUX^{™}), clenoliximab, dacetuzumab, dacliximab, daclizumab (also known as ZENAPAX^{™}), denosumab (also known as AMG 162), detumomab, dorlimomab aritox, dorlixizumab, duntumumab, durimulumab, durmulumab, ecromeximab, eculizumab (also known as SOLIRIS^{™}), edobacomab, edrecolomab (also known as Mab17-1A, PANOREX^{™}), efalizumab (also known as RAPTIVA^{™}), efungumab (also known as MYCOGRAB^{™}), elotuzumab, elsilimomab, enlimomab pegol, epitumomab cituxetan, efalizumab, epitumomab, epratuzumab, erlizumab, ertumaxomab (also known as REXOMUN^{™}), etanercept (also known as ENBREL^{™}), etaracizumab (also known as etaratuzumab, VITAXIN^{™}, ABEGRIN^{™}), exbivirumab, fanolesomab (also known as NEUTROSPEC^{™}), faralimomab, felvizumab, fontolizumab (also known as HUZAF^{™}), galiximab, gantenerumab, gavilimomab (also known as ABXCBL^{™}), gemtuzumab ozogamicin (also known as MYLOTARG^{™}), golimumab (also known as CNTO 148), gomiliximab, ibalizumab (also known as TNX-355), ibritumomab tiuxetan (also known as ZEVALIN^{™}), igovomab, imciromab, infliximab (also known as REMICADE^{™}), inolimomab, inotuzumab ozogamicin, ipilimumab (also known as MDX-010, MDX-101), iratumumab, keliximab, labetuzumab, lemalesomab, lebrilizumab, lerdelimumab, lexatumumab (also known as, HGS-ETR2, ETR2-ST01), lexitumumab, libivirumab, lintuzumab, lucatumumab, lumiliximab, mapatumumab (also known as HGSETR1, TRM-1), maslimomab, matuzumab (also known as EMD72000), mepolizumab (also known as BOSATRIA^{™}), metelimumab, milatuzumab, minretumomab, mitumomab, morolimumab, motavizumab (also known as NUMAX^{™}), muromonab (also known as OKT3), nacolomab tafenatox, naptumomab estafenatox, natalizumab (also known as TYSABRI^{™}, ANTEGREN^{™}), nebacumab, nerelimomab, nimotuzumab (also known as THERACIM hR3^{™}, THERA-CIM-hR3^{™}, THERALOC^{™}), nofetumomab merpentan (also known as VERLUMA^{™}), obinutuzumab, ocrelizumab, odulimomab, ofatumumab, omalizumab (also known as XOLAIR^{™}), oregovomab (also known as OVAREX^{™}), otelixizumab, pagibaximab, palivizumab (also known as SYNAGIS^{™}), panitumumab (also known as ABX-EGF, VECTIBIX^{™}), pascolizumab, pemtumomab (also known as THERAGYN^{™}), pertuzumab (also known as 2C4, OMNITARG^{™}), pexelizumab, pintumomab, priliximab, pritumumab, ranibizumab (also known as LUCENTIS^{™}), raxibacumab, regavirumab, reslizumab, rituximab (also known as RITUXAN^{™}, MabTHERA^{™}), rovelizumab, ruplizumab, satumomab, sevirumab, sibrotuzumab, siplizumab (also known as MEDI-507), sontuzumab, stamulumab (also known as MYO-029), sulesomab (also known as LEUKOSCAN^{™}), tacatuzumab tetraxetan, tadocizumab, talizumab, taplitumomab paptox, tefibazumab (also known as AUREXIS^{™}), telimomab aritox, teneliximab, teplizumab, ticilimumab, tocilizumab (also known as ACTEMRA^{™}), toralizumab, tositumomab, trastuzumab (also known as HERCEPTIN^{™}), tremelimumab (also known as CP-675,206), tucotuzumab celmoleukin, tuvirumab, urtoxazumab, ustekinumab (also known as CNTO 1275), vapaliximab, veltuzumab, vepalimomab, visilizumab (also known as NUVION^{™}), volociximab (also known as M200), votumumab (also known as HUMASPECT^{™}), zalutumumab, zanolimumab (also known as HuMAX-CD4), ziralimumab, zolimomab aritox, daratumumab, olaratumab, brentuximab vedotin, afibercept, abatacept, belatacept, afibercept, etanercept, romiplostim, SBT-040 (sequences listed in U.S. Patent Application Publication 2017/0158772). In some embodiments, the antibody is selected from the group consisting of olaratumab, obinutuzumab, trastuzumab, cetuximab, rituximab, pertuzumab, bevacizumab, daratumumab, etanercept, pembrolizumab, nivolumab, atezolizumab, ipilimumab, panitumumab, zalutumumab, nimotuzumab, matuzumab, and elotuzumab. In certain embodiments, the antibody is trastuzumab.

### Checkpoint Inhibitor

Any suitable immune checkpoint inhibitor is contemplated for use with the immunoconjugates disclosed herein. In some embodiments, the immune checkpoint inhibitor reduces the expression or activity of one or more immune checkpoint proteins. In another embodiment, the immune checkpoint inhibitor reduces the interaction between one or more immune checkpoint proteins and their ligands. Inhibitory nucleic acids that decrease the expression and/or activity of immune checkpoint molecules can also be used in the methods disclosed herein.

Most checkpoint antibodies are designed not to have effector function as they are not trying to kill cells, but rather to block the signaling. Immunoconjugates of the invention can add back the "effector functionality" needed to activate myeloid immunity. Hence, for most checkpoint antibody inhibitors this discovery will be critical.

In some embodiments, the immune checkpoint inhibitor is cytotoxic T-lymphocyte antigen 4 (CTLA4, also known as CD152), T cell immunoreceptor with Ig and ITIM domains (TIGIT), glucocorticoid-induced TNFR-related protein (GITR, also known as TNFRSF18), inducible T cell costimulatory (ICOS, also known as CD278), CD96, poliovirus receptor-related 2 (PVRL2, also known as CD112R, programmed cell death protein 1 (PD-1, also known as CD279), programmed cell death 1 ligand 1 (PD-L1, also known as B7-H3 and CD274), programmed cell death ligand 2 (PD-L2, also known as B7-DC and CD273), lymphocyte activation gene-3 (LAG-3, also known as CD223), B7-H4, killer immunoglobulin receptor (KIR), Tumor Necrosis Factor Receptor superfamily member 4 (TNFRSF4, also known as OX40 and CD134) and its ligand OX40L (CD252), indoleamine 2,3-dioxygenase 1 (IDO-1), indoleamine 2,3-dioxygenase 2 (IDO-2), carcinoembryonic antigen-related cell adhesion molecule 1 (CEACAM1), B and T lymphocyte attenuator (BTLA, also known as CD272), T-cell membrane protein 3 (TIM3), the adenosine A2A receptor (A2Ar), and V-domain Ig suppressor of T cell activation (VISTA protein). In some embodiments, the immune checkpoint inhibitor is an inhibitor of CTLA4, PD-1, or PD-L1.

In some embodiments, the antibody is selected from ipilimumab (also known as YERVOY^{™} pembrolizumab (also known as KEYTRUDA^{™}), nivolumab (also known as OPDIVO^{™}), atezolizumab (also known as TECENTRIG^{™}), avelumab (also known as BAVENCIO^{™}), and durvalumab (also known as IMFINZI^{™}). In some embodiments, the antibody is selected from ipilimumab (also known as YERVOY^{™}), pembrolizumab (also known as KEYTRUDA^{™}), nivolumab (also known as OPDIVO^{™}), and atezolizumab (also known as TECENTRIG^{™}).

In some embodiments, the immune checkpoint inhibitor is an inhibitor of CTLA4. In some embodiments, the immune checkpoint inhibitor is an antibody against CTLA4. In some embodiments, the immune checkpoint inhibitor is a monoclonal antibody against CTLA4. In some embodiments, the immune checkpoint inhibitor is a human or humanized antibody against CTLA4. In some embodiments, the immune checkpoint inhibitor reduces the expression or activity of one or more immune checkpoint proteins, such as CTLA4.

In some embodiments, the immune checkpoint inhibitor is an inhibitor of PD-1. In some embodiments, the immune checkpoint inhibitor is an antibody against PD-1. In some embodiments, the immune checkpoint inhibitor is a monoclonal antibody against PD-1. In some embodiments, the immune checkpoint inhibitor is a human or humanized antibody against PD-1. In some embodiments, the immune checkpoint inhibitor reduces the expression or activity of one or more immune checkpoint proteins, such as PD-1.

In some embodiments, the immune checkpoint inhibitor is an inhibitor of PD-L1. In some embodiments, the immune checkpoint inhibitor is an antibody against PD-L1. In some embodiments, the immune checkpoint inhibitor is a monoclonal antibody against PD-L1. In some embodiments, the immune checkpoint inhibitor is a human or humanized antibody against PD-L1. In some embodiments, the immune checkpoint inhibitor reduces the expression or activity of one or more immune checkpoint proteins, such as PD-L1. In some embodiments, the immune checkpoint inhibitor reduces the interaction between PD-1 and PD-L1.

In some embodiments, the immune checkpoint inhibitor is an inhibitor of PD-L2. In some embodiments, the immune checkpoint inhibitor is an antibody against PD-L2. In some embodiments, the immune checkpoint inhibitor is a monoclonal antibody against PD-L2. In some embodiments, the immune checkpoint inhibitor is a human or humanized antibody against PD-L2. In some embodiments, the immune checkpoint inhibitor reduces the expression or activity of one or more immune checkpoint proteins, such as PD-L2. In some embodiments, the immune checkpoint inhibitor reduces the interaction between PD-1 and PD-L2.

In some embodiments, the immune checkpoint inhibitor is an inhibitor of LAG-3. In some embodiments, the immune checkpoint inhibitor is an antibody against LAG-3. In some embodiments, the immune checkpoint inhibitor is a monoclonal antibody against LAG-3. In some embodiments, the immune checkpoint inhibitor is a human or humanized antibody against LAG-3. In some embodiments, the immune checkpoint inhibitor reduces the expression or activity of one or more immune checkpoint proteins, such as LAG-3.

In some embodiments, the immune checkpoint inhibitor is an inhibitor of B7-H4. In some embodiments, the immune checkpoint inhibitor is an antibody against B7-H4. In some embodiments, the immune checkpoint inhibitor is a monoclonal antibody against B7-H4. In some embodiments, the immune checkpoint inhibitor is a human or humanized antibody against B7-H4. In some embodiments, the immune checkpoint inhibitor reduces the expression or activity of one or more immune checkpoint proteins, such as B7-H4.

In some embodiments, the immune checkpoint inhibitor is an inhibitor of KIR. In some embodiments, the immune checkpoint inhibitor is an antibody against KIR. In some embodiments, the immune checkpoint inhibitor is a monoclonal antibody against KIR. In some embodiments, the immune checkpoint inhibitor is a human or humanized antibody against KIR. In some embodiments, the immune checkpoint inhibitor reduces the expression or activity of one or more immune checkpoint proteins, such as KIR.

In some embodiments, the immune checkpoint inhibitor is an inhibitor of TNFRSF4. In some embodiments, the immune checkpoint inhibitor is an antibody against TNFRSF4. In some embodiments, the immune checkpoint inhibitor is a monoclonal antibody against TNFRSF4. In some embodiments, the immune checkpoint inhibitor is a human or humanized antibody against TNFRSF4. In some embodiments, the immune checkpoint inhibitor reduces the expression or activity of one or more immune checkpoint proteins, such as TNFRSF4.

In some embodiments, the immune checkpoint inhibitor is an inhibitor of OX40L. In some embodiments, the immune checkpoint inhibitor is an antibody against OX40L. In some embodiments, the immune checkpoint inhibitor is a monoclonal antibody against OX40L. In some embodiments, the immune checkpoint inhibitor is a human or humanized antibody against OX40L. In some embodiments, the immune checkpoint inhibitor reduces the expression or activity of one or more immune checkpoint proteins, such as OX40L. In some embodiments, the immune checkpoint inhibitor reduces the interaction between TNFRSF4 and OX40L.

In some embodiments, the immune checkpoint inhibitor is an inhibitor of IDO-1. In some embodiments, the immune checkpoint inhibitor is an antibody against IDO-1. In some embodiments, the immune checkpoint inhibitor is a monoclonal antibody against IDO-1. In some embodiments, the immune checkpoint inhibitor is a human or humanized antibody against IDO-1. In some embodiments, the immune checkpoint inhibitor reduces the expression or activity of one or more immune checkpoint proteins, such as IDO-1.

In some embodiments, the immune checkpoint inhibitor is an inhibitor of IDO-2. In some embodiments, the immune checkpoint inhibitor is an antibody against IDO-2. In some embodiments, the immune checkpoint inhibitor is a monoclonal antibody against IDO-2. In some embodiments, the immune checkpoint inhibitor is a human or humanized antibody against IDO-2. In some embodiments, the immune checkpoint inhibitor reduces the expression or activity of one or more immune checkpoint proteins, such as IDO-2.

In some embodiments, the immune checkpoint inhibitor is an inhibitor of CEACAM1. In some embodiments, the immune checkpoint inhibitor is an antibody against CEACAM1. In some embodiments, the immune checkpoint inhibitor is a monoclonal antibody against CEACAM1. In some embodiments, the immune checkpoint inhibitor is a human or humanized antibody against CEACAM1. In some embodiments, the immune checkpoint inhibitor reduces the expression or activity of one or more immune checkpoint proteins, such as CEACAM1.

In some embodiments, the immune checkpoint inhibitor is an inhibitor of BTLA. In some embodiments, the immune checkpoint inhibitor is an antibody against BTLA. In some embodiments, the immune checkpoint inhibitor is a monoclonal antibody against BTLA. In some embodiments, the immune checkpoint inhibitor is a human or humanized antibody against BTLA. In some embodiments, the immune checkpoint inhibitor reduces the expression or activity of one or more immune checkpoint proteins, such as BTLA.

In some embodiments, the immune checkpoint inhibitor is an inhibitor of TIM3. In some embodiments, the immune checkpoint inhibitor is an antibody against TIM3. In some embodiments, the immune checkpoint inhibitor is a monoclonal antibody against TIM3. In some embodiments, the immune checkpoint inhibitor is a human or humanized antibody against TIM3. In some embodiments, the immune checkpoint inhibitor reduces the expression or activity of one or more immune checkpoint proteins, such as TIM3.

In some embodiments, the immune checkpoint inhibitor is an inhibitor of A2Ar. In some embodiments, the immune checkpoint inhibitor is an antibody against A2Ar. In some embodiments, the immune checkpoint inhibitor is a monoclonal antibody against A2Ar. In some embodiments, the immune checkpoint inhibitor is a human or humanized antibody against A2Ar. In some embodiments, the immune checkpoint inhibitor reduces the expression or activity of one or more immune checkpoint proteins, such as A2Ar.

In some embodiments, the immune checkpoint inhibitor is an inhibitor of VISTA protein. In some embodiments, the immune checkpoint inhibitor is an antibody against VISTA protein. In some embodiments, the immune checkpoint inhibitor is a monoclonal antibody against VISTA protein. In some embodiments, the immune checkpoint inhibitor is a human or humanized antibody against VISTA protein. In some embodiments, the immune checkpoint inhibitor reduces the expression or activity of one or more immune checkpoint proteins, such as VISTA protein.

### Biosimilar

The immunoconjugates of the invention will be effective with antibody constructs that are highly similar, or biosimilar, to the commercially available, or "innovator," antibody constructs.

### DAR Ratio

The immunoconjugates of the invention can provide any suitable, and desirable, DAR ratios.

The immunoconjugates shown with varying DAR ratios were all effective at activating myeloid cells and eliciting cytokine secretion. The data indicate that the immunoconjugates with varying DAR ratios were all superior at eliciting APC activation as CD40, CD86 and HLA-DR were expressed at higher levels in APCs stimulated with immunoconjugates as compared to those stimulated with the antibody alone. The immunoconjugates with varying DARs consistently induced the downregulation of CD14 and CD16 and increased expression of CD123, as compared to the antibody alone. From these studies, it is expected all DAR ratios will be effective at eliciting myeloid cell activation.

### Isotype Modification

The activity of the immunoconjugates of the invention can be modulated and often, improved, for the desired application by isotype modification.

Around 30% of human IgG is glycosylated within the Fab region, and the antibody in the immunoconjugates of the invention can contain an engineered Fab region with a non-naturally occurring glycosylation pattern. For example, hybridomas can be genetically engineered to secrete afucosylated mAb, desialylated mAb or deglycosylated Fc with specific mutations that enable increased FcRγIIIa binding and effector function.

Antibodies for forming immunoconjugates can contain engineered (i.e., non-naturally occurring) cysteine residues characterized by altered (e.g., enhanced) reactivity toward the reagents used for covalently bonding the adjuvant moieties to the antibodies. In certain embodiments, an engineered cysteine residue will have a thiol reactivity value in the range of 0.6 to 1.0. In many instances, the engineered antibody will be more reactive than the parent antibody.

In general, the engineered residues are "free" cysteine residues that are not part of disulfide bridges. The term "thiol reactivity value" is a quantitative characterization of the reactivity of free cysteine amino acids. As used herein, the term "thiol reactivity value" refers to the percentage of a free cysteine amino acid in an engineered antibody which reacts with a thiol-reactive reagent, converted to a maximum value of 1. For example, a cysteine residue in an engineered antibody which reacts in 100% yield with a thiol-reactive reagent, such as a maleimide, to form a modified antibody has a thiol reactivity value of 1.0. Another cysteine residue engineered into the same or different parent antibody which reacts in 80% yield with a thiol-reactive reagent has a thiol reactivity value of 0.8. Determination of the thiol reactivity value of a particular cysteine residue can be conducted by ELISA assay, mass spectroscopy, liquid chromatography, autoradiography, or other quantitative analytical tests.

Engineered cysteine residues can be located in the antibody heavy chains or the antibody light chains. In certain embodiments, engineered cysteine residues are located in the Fc region of the heavy chains. For example, amino acid residues at positions L-15, L-43, L-110, L-144, L-168 in the light chains of an antibody or H-40, H-88, H-119, H-121, H-122, H-175, and H-179 in the heavy chains of an antibody can be replaced with cysteine residues. Ranges within about 5 amino acid residues on each side of these positions can also be replaced with cysteine residues, i.e., L-10 to L-20; L-38 to L-48; L-105 to L-115; L-139 to L-149; L-163 to L-173; H-35 to H-45; H-83 to H-93; H-114 to H-127; and H-170 to H-184, as well as the ranges in the Fc region selected from H-268 to H-291; H-319 to H-344; H-370 to H-380; and H-395 to H-405, to provide useful cysteine engineered antibodies for forming immunoconjugates. Other engineered antibodies are described, for example, in U.S. Patents 7,855,275, 8,309,300, and 9,000,130.

In addition to antibodies, alternative protein scaffolds may be used as part of the immunoconjugates. The term "alternative protein scaffold" refers to a non-immunoglobulin derived protein or peptide. Such proteins and peptides are generally amenable to engineering and can be designed to confer monospecificity against a given antigen, bispecificity, or multispecificity. Engineering of an alternative protein scaffold can be conducted using several approaches. A loop grafting approach can be used where sequences of known specificity are grafted onto a variable loop of a scaffold. Sequence randomization and mutagenesis can be used to develop a library of mutants, which can be screened using various display platforms (e.g., phage display) to identify a novel binder. Site-specific mutagenesis can also be used as part of a similar approach. Alternative protein scaffolds exist in a variety of sizes, ranging from small peptides with minimal secondary structure to large proteins of similar size to a full-sized antibody. Examples of scaffolds include, but are not limited to, cystine knotted miniproteins (also known as knottins), cyclic cystine knotted miniproteins (also known as cyclotides), avimers, affibodies, the tenth type III domain of human fibronectin, DARPins (designed ankyrin repeats), and anticalins (also known as lipocalins). Naturally occurring ligands with known specificity can also be engineered to confer novel specificity against a given target. Examples of naturally occurring ligands that may be engineered include the EGF ligand and VEGF ligand. Engineered proteins can either be produced as monomeric proteins or as multimers, depending on the desired binding strategy and specificities. Protein engineering strategies can be used to fuse alternative protein scaffolds to Fc domains.

### Linker

Any suitable linker can be used in the context of the invention provided that that linker can be bound to the antibody through an ester. For example, the linker ("L") can have the following formula 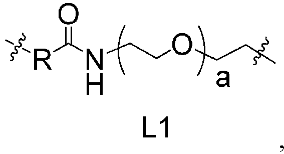 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units, and a is an integer from 1 to 40. In some embodiments, a is an integer from 1 to 20. In some embodiments, a is an integer from 1 to 10. In some embodiments, a is an integer from 1 to 5. In some embodiments, a is an integer from 1 to 3. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

The linker ("L") can have the following formula 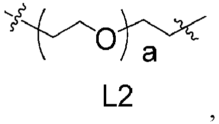 wherein a is an integer from 1 to 40. In some embodiments, a is an integer from 1 to 20. In some embodiments, a is an integer from 1 to 10. In some embodiments, a is an integer from 1 to 5. In some embodiments, a is an integer from 1 to 3.

The linker ("L") can also have the following formula 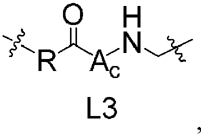 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units, each A is independently selected from any amino acid, and c is an integer from 1 to 20. In some embodiments, c is an integer from 1 to 10. In some embodiments, c is an integer from 1 to 5. In some embodiments, c is an integer from 1 to 2. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

The linker ("L") can also have the following formula 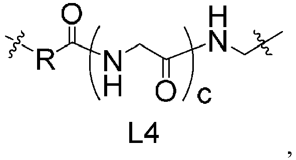 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units, and c is an integer from 1 to 20. In some embodiments, c is an integer from 1 to 10. In some embodiments, c is an integer from 1 to 5. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

The linker ("L") can also have the following formula 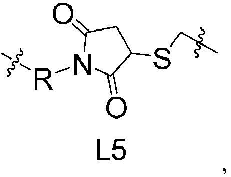 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

The linker ("L") can also have the following formula 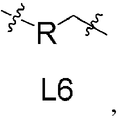 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

The linker ("L") can also have the following formula 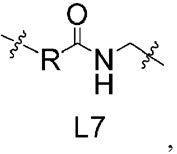 wherein R is optionally present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units. In certain embodiments, R is present and is a linear or branched, cyclic or straight, saturated or unsaturated alkyl, heteroalkyl, aryl, or heteroaryl chain comprising from 1 to 8 (i.e., 1, 2, 3, 4, 5, 6, 7, or 8) carbon units.

Linkers L1-L7 can be used bilaterally, meaning that the linker can be bound to the ester at either end, designated by the wavy line (" 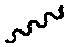 ").

### Ester in Conjugation Method

As previously discussed, there are many ways of forming an immunoconjugate. Each of the prior art methods suffers from downsides. The present method includes a one-step process which conjugates an adjuvant, modified to include a linker, to the lysine side chain of an antibody (compound of Formula II). This process is possible by using an ester. The ester can be any suitable ester capable of conjugation to a lysine residue of an antibody.

For example, the ester of Formula I can be an N-hydroxysuccinimide ("NHS") ester of the formula: 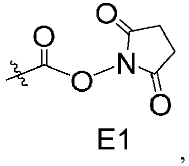 wherein the wavy line (" 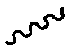 ") represents the point of attachment to the linker ("L").

The ester of Formula I can also be a sulfo-N-hydroxysuccinimide ester of the formula: 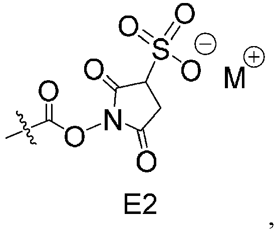 wherein M is any cation and the wavy line (" 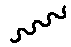 ") represents the point of attachment to the linker ("L"). For example, the cation counter ion ("M") can be a proton, ammonium, a quaternary amine, a cation of an alkali metal, a cation of an alkaline earth metal, a cation of a transition metal, a cation of a rare-earth metal, a main group element cation, or a combination thereof.

The ester of Formula I can also be a phenol ester of the formula: 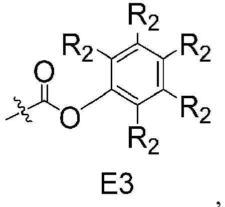 wherein each R₂ is independently selected from hydrogen, iodine, bromine, chlorine, or fluorine and the wavy line (" 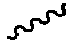 ") represents the point of attachment to the linker ("L").

The ester of Formula I can also be a phenol ester of the formula: 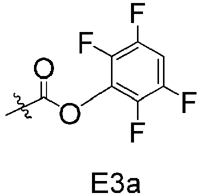 (tetrafluorophenyl) or 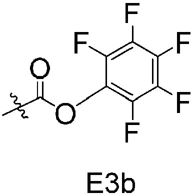 (pentafluorophenyl), wherein the wavy line (" 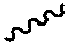 ") represents the point of attachment to the linker ("L").

Using a tetrafluorophenyl ("TFP") or pentafluorophenyl ("PFP") is especially effective in the methods of the invention.

The invention provides a method for producing an immunoconjugate, the method comprising combining one or more compounds of Formula I: 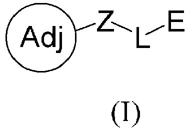 and an antibody of Formula II: 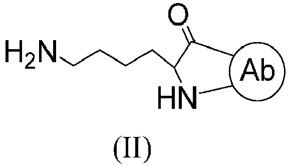 wherein Formula II is an antibody with residue one or more lysine residues of the antibody, 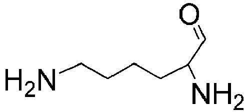 representing in an aqueous solution buffered at a pH of about 7.5 to about 9 until at least 33 mol% of the one or more compounds of Formula I is conjugated to the antibody of Formula II to provide the immunoconjugate of Formula III: 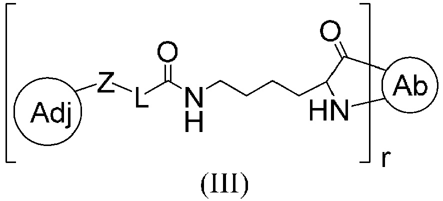 wherein
Adj is an adjuvant,
Z is -CH₂-, -C(O)NH-, -C(O)O-, or -C(O)-,
L is a linker,
E is an ester, and
r is the average number of adjuvants attached to the antibody and is a positive number up to about 8,
in a first buffered aqueous solution. In certain embodiments, Z is -CH₂- or -C(O)-.

The method comprises combining the one or more compounds of Formula I and the antibody of Formula II until at least about 33 mol% (e.g., at least about 35 mol%, at least about 36 mol%, at least about 37 mol%, at least about 38 mol%, at least about 39 mol%, at least about 40 mol%, at least about 41 mol%, at least about 42 mol%, at least about 43 mol%, at least about 44 mol%, at least about 45 mol%, at least about 46 mol%, at least about 47 mol%, at least about 48 mol%, at least about 49 mol%, or at least about 50 mol%) of the one or more compounds of Formula I is conjugated to the antibody of Formula II to provide the immunoconjugate of Formula III. In certain embodiments, the method comprises combining the one or more compounds of Formula I and the antibody of Formula II in the aqueous solution until at least 40 mol% of the one or more compounds of Formula I is conjugated to the antibody of Formula II to provide the immunoconjugate of Formula III. In other embodiments, the method comprises combining the one or more compounds of Formula I and the antibody of Formula II in the aqueous solution until at least 50 mol% of the one or more compounds of Formula I is conjugated to the antibody of Formula II to provide the immunoconjugate of Formula III.

In some embodiments, the method comprises combining the one or more compounds of Formula I and the antibody of Formula II for a period of at least about 1 hour (e.g., at least about 2 hours, at least about 3 hours, at least about 4 hours, at least about 5 hour, at least about 6 hours, at least about 8 hours, at least about 10 hours, at least about 12 hours, at least about 16 hours, at least about 20 hours, at least about 24 hours, or at least about 48 hours). Alternatively, or in addition, the method comprises combining the one or more compounds of Formula I and the antibody of Formula II for a period of no more than about 48 hours (e.g., no more than about 36 hours, no more than about 30 hours, no more than about 24 hours, no more than about 21 hours, no more than about 18 hour, no more than about 15 hours, or not more than about 12 hours). Thus, the method can comprise combining the one or more compounds of Formula I and the antibody of Formula II for a period bounded by any two of the aforementioned endpoints. For example, the method can comprise combining the one or more compounds of Formula I and the antibody of Formula II for a period of from about 1 hour to about 48 hours, from about 1 hour to about 36 hours, from about 1 hour to about 30 hours, from about 1 hour to about 24 hours, from about 1 hour to about 21 hours, from about 1 hour to about 18 hours, from about 1 hour to about 15 hours, from about 1 hour to about 12 hours, from about 2 hours to about 24 hours, from about 2 hours to about 15 hours, from about 2 hours to about 12 hours, from about 3 hours to about 24 hours, from about 3 hours to about 12 hours, from about 4 hours to about 24 hours, from about 5 hours to about 15 hours, from about 6 hours to about 48 hours, from about 6 hours to about 36 hours, from about 6 hours to about 30 hours, from about 6 hours to about 24 hours, from about 6 hours to about 21 hours, from about 6 hours to about 18 hours, from about 6 hours to about 15 hours, or from about 6 hours to about 12 hours.

The amount of the one or more compounds of Formula I conjugated to the one or more lysine residues of the antibody to form the immunoconjugate of Formula III can be assessed by any means known by one of skill in the art. In some embodiments, the immunoconjugate of Formula III is analyzed by LC/MS to determine amount of the one or more compounds of Formula I conjugated to the one or more lysine residues of the antibody. In some embodiments, the immunoconjugate of Formula III is buffer exchanged into PBS (pH 7.2) using a SEPHADEX^{™}-G25 column to remove excess small molecular weight impurities, and the drug to antibody ratio ("DAR") was measured at 40 °C, as a function of time. Without wishing to be bound by any particular theory, it is believed that once the DAR reaches a plateau for the immunoconjugate of Formula III incubated at 40 °C, the resulting DAR can be considered the amount of the one or more compounds of Formula I conjugated to the one or more lysine residues of the antibody.

The variable "r" refers to the average number of adjuvants attached to the antibody. Accordingly, the variable "r" can be used as a means of describing the drug to antibody ratio. Generally, r is a positive number up to about 8 (e.g., about 0.5, about 1, about 1.5, about 2, about 2.5, about 3, about 3.5, about 4, about 4.5, about 5, about 5.5, about 6, about 6.5, about 7, about 7.5, or about 8). As used herein, an immunoconjugate with an r value of about 0 to about 1 refers to a mixture of immunoconjugates comprising an amount of unconjugated antibody, such that the average drug to antibody ratio ("DAR"), or r, is from a positive number up to about 1. In some embodiments, r is the average number of adjuvants attached to the antibody and is a positive number up to about 4. In certain embodiments, r is the average number of adjuvants attached to the antibody and is from about 1 to about 4.

The desirable drug to antibody ratio can be determined by one of skill in the depending on the desired effect of the treatment. For example, a drug to antibody ratio of greater than 1.2 may be desired. In an embodiment, a drug to antibody ratio of greater than 0.2, 0.4, 0.6, 0.8, 1, 1.2, 1.4, 1.6. 1.8, 2.0, 2.2, 2.4, 2.6, 2.8, 3.0, 3.2, 3.4, 3.6, 3.8, 4.0, 5.0, 6.0, 7.0, 8.0, or 9.0 may be desired. In another embodiment, a drug to antibody ratio of less than 10.0, 9.0, 8.0, 7.0, 6.0, 5.0, 4.0, 3.8, 3.6, 3.4, 3.2, 3.0, 2.8, 2.6, 2.4, 2.2, 2.0, 1.8, 1.6, 1.4, 1.2, 0.8, 0.6, 0.4 or 0.2 may be desirable. The drug to antibody ratio can be assessed by any means known by one of skill in the art.

The method for producing an immunoconjugate of Formula III comprises combining the one or more compounds of Formula I and the antibody of Formula II in an alkaline aqueous solution (i.e., greater than a pH of 7). The method for producing an immunoconjugate of Formula III comprises combining the one or more compounds of Formula I and the antibody of Formula II in an aqueous solution that is buffered at a pH of about 7.5 to about 9, for example, about 7.6 to about 9, about 7.7 to about 9, about 7.8 to about to about 9, about 7.9 to about 9, about 8.0 to about 9, about 8 to about 8.9, about 8 to about 8.8, about 8 to about 8.7, about 8 to about 8.6, about 8.1 to about 8.6, about 8.2 to about 8.6, about 8.2 to about 8.5, or about 8.2 to about 8.4. Accordingly, the method for producing an immunoconjugate of Formula III comprises combining the one or more compounds of Formula I and the antibody of Formula II in an aqueous solution that is buffered at a pH of about 7.5, about 7.6, about 7.7, about 7.8, about 7.9, about 8, about 8.1, about 8.2, about 8.3, about 8.4, about 8.5, about 8.6, about 8.7, about 8.8, about 8.9, or about 9. In preferred embodiments, the method for producing an immunoconjugate of Formula III comprises combining the one or more compounds of Formula I and the antibody of Formula II in an aqueous solution that is buffered at a pH of about 8 to about 8.3.

The antibody of Formula II and the one or more compounds of Formula I are combined in any suitable aqueous solution buffer such that the aqueous solution has an alkaline pH. An exemplary list of suitable aqueous solution buffers or first buffered aqueous solution is TES buffered saline, HEPES buffered saline, DIPSO buffered saline, MOBS buffered saline, acetamidoglycine buffered saline, TAPSO buffered saline, TEA buffered phosphate buffered saline, POPSO buffered saline, HEPPSO buffered saline, EPS buffered saline, HEPPS buffered saline, tricine buffered saline, glycinamide buffered saline, glycylglycine buffered saline, HEPBS buffered saline, bicine buffered saline, TAPS buffered saline, AMPB buffered saline, phosphate buffered saline, borate buffered saline, and tris buffered saline. In preferred embodiments, the aqueous solution buffer or first buffered aqueous solution is borate buffered saline. In another preferred embodiment, the aqueous solution buffer or first buffered aqueous solution is phosphate buffered saline.

The method for producing an immunoconjugate of Formula III comprises combining the one or more compounds of Formula I and the antibody of Formula II in an aqueous solution at any suitable temperature. In some embodiments, the method for producing an immunoconjugate of Formula III comprises combining the one or more compounds of Formula I and the antibody of Formula II in an aqueous solution at a temperature of about 0 °C to about 50 °C, for example, about 0 °C to about 45 °C, about 0 °C to about 40 °C, about 5 °C to about to about 40 °C, about 10 °C to about 40 °C, about 15 °C to about 40 °C, about 20 °C to about 40 °C, about 25 °C to about 40 °C, or about 25 °C to about 35 °C. Accordingly, the method for producing an immunoconjugate of Formula III comprises combining the one or more compounds of Formula I and the antibody of Formula II in an aqueous solution at a temperature of about 1 °C, about 2 °C, about 3 °C, about 4 °C, about 5 °C, about 6 °C, about 7 °C, about 8 °C, about 9 °C, about 10 °C, about 11 °C, about 12 °C, about 13 °C, about 14 °C, about 15 °C, about 16 °C, about 17 °C, about 18 °C, about 19 °C, about 20 °C, about 21 °C, about 22 °C, about 23 °C, about 24 °C, about 25 °C, about 26 °C, about 27 °C, about 28 °C, about 29 °C, about 30 °C, about 31 °C, about 32 °C, about 33 °C, about 34 °C, about 35 °C, about 36 °C, about 37 °C, about 38 °C, about 39 °C, about 40 °C, about 41 °C, about 42 °C, about 43 °C, about 44 °C, about 45 °C, about 46 °C, about 47 °C, about 48 °C, about 49 °C, or about 50 °C. In preferred embodiments, the method for producing an immunoconjugate of Formula III comprises combining the one or more compounds of Formula I and the antibody of Formula II in an aqueous solution at a temperature of about 30 °C.

In some embodiments, the invention provides the immunoconjugate of Formula III in a first buffered aqueous solution. Typically, the first buffered aqueous solution is the same as the aqueous solution in which the antibody of Formula II and the one or more compounds of Formula I are combined. However, it will be understood by a person of ordinary skill in the art that the pH, temperature, and chemical composition of the first buffered aqueous solution may change slightly relative to the aqueous solution due to the combination of the antibody of Formula II and the one or more compounds of Formula I to form the immunoconjugate of Formula III.

Without wishing to be bound by any particular theory, conventional techniques for conjugating a chemical moiety to an antibody using an ester such as NHS, TFP, or PFP result in greater than about 5% conjugation to a tyrosine amino acid of the antibody. Unlike the amide bond formed upon conjugation to lysine amino acid residues, the ester bond formed upon conjugation to tyrosine is unstable. Accordingly, tyrosine conjugation results in decreased stability, lower DAR for a given number of equivalents of the one or more compounds of Formula I, and increased impurities (e.g., the acid formed upon hydrolysis of the tyrosine conjugated ester). The methods of the invention reduce the amount of initial tyrosine conjugation, thereby improving immunoconjugate stability, DAR for a given number of equivalents of the one or more compounds of Formula I, and reducing impurities, which facilitates immunoconjugate isolation.

Accordingly, in some embodiments, the methods of the invention provide more than a 5% reduction in tyrosine conjugation of the one or more compounds of Formula I to the antibody of Formula II in the first buffered aqueous solution relative to tyrosine conjugation of the one or more compounds of Formula I to the antibody of Formula II in the first buffered aqueous solution prepared by combining the one or more compounds of Formula I and the antibody of Formula II in an aqueous solution buffered at a pH of less than 7.5 using phosphate buffered saline, wherein all reaction conditions are identical except for the buffer. In other embodiment, the methods of the invention provide more than a 10%, 15%, 20%, 25%, 30%, 35%, 40%, 45%, 50%, 55%, 60%, 65%, or 70% reduction of tyrosine conjugation of the one or more compounds of Formula I to the antibody of Formula II in the first buffered aqueous solution relative to tyrosine conjugation of the one or more compounds of Formula I to the antibody of Formula II in the first buffered aqueous solution prepared by combining the one or more compounds of Formula I and the antibody of Formula II in an aqueous solution buffered at a pH of less than 7.5 using phosphate buffered saline, wherein all reaction conditions are identical except for the buffer. The level of tyrosine conjugation can be assessed by any means known by one of skill in the art or described herein.

Any suitable number of equivalents of the one or more compounds of Formula I can be combined with the antibody of Formula II to achieve the desirable drug to antibody ratio, so long as at least 33 mol% of the one or more compounds of Formula I is conjugated to the antibody of Formula II to provide the immunoconjugate of Formula III. Accordingly, about 0.1 equivalents or more of the one or more compounds of Formula I can be combined with the antibody of Formula II, for example, about 0.5 equivalents or more, about 1 equivalent or more, about 1.5 equivalents or more, about 2 equivalents or more, about 2.5 equivalents or more, about 3 equivalents or more, about 3.5 equivalents or more, about 4 equivalents or more, about 4.5 equivalents or more, about 5 equivalents or more, about 5.5 equivalents or more, about 6 equivalents or more, about 6.5 equivalents or more, about 7 equivalents or more, about 7.5 equivalents or more, about 8 equivalents or more, about 8.5 equivalents or more, about 9 equivalents or more, about 9.5 equivalents or more, about 10 equivalents or more, about 11 equivalents or more, about 12 equivalents or more, about 13 equivalents or more, about 14 equivalents or more, about 15 equivalents or more, about 16 equivalents or more, about 17 equivalents or more, about 18 equivalents or more, about 19 equivalents or more, or about 20 equivalents or more. Alternatively, or in addition, about 50 equivalents or less of the one or more compounds of Formula I can be combined with the antibody of Formula II, for example, about 45 equivalents or less, about 40 equivalent or less, about 35 equivalents or less, about 30 equivalents or less, about 25 equivalents or less, about 20 equivalents or less, about 18 equivalents or less, about 16 equivalents or less, about 14 equivalents or less, about 12 equivalents or less, about 10 equivalents or less, about 8 equivalents or less, about 6 equivalents or less, or about 4 equivalents or less. Thus, number of equivalents of the one or more compounds of Formula I combined with the antibody of Formula II can be bounded by any two of the aforementioned endpoints. For example, the number of equivalents of the one or more compounds of Formula I combined with the antibody of Formula II can be from about 0.1 to about 50, from about 1 to about 50, from about 1 to about 40, from about 1 to about 30, from about 1 to about 20, from about 2 to about 50, from about 2 to about 40, from about 2 to about 30, from about 2 to about 20, from about 3 to about 50, from about 3 to about 40, from about 3 to about 30, from about 3 to about 20, from about 4 to about 50, from about 4 to about 40, from about 4 to about 30, from about 4 to about 20, from about 6 to about 30, from about 6 to about 20, from about 8 to about 40, from about 8 to about 20, from about 10 to about 50, from about 10 to about 20, from about 12 to about 50, from about 12 to about 30, from about 12 to about 20, from about 4 to about 16, from about 8 to about 12, from about 1 to about 4, from about 1 to about 6, from about 1 to about 8, from about 1 to about 12, from about 1 to about 16, from about 2 to about 4, from about 2 to about 6, from about 2 to about 8, or from about 2 to about 12.

In some embodiments, the methods of the invention provide more than a 5% increase in yield of the immunoconjugate of Formula III compared to the conjugation methods of the prior art (for example, the SATA method). In another embodiment, the methods of the invention provide more than a 10%, 15%, 20%, 25%, 30%, 35%, 40%, 45%, 50%, 55%, 60%, 65%, or 70% increase in yield of the immunoconjugate of Formula III compared to the conjugation methods of the prior art (for example, the SATA method or the '528 synthesis method). The yield can be assessed by any means known by one of skill in the art.

In some embodiments, the methods of the invention provide more than a 5% reduction in detectable impurities prior to purification compared to the conjugation methods of the prior art (for example, the SATA method). In another embodiment, the methods of the invention provide more than a 10%, 15%, 20%, 25%, 30%, 35%, 40%, 45%, 50%, 55%, 60%, 65%, or 70% decrease in detectable impurities prior to purification compared to the conjugation methods of the prior art (for example, the SATA method or the '528 synthesis method). The level of detectable impurities can be assessed by any means known by one of skill in the art. The impurities can include, for example, free thiol groups, unconjugated antibody, unconjugated adjuvant, acid formed upon hydrolysis of unwanted conjugation sites, and linker molecules.

In some embodiments, the methods of the invention provide more than a 5% reduction in aggregation prior to purification compared to the conjugation methods of the prior art (for example, the ' 528 synthesis methods). In another embodiment, the methods of the invention provide more than a 10%, 15%, 20%, 25%, 30%, 35%, 40%, 45%, 50%, 55%, 60%, 65%, or 70% decrease in aggregation prior to purification compared to the conjugation methods of the prior art (for example, the ' 528 synthesis methods). The level of aggregation can be assessed by any means known by one of skill in the art.

In some embodiments, the methods of the invention provide more than a 5% reduction in aggregation prior to purification compared to the conjugation methods of the prior art (for example, the SATA method). In another embodiment, the methods of the invention provide more than a 10%, 15%, 20%, 25%, 30%, 35%, 40%, 45%, 50%, 55%, 60%, 65%, or 70% decrease in aggregation prior to purification compared to the conjugation methods of the prior art (for example, the SATA method). The level of aggregation can be assessed by any means known by one of skill in the art.

In some embodiments, the methods of the invention provide more than a 5% increase in yield of the immunoconjugate of Formula III compared to the conjugation method of combining the one or more compounds of Formula I and the antibody of Formula II in an aqueous solution buffered at a pH of less than 7.5 using phosphate buffered saline, wherein all reaction conditions are identical except for the buffer. In another embodiment, the methods of the invention provide more than a 10%, 15%, 20%, 25%, 30%, 35%, 40%, 45%, 50%, 55%, 60%, 65%, or 70% increase in yield of the immunoconjugate of Formula III compared to the conjugation method of combining the one or more compounds of Formula I and the antibody of Formula II in an aqueous solution buffered at a pH of less than 7.5 using phosphate buffered saline, wherein all reaction conditions are identical except for the buffer. The yield can be assessed by any means known by one of skill in the art.

In some embodiments, the methods of the invention provide more than a 5% reduction of detectable impurities in the immunoconjugate of Formula III in the first buffered aqueous solution relative to an immunoconjugate of Formula III in a first buffered aqueous solution prepared by combining the one or more compounds of Formula I and the antibody of Formula II in an aqueous solution buffered at a pH of less than 7.5 using phosphate buffered saline, wherein all reaction conditions are identical except for the buffer. In other embodiment, the methods of the invention provide more than a 10%, 15%, 20%, 25%, 30%, 35%, 40%, 45%, 50%, 55%, 60%, 65%, or 70% reduction of detectable impurities in the immunoconjugate of Formula III in the first buffered aqueous solution relative to an immunoconjugate of Formula III in a first buffered aqueous solution prepared by combining the one or more compounds of Formula I and the antibody of Formula II in an aqueous solution buffered at a pH of less than 7.5 using phosphate buffered saline, wherein all reaction conditions are identical except for the buffer. The level of detectable impurities can be assessed by any means known by one of skill in the art. The impurities can include, for example, free thiol groups, unconjugated antibody, unconjugated adjuvant, acid formed upon hydrolysis of unwanted conjugation sites, and linker molecules.

In some embodiments, the methods of the invention provide an improved drug to antibody ratio prior to purification for a given number of equivalents of the adjuvant/linker moiety compared to the conjugation method of combining the one or more compounds of Formula I and the antibody of Formula II in an aqueous solution buffered at a pH of less than 7.5 using phosphate buffered saline, wherein all reaction conditions are identical except for the buffer. In some embodiments, at least about 33% of the one or more compounds of Formula I become conjugated to a lysine residue of the antibody, for example, at least about 35%, at least about 40%, at least about 45%, or at least about 50%. Accordingly, the DAR of the immunoconjugate of Formula III in the first buffered aqueous solution will be at least about 33% of the number of equivalents of the one or more compounds of Formula I, for example, at least about 35%, at least about 40%, at least about 45%, or at least about 50%.

In one embodiment, the methods of the invention provide more than a 5% reduction in aggregation prior to purification compared to the conjugation method of combining the one or more compounds of Formula I and the antibody of Formula II in an aqueous solution buffered at a pH of less than 7.5 using phosphate buffered saline, wherein all reaction conditions are identical except for the buffer. In another embodiment, the methods of the invention provide more than a 10%, 15%, 20%, 25%, 30%, 35%, 40%, 45%, 50%, 55%, 60%, 65%, or 70% decrease in aggregation prior to purification compared to the conjugation method of combining the one or more compounds of Formula I and the antibody of Formula II in an aqueous solution buffered at a pH of less than 7.5 using phosphate buffered saline, wherein all reaction conditions are identical except for the buffer. The level of aggregation can be assessed by any means known by one of skill in the art.

As used herein, the term "aggregation" can refers to the formation of large, tangled clusters of denatured antibodies or immunoconjugates (i.e., aggregates). For example, the aggregates can be denatured antibodies formed from the scrambling of disulfide bonds.

In some embodiments, the method further comprises purifying the immunoconjugate of Formula III in the first buffered aqueous solution and/or second buffered aqueous solution. Purification of the immunoconjugate of Formula III in the first buffered aqueous solution and/or second buffered aqueous solution can occur by any suitable means. For example, the immunoconjugate of Formula III in the first buffered aqueous solution and/or second buffered aqueous solution can be purified by column chromatography (e.g., anion exchange chromatography, cation exchange chromatography, hydrophobic interaction chromatography, or mixed-mode chromatography), centrifugation, filtration, or crystallization.

In some embodiments, the method for producing an immunoconjugate of Formula III comprises storing the immunoconjugate of Formula III at a lower pH than the pH at which the immunoconjugate was synthesized. Without wishing to be bound by any particular theory, it is believed that the immunoconjugate is more stable in neutral (i.e., a pH of about 6.5 to about 7.5) and/or acidic aqueous solutions (i.e., less than a pH of 7). Accordingly, the immunoconjugate of Formula III can be buffer exchanged to a second buffered aqueous solution that is buffered at a pH of about 7.5 or less, for example, about 7.4 or less, about 7.3 or less, about 7.2 or less, about 7.1 or less, about 7 or less, about 6.9 or less, about 6.8 or less, about 6.7 or less, about 6.6 or less, about 6.5 or less, about 6.4 or less, about 6.3 or less, about 6.2 or less, about 6.1 or less, or about 6 or less. In certain embodiments, the immunoconjugate of Formula III is synthesized in an alkaline first buffered aqueous solution, and stored in an acidic second buffered aqueous solution.

In some embodiments, the method further comprises (iii) performing a buffer exchange on the first buffered aqueous solution of the immunoconjugate of Formula III to provide a second buffered aqueous solution buffered at a pH of about 6 to about 7.5. In certain embodiments, the method further comprises (iii) performing a buffer exchange on the first buffered aqueous solution of the immunoconjugate of Formula III to provide a second buffered aqueous solution buffered at a pH of about 7 to about 7.5. In preferred embodiments, the method further comprises (iii) performing a buffer exchange on the first buffered aqueous solution of the immunoconjugate of Formula III to provide a second buffered aqueous solution buffered at a pH of about 7.2 to about 7.4.

The immunoconjugate of Formula III can be buffer exchanged to any suitable second aqueous solution buffer. In some embodiments, the second aqueous solution buffer is neutral (i.e., a pH of about 6.5 to about 7.5) or acidic aqueous solutions (i.e., less than a pH of 7). An exemplary list of suitable second aqueous solution buffers is MOPS buffered saline, cholamine chloride buffered saline, MOPSO buffered saline, ACES buffered saline, PIPES buffered saline, bis-tris propane buffered saline, ACES buffered saline, ADA buffered saline, bis-tris methane buffered saline, MES buffered saline, phosphate buffered saline, citrate buffered saline, and BES buffered saline. In preferred embodiments, the second aqueous solution is buffered with phosphate buffered saline.

### Formulation and Administration of Immunoconjugate

The present disclosure provides a composition comprising the immunoconjugate as described herein. In some embodiments, the composition further comprises one or more pharmaceutically acceptable excipients. For example, the immunoconjugates as described herein can be formulated for parenteral administration, such as intravenous (IV) administration or administration into a body cavity or lumen of an organ. Alternatively, the immunoconjugates can be injected intratumorally. Formulations for injection will commonly comprise a solution of the immunoconjugate dissolved in a pharmaceutically acceptable carrier. Among the acceptable vehicles and solvents that can be employed are water and Ringer's solution, an isotonic sodium chloride. In addition, sterile fixed oils can conventionally be employed as a solvent or suspending medium. For this purpose, any bland fixed oil can be employed including synthetic monoglycerides or diglycerides. In addition, fatty acids such as oleic acid can likewise be used in the preparation of injectables. These solutions are sterile and generally free of undesirable matter. These formulations can be sterilized by conventional, well known sterilization techniques. The formulations can contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of the immunoconjugate in these formulations can vary widely, and will be selected primarily based on fluid volumes, viscosities, body weight, and the like, in accordance with the particular mode of administration selected and the patient's needs. In certain embodiments, the concentration of an immunoconjugate in a solution formulation for injection will range from about 0.1% (w/w) to about 10% (w/w).

In another aspect, the present disclosure provides an immunoconjugate or pharmaceutically acceptable salt thereof or the composition for use in treating cancer. The use includes comprising administering a therapeutically effective amount of an immunoconjugate a composition as described above to a subject in need thereof. For example, the use can include administering the immunoconjugate to provide a dose of from about 100 ng/kg to about 50 mg/kg to the subject. The immunoconjugate dose can range from about 10 µg/kg to about 5 mg/kg, or from about 100 µg/kg to about 1 mg/kg. The immunoconjugate dose can be about 100, 200, 300, 400, or 500 µg/kg. The immunoconjugate dose can be about 1, 2, 3, 4, 5, 6, 7, 8, 9, or 10 mg/kg. The immunoconjugate dose can also lie outside of these ranges, depending on the particular conjugate as well as the type and severity of the cancer being treated. Frequency of administration can range from a single dose to multiple doses per week, or more frequently. In some embodiments, the immunoconjugate is administered from about once per month to about five times per week. In some embodiments, the immunoconjugate is administered once per week.

### EXAMPLES

The following examples further illustrate the invention but, of course, should not be construed as in any way limiting its scope.

### Example 1. Synthesis of a TLR7/TLR8 Adjuvant

The following steps were utilized to prepare a TLR7/TLR8 adjuvant suitable for conjugation to an antibody to form an immunoconjugate of the invention.

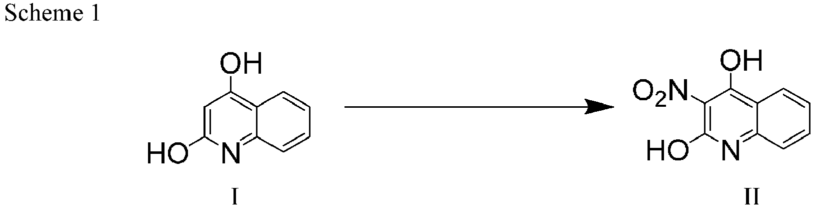

Chilled (0 °C) nitric acid (70%, 160 mL) was slowly added to the quinoline-2,4-diol I (100 g, 621 mmol) in 600 mL of glacial acetic acid and stirred in an ice bath. The mixture was removed from the ice bath and then stirred at room temperature for 2 hours. The mixture was then cooled to 0 °C. One liter of water was slowly added to the mixture to precipitate a yellow solid. The mixture was stirred vigorously for 15 minutes and then filtered. The solid was resuspended in 1 L of water, stirred vigorously for 15 minutes, and filtered again. The solid was resuspended in 1 L of water again while solid NaHCO₃ was slowly added to bring the pH to greater than 6, and then filtered overnight. The solid was resuspended in 750 mL of ethyl ether and stirred vigorously to create a fine suspension. The suspension was filtered, the solid was resuspended in 750 mL of ethyl ether again, stirred vigorously to create a fine suspension, and filtered under suction overnight. The process yielded 112 g II (88%) of a yellow solid.

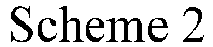

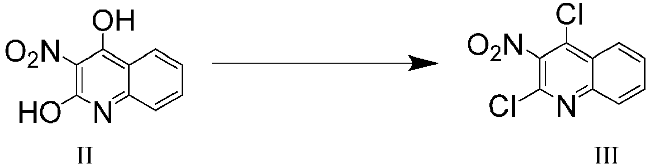

Triethylamine (60 mL, 44 g, 0.44 mol, 3 eq.) was slowly added to 300 mL of POCl₃ at room temperature. The mixture was heated to 90 °C. Nitro-diol 11 (30 g, 145 mmol, 1 eq.) was slowly added to the mixture in 1 g portions over 30 minutes. The mixture was then heated at 90 °C for 45 minutes. The mixture was then cooled to 0 °C and ice and cooled water was added to the mixture with vigorous stirring until it reached 1.2 L total volume. The mixture was stirred vigorously and then the aqueous mother liquor was decanted and 1 L of water was added to the remaining dark solid. The walls of the flask were scraped to remove the sticky solid and create a suspension, and then filtered. The solid was resuspended in one liter of water and then solid NaHCO₃ was slowly added until the pH was greater than 6. The solid was filtered out and then dissolved in 500 mL of EtOAc. The crude solid was filtered to remove insoluble impurities. The filtrate was washed with saturated NaHCO₃, water, and brine, and then separated. The organic layer was dried with Na₂SO₄, filtered, and concentrated under vacuum. The brown solid that was formed was triturated with 500 mL of 1:1 diethyl ether/hexanes and filtered. The tan solid III (22 g, 30 mmol, 62%) was used as is in the next reaction.

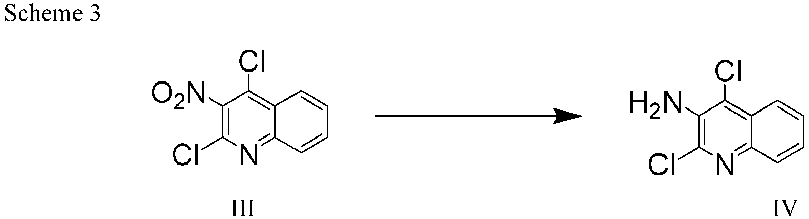

NiCl₂·6H₂O (0.35 g, 1.5 mmol, 0.05 eq) was added to a solution of nitro-dichloro compound III (22 g, 30 mmol, 1 eq.) in 600 mL of methanol and 60 mL of water at 0 °C. Sodium borohydride pellets (75 mmol, 2.5 eq.) were added and the reaction was stirred for 1 hour at 0 °C and then warmed to room temperature under agitation for an addition 1 hour. Glacial acetic acid was added in parts to neutralize any unreacted NaBH₄ until a pH of about 5 was obtained. The black solution was filtered through a bed of celite to remove the black insoluble material. The solvent was removed under vacuum. The dark brown solid was triturated with ether and then filtered to obtain a tan solid IV (13.3 g, 62 mmol, 69%) that was used in the next reaction.

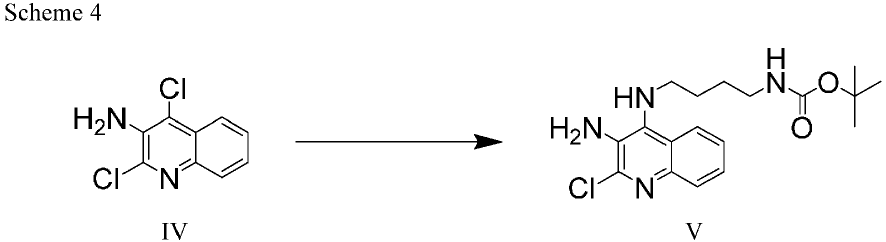

A solution of N-Boc-1,4-diaminobutane (12.8 g, 1.1 eq.) in 60 mL of DMF was added to a solution of amino-dichloro compound IV (13.3 g, 62 mmol, 1 eq.) and solid K₂CO₃ (17 g, 124 mmol, 2 eq.) in 250 mL of DMF at 0 °C over the course of 30 minutes. The reaction was then warmed to room temperature and stirred for an additional 30 minutes. Water (800 mL) was added to the mixture and stirred. The supernatant was poured off and the wet solid was dissolved in 500 mL of ethyl acetate. The solution was washed with 500 mL brine, separated, dried with Na₂SO₄, filtered, and concentrated under vacuum. The brown solid was triturated with 400 mL of diethyl ether and filtered to obtain a yellow solid V (14 g, 38 mmol, 62%) that was used as in the next reaction.

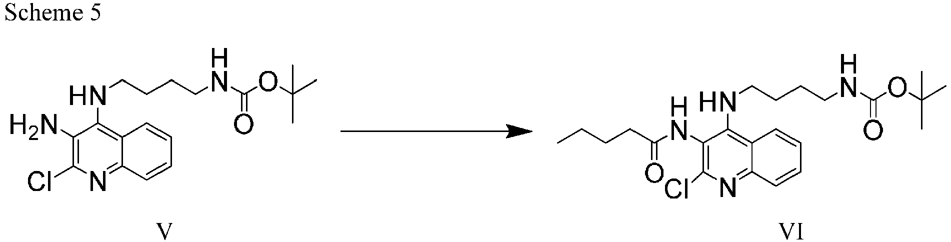

To a solution of amino compound V (14 g, 38 mmol, 1 eq.) in 250 mL DMF containing K₂CO₃ (13.8 g, 76 mmol, 2 eq.) stirring at 50 °C was added neat valeroyl chloride (5.5 mL, 5.5 g, 42 mmol, 1.2 eq). The mixture was stirred for 10 minutes at 50 °C then another addition of valeroyl chloride (2.8 mL, 2.8 g, 21 mmol, 0.6 eq.) was added. After stirring for an additional 20 minutes at 50 °C, ice was added, and then water to a final volume of 1 L. The mixture was stirred vigorously until a clear supernatant was formed. The supernatant was poured off and the crude solid was dissolved in 400 mL ethyl acetate and filtered through a bed of Celite. The filtrate was washed with 400 mL water, 400 mL brine, separated then dried (Na₂SO₄), filtered and concentrated. The solid was triturated with ether, filtered and suction dried. The brown solid obtained VI (11.4 g, 25 mmol, 70%) was used in the next reaction as is.

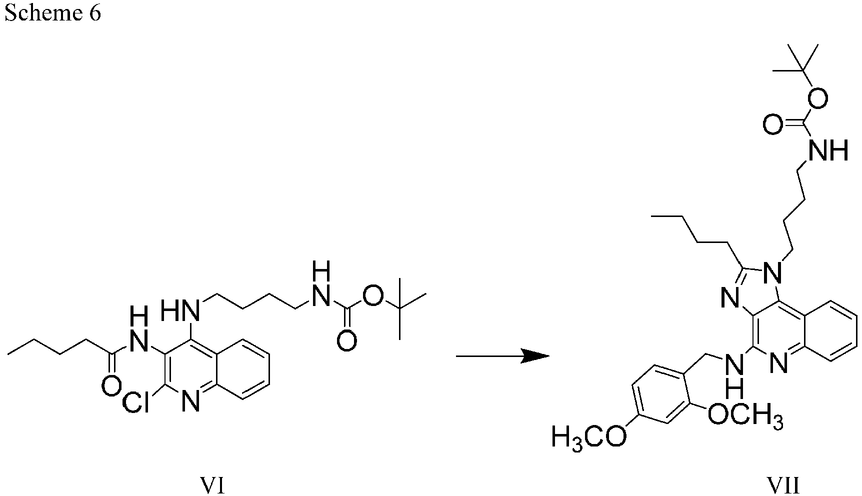

A mixture of amide VI (10.5 g, 23.3 mmol, 1 eq.) and 2-nitrobenzoic (3.0 g, 18.7 mmol. 0.8 eq.) and powdered molecular sieves (4Å, 10 g) in toluene was heated at 120 °C for 4 hours. The reaction was cooled to room temperature and the solvent was removed. In a 1 L beaker, the mixture was suspended syrup in 400 mL of ethyl acetate and filtered through celite. To the filtrate, 400 mL of water and 20 mL of saturated aqueous NaHCO₃ was added and then stirred vigorously for 15 minutes. The mixture was transferred to a separatory funnel and the aqueous layer was removed. The organic layer was washed with brine, dried with Na₂SO₄, filtered, and concentrated. The crude product was triturated with 100 mL of diethyl ether/ethyl acetate (3:1), filtered and dried under vacuum. The resultant tan solid VII (6.4 grams, 64%) was used as is in the next reaction.

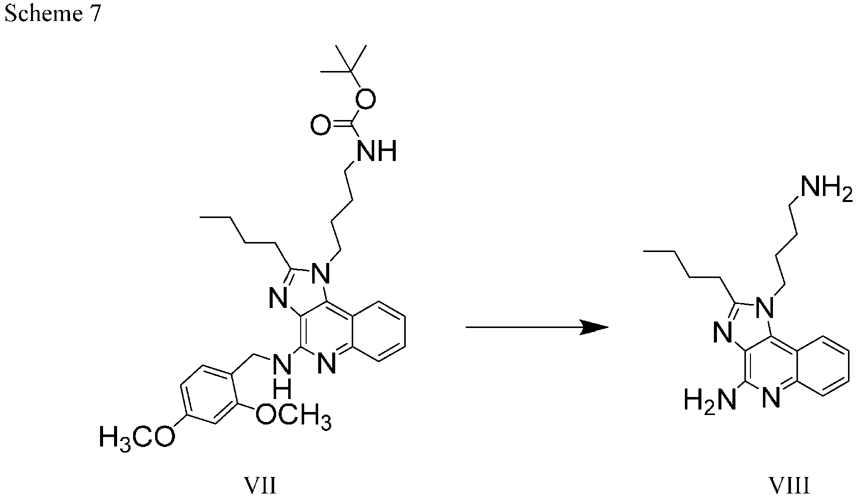

Neat 2,4-dimethoxybenzylamine (12.8 mL, 11.5 g, 69 mmol, 4.7 eq.) and granular K₂CO₃ (4.1 g, 30 mmol, 2 eq.) was added to solid chloroquinoline VII (6.4 g, 14.8 mmol, 1 eq.). The mixture was heated to 100 °C for 3 hours. The mixture was cooled and portioned between water and ethyl acetate (400 mL each) in a beaker. Acetic acid (7 mL) was added slowly and the mixture was stirred vigorously for 15 minutes. The pH of the aqueous layer was measured at about 5. The layers were separated, and the organic layer was washed with water, brine, dried (Na₂SO₄), filtered and concentrated to a syrup. The syrup was placed in an ice bath and 60 mL of concentrated HCl was slowly added. The mixture was stirred at room temperature for 15 minutes then heated to reflux for 3 hours. The solution was cooled in an ice bath and then diluted with 300 mL water. The solution was stirred in an ice bath while solid NaOH (29 g, 725 mmol) was slowly added until a basic pH was achieved. The solution was warmed to room temperature and stirred vigorously. Solid NaCl was added until a saturated solution was achieved. This aqueous layer was washed 3 times with 400 mL of 10% isopropanol/dichloromethane. The combined organic layers were dried with Na₂SO₄, filtered, and concentrated. The resultant brown syrup was filtered through a 30 g silica plug packed with ethyl acetate. The plug was eluted with ethyl acetate until less polar impurities were removed, then with 10% methanol/dichloromethane until the product was completely eluted. After concentration, a brown solid VIII was obtained (3.7 grams, 12 mmol, 80% for two steps) was obtained. This material was greater than 90-95% pure by LC/MS.

### Example 2. Synthesis of Immunoconjugate BB5 with a Tetrafluorophenyl ("TFP") Ester

A 20 mM stock solution of TFP activated adjuvant XI was prepared in accordance with Schemes 8, 9, and 10.

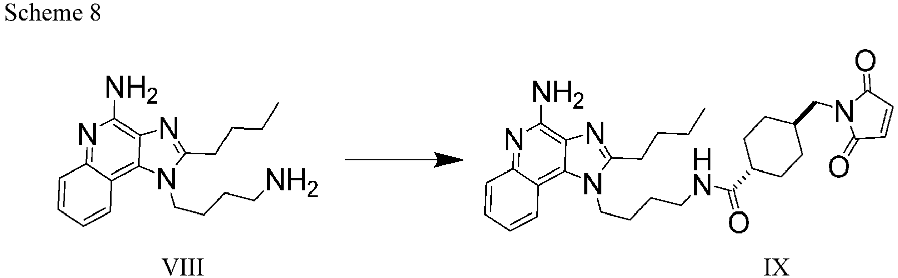

This example provides guidance on synthesis of an immunoconjugate using the TFP ester method. Compound VIII (311 mg, 1 mmol) was dissolved in 10 mL of dimethylformamide (DMF) and then 2 molar equivalents of diisopropylethylamine (DIPEA) was added. An SMCC linker (1.5 mmol) was dissolved in 10 mL of dichloromethane and added in one portion to VIII. The reaction was stirred overnight at 20 °C and concentrated to dryness via rotary evaporation. The crude product IX was purified on a silica gel using a Buchi flash chromatography system loaded with a 12 g disposable cartridge and eluted with a gradient of 0-10% methanol over 15 minutes. Pure fractions were combined and evaporated to dryness to provide160 mg of a pale yellow solid IX.

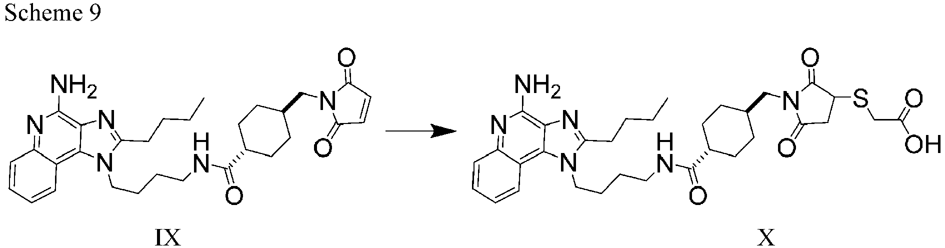

Compound IX (0.1 mmol, 53 mg) was dissolved in 10 mL of dichloromethane and then 2 equivalents of thioacetic acid were added at one time. The mixture was concentrated to dryness under vacuum and the residue was washed three times with 5 mL of diethyl ether.

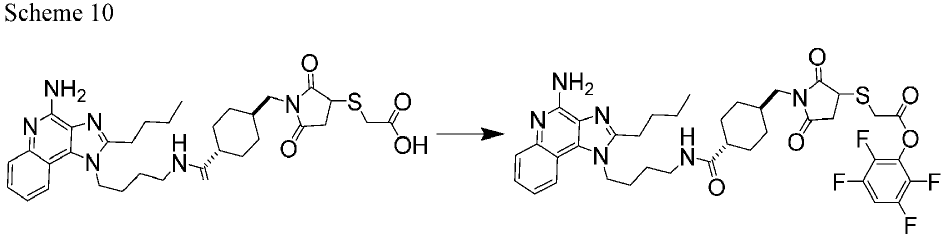

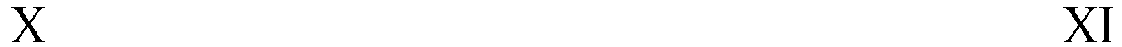

Compound X (6.2 mg, 0.01 mmol) was dissolved in 2 mL of THF and then 5 mg of tetrafluorophenol was added. Then 5 mg of dicyclohexylcarbodiimide (DCC) was added. The mixture was stirred overnight at room temperature and then concentrated to dryness under vacuum. The crude product XI was purified via flash chromatography on silica gel (4 gram prepacked column) and eluted with 0-10% MeOH in dichloromethane. Pure fractions were combined and evaporated to provide 3.6 mg of pure XI (confirmed by LC/MS). The TFP ester XI was then used in the antibody conjugation step below.

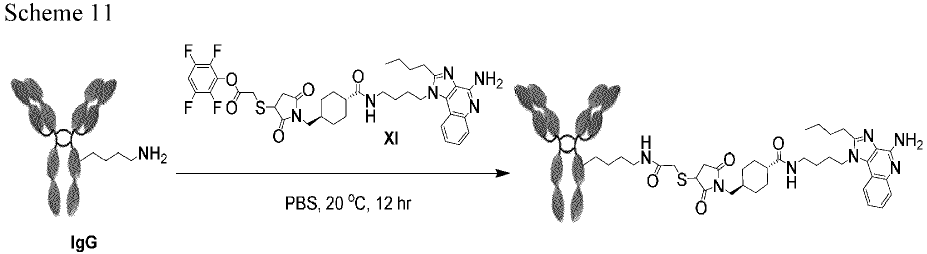

An IgG1 antibody (specifically, the anti-CD20 antibody rituximab) was buffer exchanged into PBS at a pH of 7.2 and diluted to 10 mg/mL (66 µM). The TFP activated adjuvant, XI, was added to DMSO and 6 molar equivalents (relative to IgG) was added to 1 mL of the antibody solution (10 mg) in one portion. The mixture was inverted several times to mix and incubated overnight at 20 °C. The resulting immunoconjugate ("BB5") was purified via buffer exchange into PBS (pH 7.2) using a PD10 column (SEPHADEX^{™}-G25) size exclusion chromatography column. Pure fractions were pooled and the concentration determined by measuring the absorbance at 280 nm on a nano-drop spectrophotometer. The yield was 8 milligram or approximately 80% based on recovered protein. The immunoconjugate product was sterile filtered through a 2 µm syringe filter and stored at 4 °C until needed.

Characterization of the resulting immunoconjugate's DAR was performed via liquid chromatography-mass spectrometry ("LC/MS") analysis on a UPLC system (Waters Aquity) equipped with a Xevo XS QToF mass spectrometer detector. Analysis was performed via injection of 5 µg of the immunoconjugate onto a BEH200 C4 column (2.1 mm diameter x 50 mm length) eluted with a 10-90% gradient of acetonitrile:water over 4 minutes.

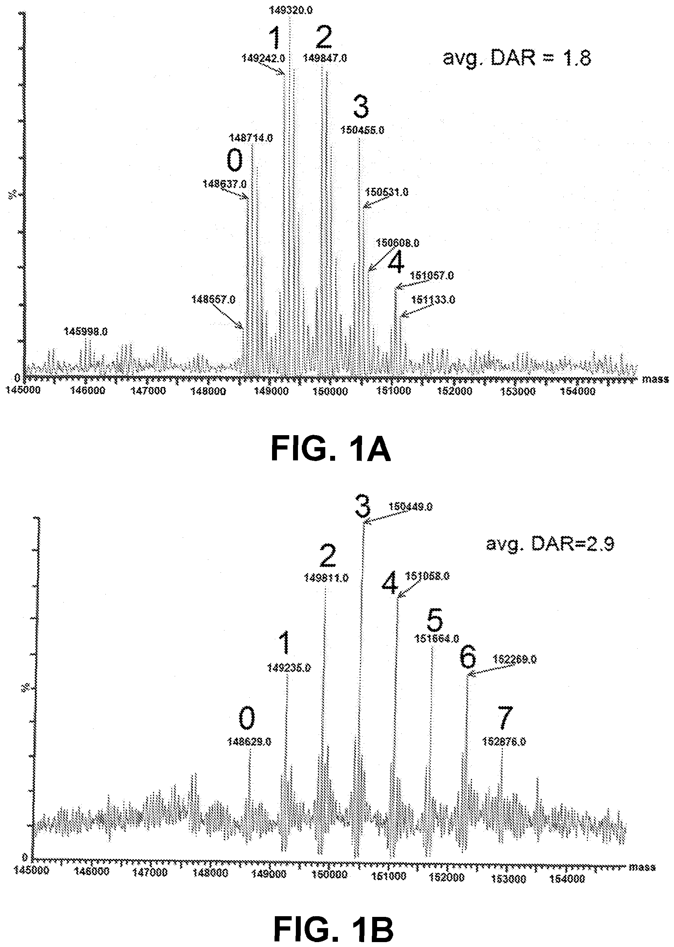

The analysis indicated that the immunoconjugates synthesized via the TFP method demonstrated higher DAR than the immunoconjugate synthesized using the SATA method. In addition, the TFP method yielded immunoconjugates with reduced amounts of unconjugated antibody (only about 5%) compared to the SATA synthesis method (about 20%) (compare Figures 1A and 1B).

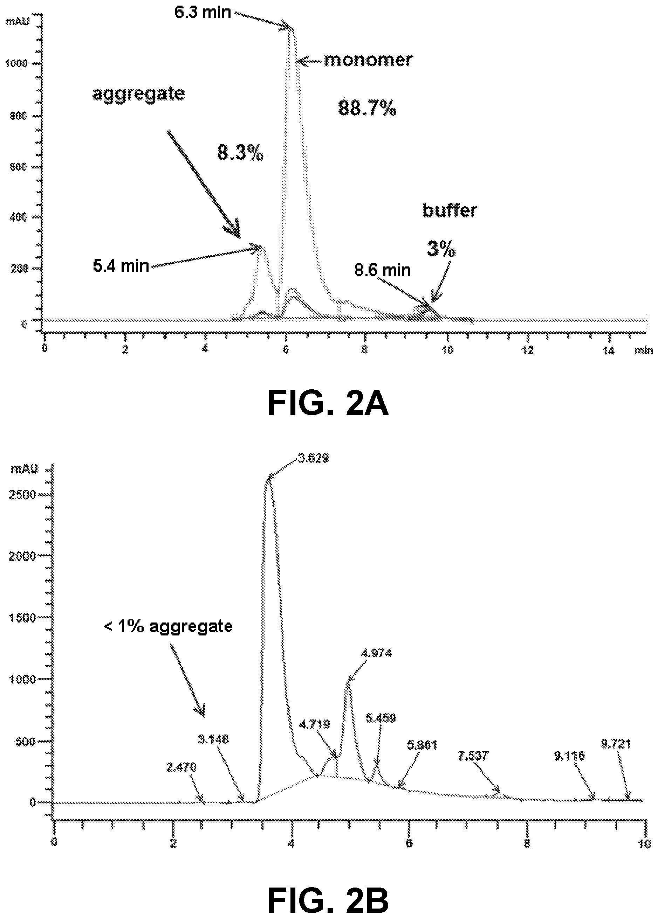

Size exclusion chromatography ("SEC") analysis of BB5 was performed to determine the monomeric purity. Analysis was performed on a BEH200 SEC column eluted with PBS (pH 7.2) and 0.2 mL/min. The immunoconjugate BB5 synthesized using the TFP active ester method contained less than 2% of high molecular weight aggregate (Figure 2B) compared to greater than 8% aggregate observed when the SATA method was used (Figure 2A).

### Example 3. Synthesis of Immunoconjugate BB1 with a Pentafluorophenyl ("PFP") Ester

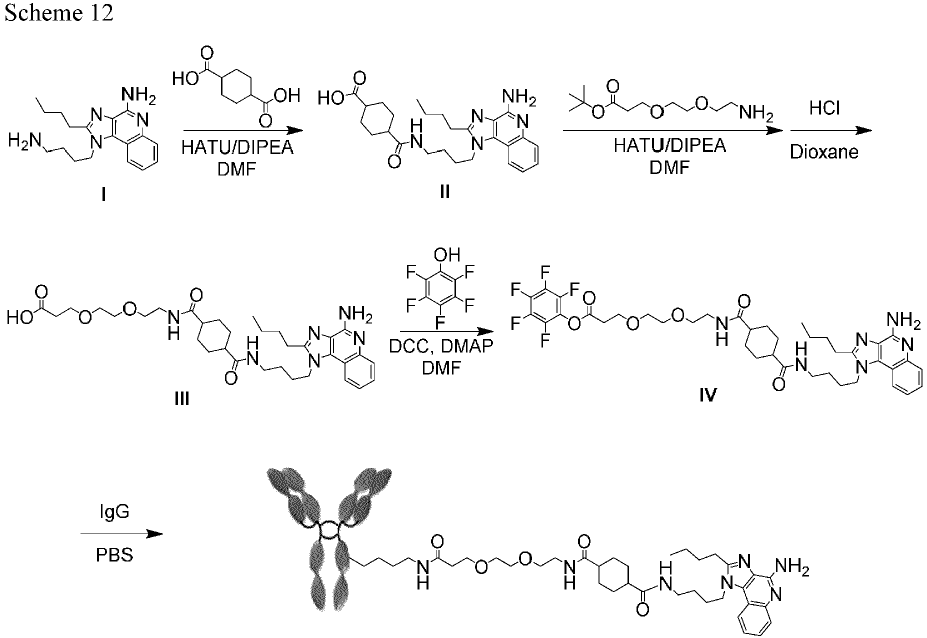

This example provides guidance on synthesis of an immunoconjugate using the PFP ester method. Ester modification of the adjuvant and conjugation of the modified adjuvant to the antibody is shown above in Scheme 12. Cyclohexane trans-1,4-dicarboxylate (1 g) was dissolved in 10 mL of dimethylformamide ("DMF") and 1-[bis(dimethylamino)methylene]-1H-1,2,3-triazolo[4,5-b]pyridinium 3-oxid hexafluorophosphate) ("HATU") (1 mmol) was added followed by 1 mL of *N*-ethyl-*N-*(propan-2-yl)propan-2-amine ("DIPEA"). Compound 1 (311 mg) was added and the mixture stirred overnight at 20 °C. The reaction mixture was diluted with 50 mL of dichloromethane ("DCM") and washed with 20 mL of IN HCl. The DCM layer was evaporated to dryness and the product purified on silica gel eluted with 0-10% MeOH in DCM containing 1% acetic acid. Pure fractions were concentrated to provide 220 mg of purified acid II. Compound II (100 mg) was dissolved in THF and 100 mg of HATU was added followed by 200 µL of DIPEA. Two equivalents of amino-PEG2-tertbutyl-carboxylate was added and stirred for one hour at 20 °C. The mixture was concentrated to dryness and 10 milliliters of 4N HCl in dioxane was added. The mixture was concentrated to dryness and the crude product III was purified by prep HPLC to provide 40 mg of compound III.

Compound III was converted to PFP ester IV as described below. Compound III (35 mg) was added to 50 mg of PFP in 5 mL THF and 5 mL DMF was added followed by 20 mg of DCC. DMAP (2-3 mg) was added and the solution was stirred overnight at 20 °C. The reaction was concentrated and purified by flash chromatography (eluted with 0-10% MeOH) to provide 17 mg of PFP ester IV after lyophillization from 1:2 acetonitrile water.

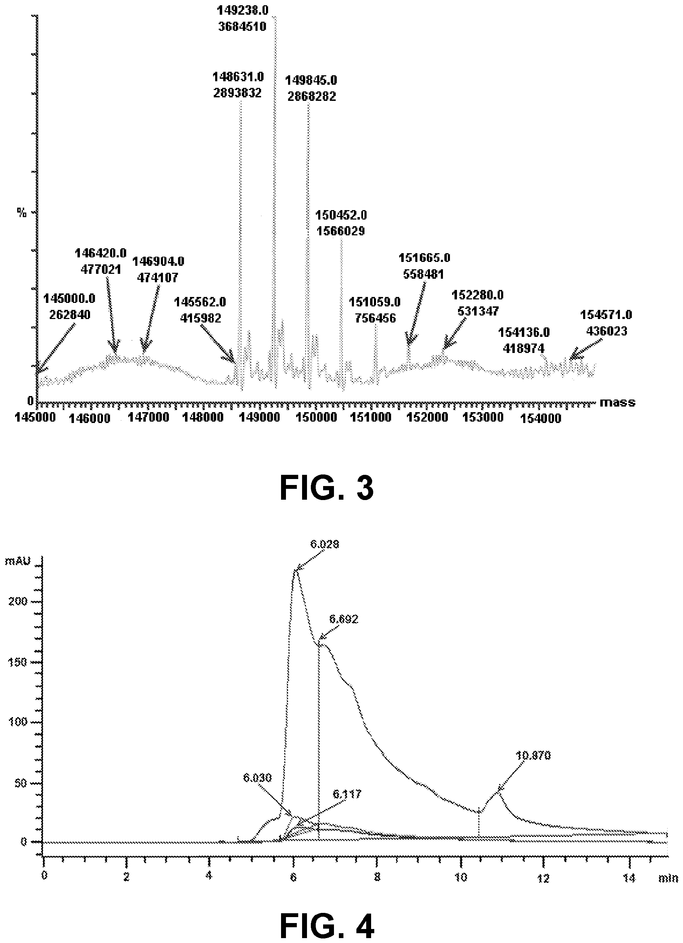

PFP ester IV (6 molar eq. relative to IgG) was added to 20 mg of an IgG antibody (specifically, the anti-CD20 antibody rituximab) (10 mg/mL in PBS) and incubated at 37 °C overnight. The resulting immunoconjugate BB1 was buffer exchanged into PBS (pH 7.2) to remove excess small molecular weight reagent and the concentration determined on the nanodrop. The yield was 15 mg of immunoconjugate (75% yield). The product was stored at 4 °C. A DAR of 2.2 was determined via LC/MS analysis. Besides the desirable DAR and high yield, the product also had few impurities as determined by SEC analysis (see Figures 3 and 4).

### Example 4. Synthesis of Immunoconjugate BB4 with a NHS Ester

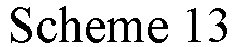

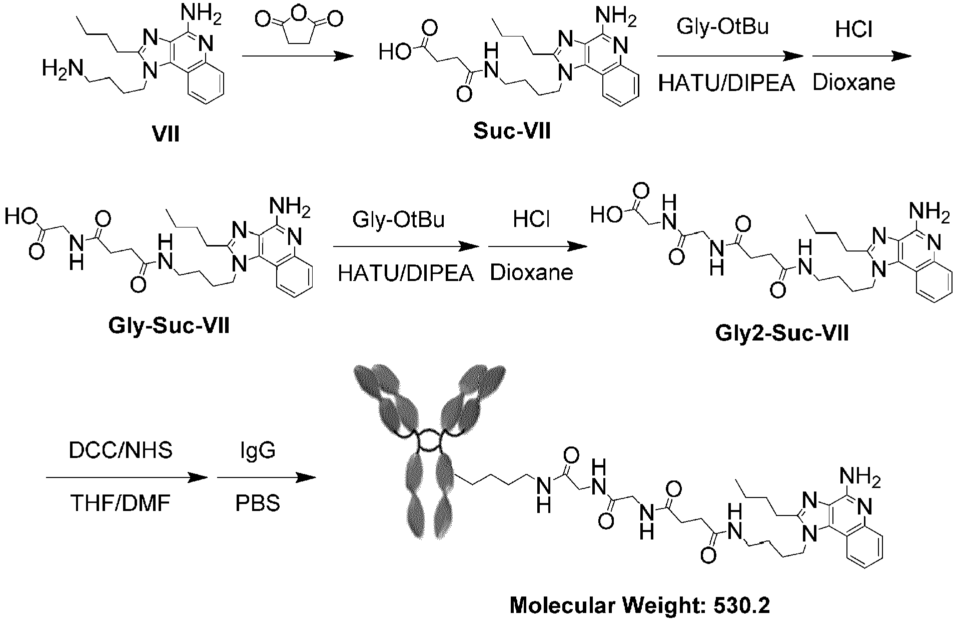

Ester modification of the adjuvant and conjugation of the modified adjuvant to the antibody is shown above in Scheme 13. Compound VII (150 mg) was dissolved in 20 mL of tetrahydrofuran ("THF") and 10 mL of aqueous, saturated sodium bicarbonate was added. Then, 50 mg of succinic anhydride was added in one portion and the mixture was stirred for one hour at room temperature. Twenty milliliters of IN HCl was added slowly and the mixture was extracted with 2 × 50 mL of dichloromethane. The combined organic extracts were evaporated to dryness. The crude product (Sue-VII) was purified on a 4 gram silica gel column eluted with 0-15% MeOH (1% acetic acid) over 15 minutes. Pure fractions were combined and evaporated to provide 190 mg of pure VII-Suc.

Compound VII-Suc (150 mg) was dissolved in 10 mL of DMF and 1 equivalent of HATU was added followed by 2 equivalents of DIPEA. 1.5 equivalents of glycine-OtBu were added and stirred overnight. The DMF was evaporated and the residue treated with 5 mL of IN HCl in dioxane for 30 minutes. The solvent was evaporated and the crude Gly-Suc-VII was flash purified on a 4 gram silica gel column eluted with 0-10% MeOH over 10 minutes. Evaporation of pure fractions provided 110 mg of Gly-Suc-VII; the pure material was dissolved in DMF and the above process was repeated to provide 60 mg of pure Gly2-Suc-VII.

The pure Gly2-Suc-VII (30 mg) was dissolved in 5 mL of DMF and 1.5 equivalents of NHS was added followed by 5 mL of THF. DCC (1.5 equivalents) was added and the mixture was stirred overnight at room temperature. The solvent was evaporated and the crude NHS ester was flash purified on a silica gel eluted with 0-10% MeOH in DCM over 10 minutes. Pure fractions (determined by TLC) were combined and evaporated to provide 1 mg of pure NHS-Gly2-Suc-VII after lyophilization from acetonitrile water.

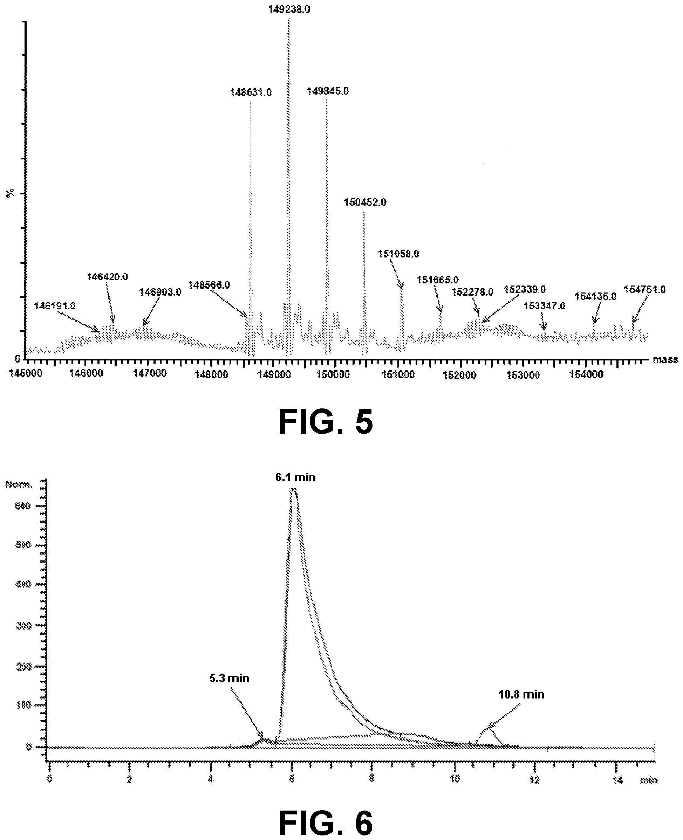

The pure NHS ester was dissolved in DMSO to make a 20 mM solution and 6 eq. was added to 2 mL of an IgG antibody (specifically, the anti-CD20 antibody rituximab) (10 mg/mL in PBS). The conjugation reaction was incubated at room temperature overnight and buffer exchanged into fresh PBS to remove excess adjuvant. The purified immunoconjugate BB4 was sterile filtered and stored at 4 °C. The yield was about 16 mg. Besides having a high yield, the LC/MS analysis showed high levels of purity, low levels of aggregation, and a desirable DAR ratio (see Figures 5 and 6).

### Example 5. Synthesis of Immunoconjugate BB2 with a TFP Ester

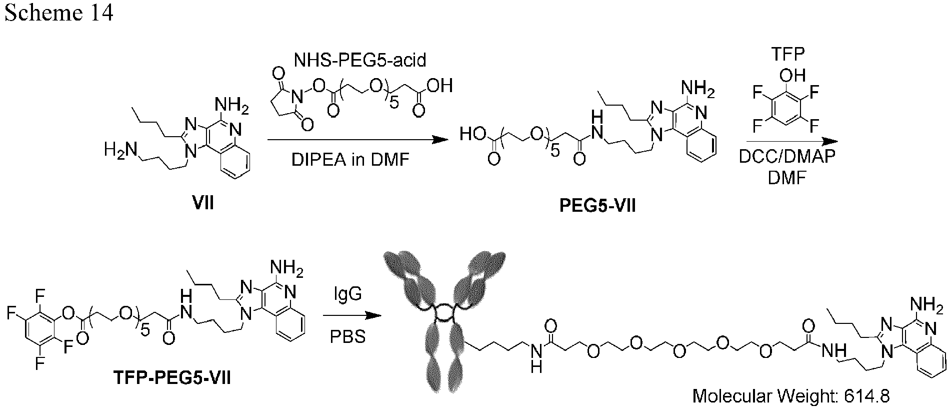

This example provides guidance on synthesis of an immunoconjugate with a different linker using the TFP ester method. Ester modification of the adjuvant and conjugation of the modified adjuvant to the antibody is shown above in Scheme 14. Compound VII (311 mg, 1 mmol) was dissolved in 10 mL of DMF and then 0.3 mL of DIPEA was added. The NHS-PEG5-acid (1.2 equivalents) was dissolved in 5 mL of dichloromethane and added to compound VII in one portion. The mixture was stirred overnight at room temperature and then concentrated to dryness. The crude residue was purified via silica gel chromatography on a 4 gram column eluted with 0-10% MeOH in DCM containing 1% acetic acid over 10 minutes to provide 260 mg (57% yield) of PEGS-VII after concentration of the pure fractions.

PEG5-VII (50 mg) was dissolved in 10 mL DMF and 1.5 eq. of TFP was added followed by 1.2 eq. DCC and 5 mg of DMAP. The reaction was stirred overnight, concentrated to dryness and purified on silica gel 4 gram column eluted with 0-10% MeOH in DCM to provide 35 mg of pure TFP-PEG5-VII after lyophilization from 1:2 acetonitrile water.

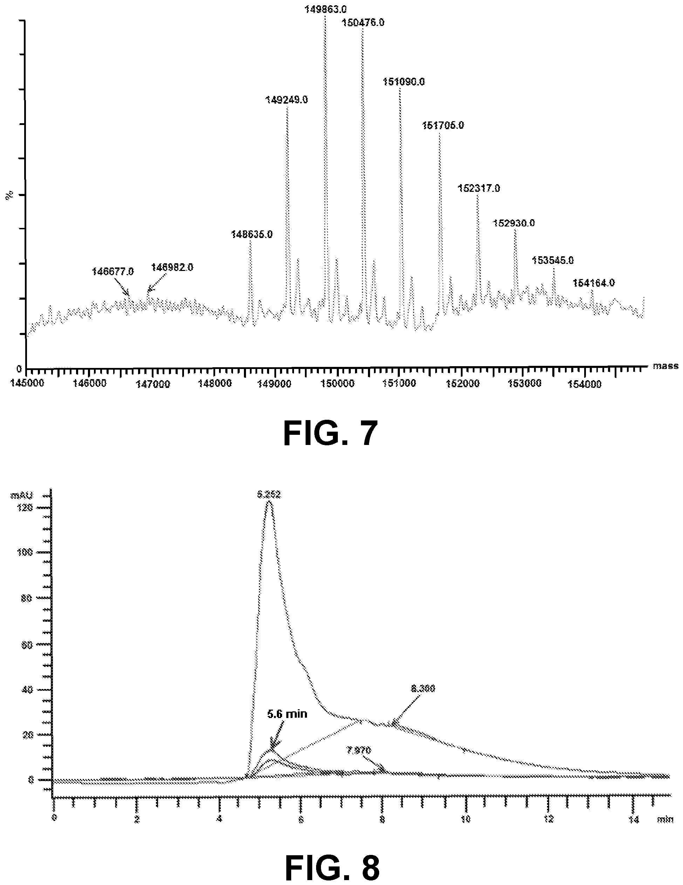

The TFP ester (TFP-PEG5-VII) was dissolved in DMSO to make a 20 mM stock solution and added to 20 mg of an IgG antibody (specifically, the anti-CD20 antibody rituximab) in PBS at 10 mg/mL. The conjugation reaction was allowed to proceed overnight at room temperature. The resulting immunoconjugate was buffer exchanged (GE, PD10 desalting column) into PBS at pH 7.4. The purified immunoconjugate was sterile filtered using a 2 µm syringe filter and stored at 4 °C. LC/MS analysis confirmed that the process provided a DAR of 2.9 adjuvants per antibody (see Figure 7). SEC analysis indicated minimal amounts of aggregate (i.e., less than 2%) (see Figure 8).

### Example 6. Synthesis of Another TLR7/TLR8 Adjuvant

This example provides guidance on how to synthesize another TLR7/8 adjuvant. Compound XIV was synthesized starting from compound VI of Scheme 6 of Example 1.

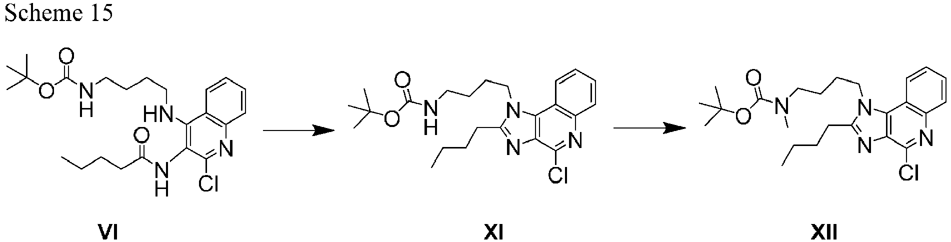

Compound VI (2 g) was dissolved in toluene with 20% dry acetic acid and heated to 75 °C overnight. The solvent was removed under vacuum to provide 2 grams of crude compound XI. Compound XI was used without further purification. Compound XI (2 g) was dissolved in 20 mL DMF and 1.2 equivalents of NaH (50% dispersion) was added slowly and the mixture was stirred for 30 minutes at room temperature. Methyl iodide (2 equivalents) was added in one portion and the reaction mixture was stirred overnight at room temperature. The reaction was concentrated to dryness and the product purified via flash chromatography. The product was eluted with a gradient of 0-10% MeOH in dichloromethane over 15 min. Pure fractions were combined and concentrated to yield 1 g of compound XII (50% yield for 2 steps).

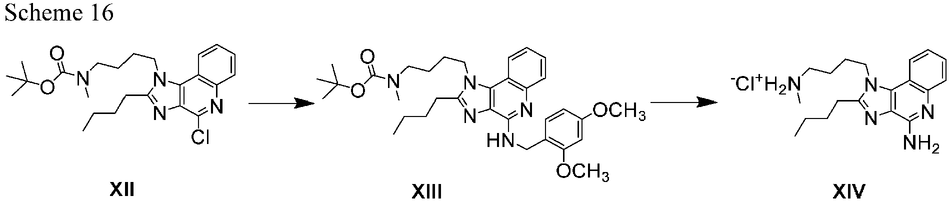

Compound XII (10 g) was dissolved in 10 mL of neat dimethoxybenzylamine ("DMBA") and heated to 120 °C for 3 hours. The reaction mixture was cooled and diluted with 100 mL of ethyl acetate. The resulting solution was washed two times with 10% citric acid in water and once with water to remove excess DMBA. The organic layer was dried over MgSO4 and concentrated under vacuum to provide crude compound XIII as a brown oil. The crude DMB derivative, compound XIII, was dissolved in dichloromethane and 2 mL of 4N HCl in dioxane was added. After 2 hours, the reaction mixture was concentrated to dryness and the crude HCl salt compound XIV was dissolved in 3 mL of methanol. Ethyl ether (20 mL) was added slowly with stirring to the crude solution and a white precipitate formed. The reaction was filtered and the white solid product was washed twice with 10 mL ethyl ether and dried under vacuum to provide 4 gram of HCl salt compound XIV. LC/MS analysis confirmed the correct molecular weight (M/z = 326.5) and a purity of greater than 95%.

### Example 7. Synthesis of Immunoconjugate BB6 with a TFP Ester

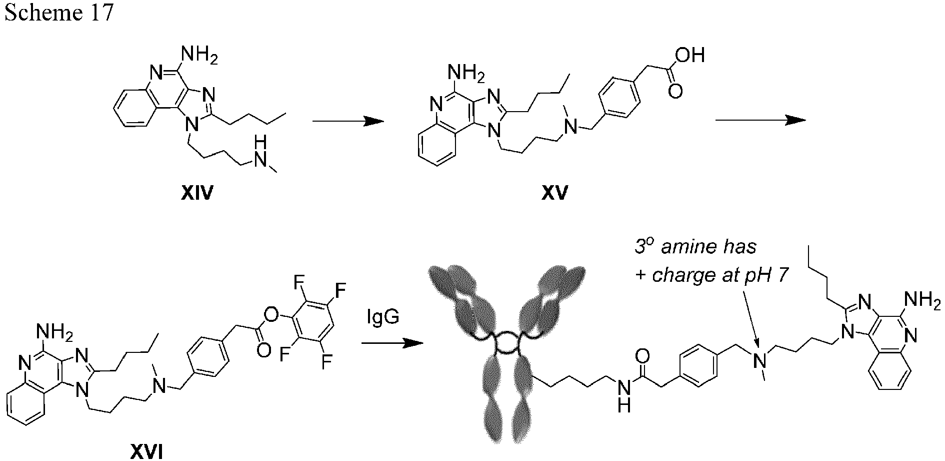

This example provides guidance on synthesis of an immunoconjugate that contains an aryl tertiary amine linker using the TFP ester method. Compound XIV (300 mg) of Example 6 was dissolved in THF (10 mL) and 1.2 eq. of NaH (50% dispersion) was added. The mixture was stirred for 15 minutes and 2 equivalents of 4-bromomethylphenyl acetic acid was added. The reaction was stirred overnight at room temperature and concentrated to dryness. One mL of acetic acid was added, and the product was purified by preparative HPLC on a C-18 column eluted with a gradient of 10-90% acetonitrile in water (0.1% TFA) over 20 minutes to provide 165 mg of purified phenylacetic acid compound XV.

Compound XV (50 mg) was dissolved in dichloromethane/dimethylformamide (5 mL, 1:1) and 2 equivalents of TFP was added followed by 1.5 equivalents of 1-ethyl-3-(3-dimethylaminopropyl)carbodiimide ("EDCI"). The reaction was stirred overnight at room temperature and the product purified via flash chromatography on a 4 gram silica gel column eluted with 0-10% isopropanol over 10 minutes. Pure fractions were concentrated and lyophilized from 30% acetonitrile water to provide 21 mg of purified TFP ester compound XVI as a pale yellow solid. The molecular weight and purity were confirmed by LC/MS (m/z = 621.7).

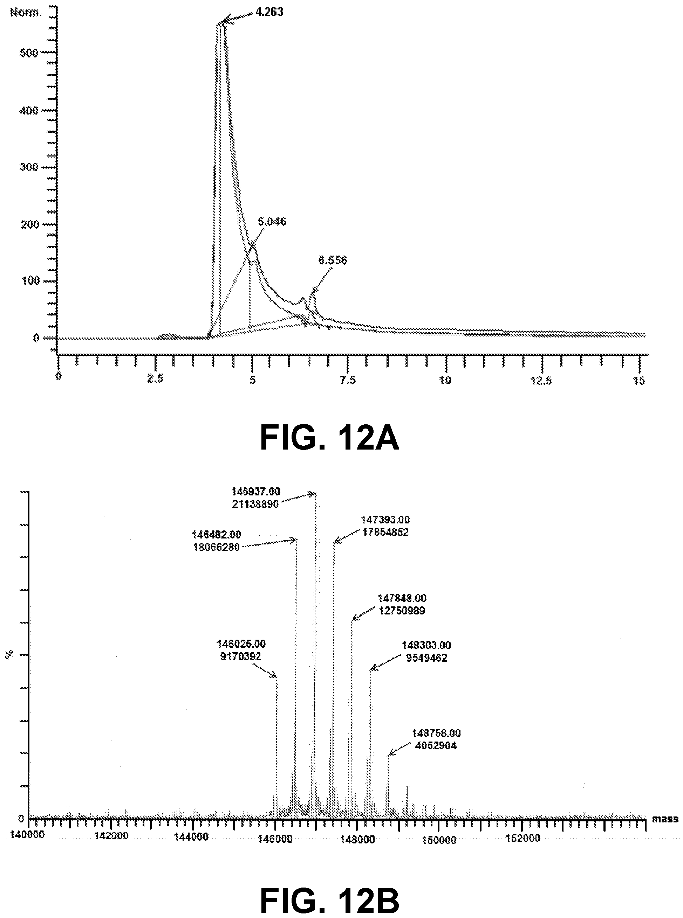

*Conjugation to antibody:* The TFP ester XVI was dissolved in dry DMSO to make a 20 mM stock solution and 6 molar equivalents (relative to the antibody) was added to 20 mg IgG antibody (specifically, the anti-CD20 antibody rituximab) (10 mg/mL in PBS). The conjugation reaction was incubated at 4 °C overnight. The resulting immunoconjugate, BB6, was buffer exchanged into PBS (pH 7.2) to remove excess small molecular weight reagents. The final concentration was determined by measuring the antibody at 280 nm on the Nanodrop 1000 spectrophotometer. The yield was 15 mg of BB6, or 75% based on recovered protein. As seen in Figure 12A, minimal aggregate was seen (less than 1%) as detected by SEC analysis. As seen in Figure 12B, the product had a DAR ratio of 2.8 as determined via LC/MS analysis. The purified immunoconjugates BB6 was filtered through a 2 µM sterile filter and stored at -20 °C.

### Example 8. Synthesis of Immunoconjugate BB7 with a TFP Ester

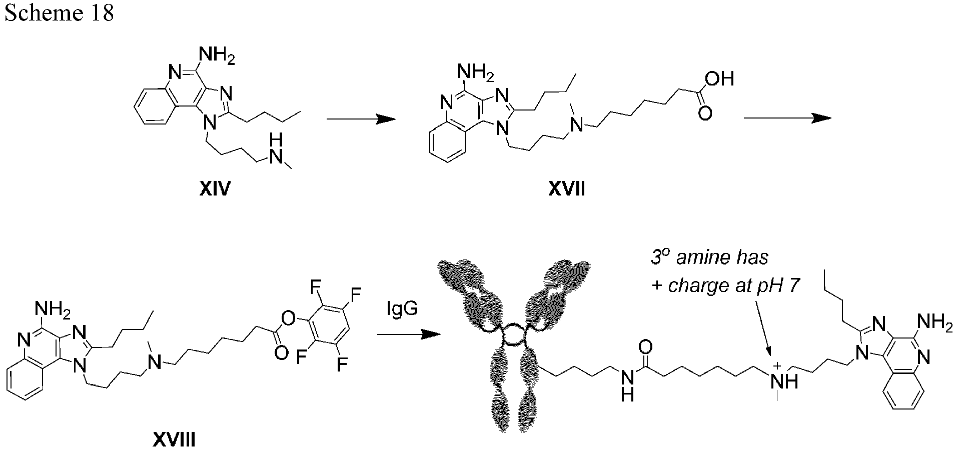

This example provides guidance on synthesis of an immunoconjugate that contains an alkyl tertiary amine linker using the TFP ester method. Compound XIV (200 mg) was dissolved in methanol (20 mL) and 3 equivalents of 1-formyl-7-tert-butyl heptanoate was added followed by 1.1 equivalents of NaCNBH₄. The mixture was stirred for 1.5 hours at room temperature and concentrated to dryness. TFA (5 mL) was added and the mixture stirred overnight at room temperature. The TFA was evaporated under vacuum and the crude product was purified by preparative HPLC on a C-18 column. The product was eluted with a gradient of 10-90% acetonitrile in water (0.1% TFA) over 20 minutes to provide 110 mg of purified acid compound XVII (which was confirmed by LC/MS).

Compound XVII (50 mg) was dissolved in dichloromethane/dimethylformamide (5 mL, 1:1) and 2 equivalents of TFP was added followed by 1.5 equivalents of EDCI. The reaction was stirred overnight at room temperature. The crude TFP ester product XVIII was purified via flash chromatography on a 4-gram silica gel column eluted with 0-10% isopropanol over 10 minutes. Pure fractions were concentrated, and the residue lyophilized from 30% acetonitrile water to provide 14 mg of purified TFP ester compound XVIII as a white solid. The molecular weight and purity were confirmed by LC/MS (m/z =601.7).

*Conjugation to antibody:* TFP ester XVIII was dissolved in dry DMSO to make a 20 mM stock solution and 8 molar equivalents (relative to the antibody) was added to 20 mg of an IgG antibody (specifically, the anti-CD20 antibody rituximab) (10 mg/mL in PBS). The conjugation reaction was incubated at 4 °C overnight. The resulting immunoconjugate BB7 was buffer exchanged into PBS (pH 7.2) to remove excess small molecular weight reagents. The final concentration was determined by measuring the antibodies at 280 nm on the Nanodrop 1000 spectrophotometer. The yield was 16 mg of immunoconjugate BB7 (80%).

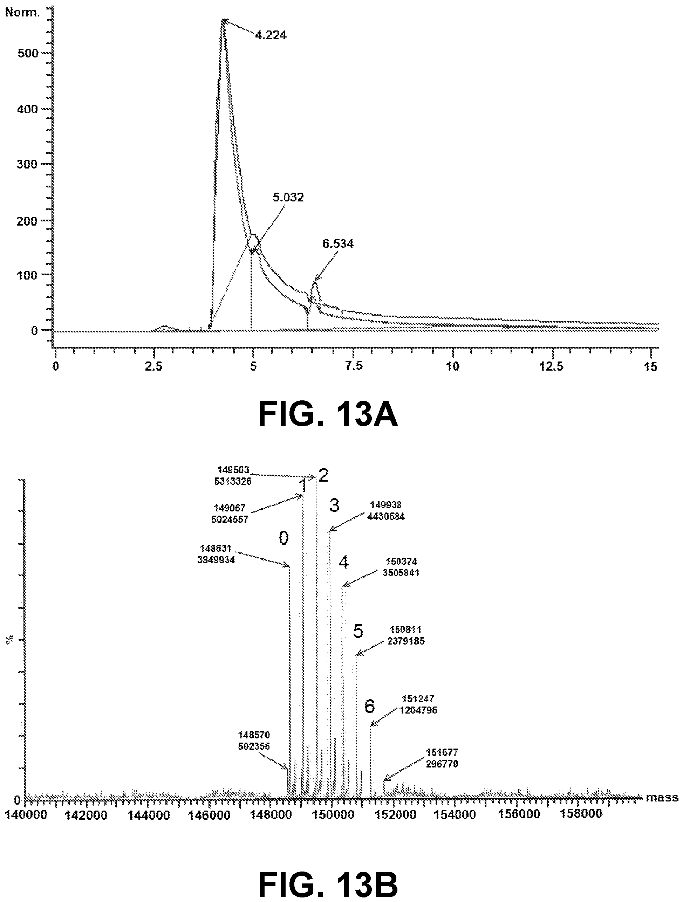

As seen in Figure 13A, minimal aggregate was seen (less than 1%) as detected by SEC analysis. As seen in Figure 13B, the product had a DAR ratio of 2.5 as determined via LC/MS analysis. The purified BB7 was filtered through a 2 µM sterile filter and stored at -20 °C.

### Example 9. Synthesis of Immunoconjugate BB8 with a TFP Ester

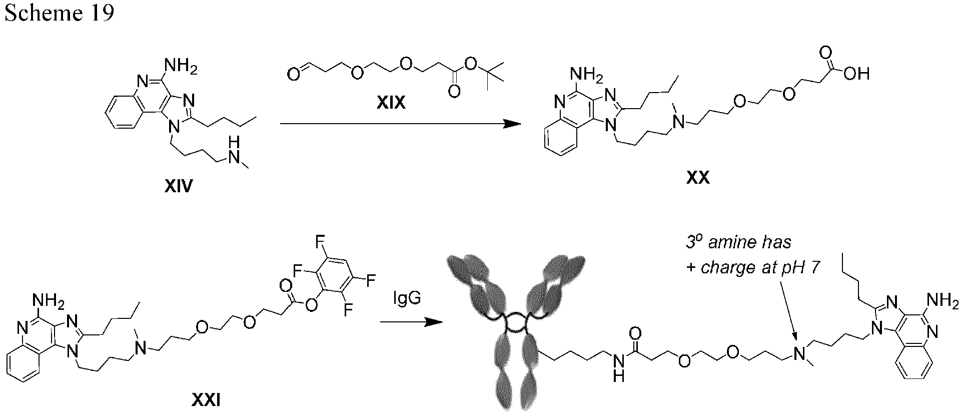

This example provides guidance on synthesis of an immunoconjugate that contains a PEG tertiary amine linker using the TFP method. Compound XIV (200 mg) was dissolved in methanol (20 mL) and 3 eq. of aldehyde XIX was added followed by 1.1 equivalents of NaCNBH₄. The mixture was stirred for 3 hours at room temperature and concentrated to dryness. Trifluoroacetic acid (TFA, 10 mL) was added and the reaction stirred for 2 hours at room temperature. The TFA was evaporated under vacuum and the crude product was purified by preparative HPLC on a C-18 column. The product was eluted with a gradient of 10-90% acetonitrile in water (0.1% TFA) over 20 minutes to provide 85 mg of purified acid XX after lyophilization of the combined pure fractions (confirmed by LC/MS).

Compound XX (80 mg) was dissolved in dichloromethane/dimethylformamide (5 mL, 1:1) and 2 equivalents of TFP was added followed by 1.2 equivalents of EDCI. The reaction was stirred overnight at room temperature. The crude TFP ester product XXI was purified via flash chromatography on a 4-gram silica gel column eluted with 0-10% isopropanol over 10 minutes. Pure fractions were concentrated, and the residue lyophilized from 30% acetonitrile water to provide 45 mg of purified TFP ester of compound XXI as a beige solid. The molecular weight and purity were confirmed by LC/MS (m/z = 647.7).

*Conjugation to antibody*: The TFP ester of compound XXI was dissolved in dry DMSO to make a 20 mM stock solution and 8 molar equivalents (relative to the antibody) was added to an IgG1 antibody (specifically, the anti-CD20 antibody rituximab) (10 mg/mL in PBS). The conjugation reaction was incubated at 4 °C overnight. The resulting immunoconjugate BB8 was buffer exchanged into PBS (pH 7.2) to remove excess small molecular weight reagents. The final concentration was determined by measuring the antibodies at 280 nm on the Nanodrop 1000 spectrophotometer. The yield was 15 mg of immunoconjugate BB8 (75%) which was stored at 4 °C until used.

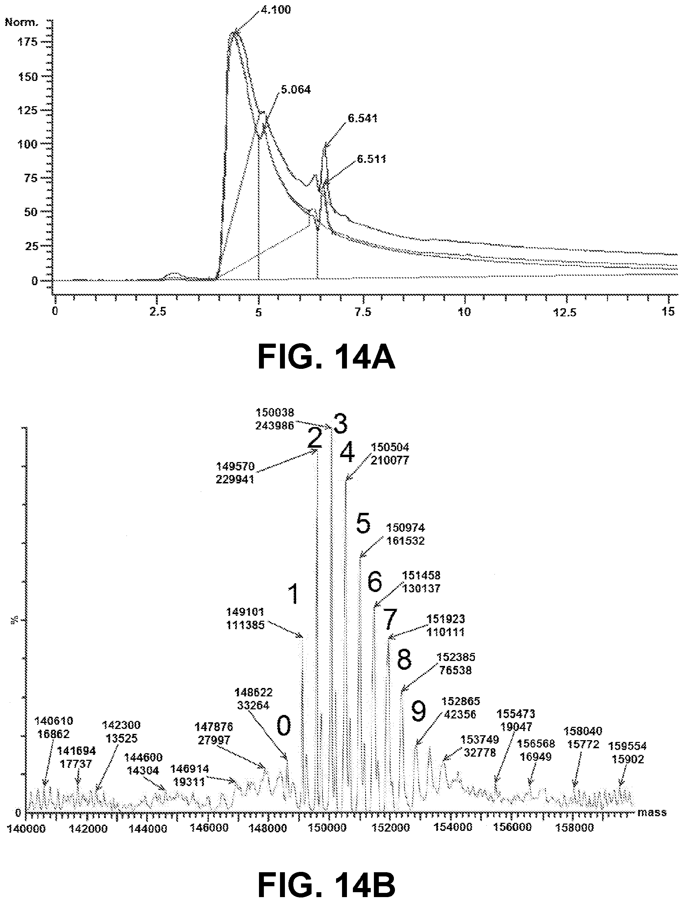

As seen in Figure 14A, minimal aggregate was seen (less than 1%) as detected by SEC analysis. As seen in Figure 14B, the product had a DAR ratio of 2.2 as determined via LC/MS analysis. The purified immunoconjugate BB8 was filtered through a 2 µM sterile filter and stored at -20 °C.

### Example 10. Synthesis of Immunoconjugate BB9 with a TFP Ester

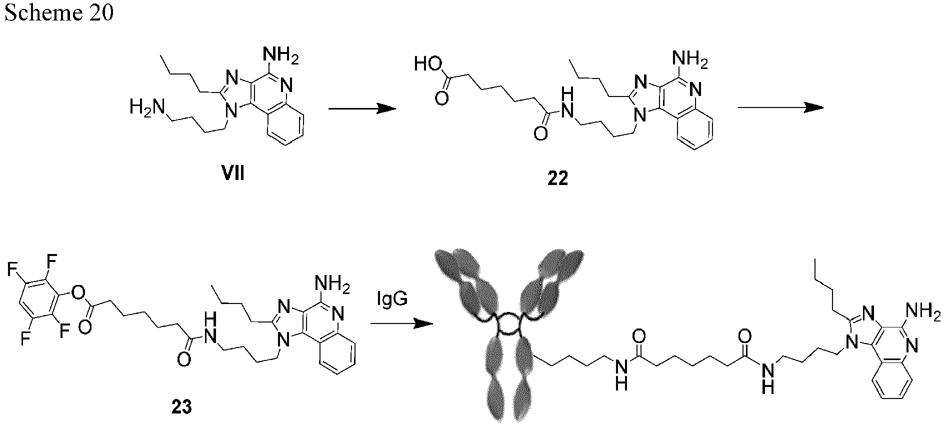

This example provides guidance on synthesis of an immunoconjugate with a different linker using the TFP ester method. Compound VII (311 mg, 1 mmol) was dissolved in 10 mL of DMF and 0.3 mL of DIPEA was added. In a separate container, 1.2 equivalents of 7-methoxy-7-oxoheptanoic acid was dissolved in 5 mL of DMF and 1.5 equivalents DIPEA was added followed by HATU (1.2 equivalents). The mixture was added to VII and stirred overnight at room temperature. The reaction mixture was concentrated to dryness under vacuum and the residue was dissolved in 10 mL of (1:1) tetrahydrofuran:water. One mL of 2M lithium hydroxide in water was added and the reaction stirred for 2 hours at room temperature. The THF was removed via rotary evaporation and the aqueous solution was acidified by adding 10 mL of 1M hydrochloric acid. The aqueous solution was extracted 2x with dichloromethane (20 mL) and the organic layer was combined and dried over magnesium sulfate. The solution was filtered, and the filtrate concentrated to dryness. The crude product 22 was purified via silica gel chromatography on a 4-gram column eluted with 0-10% isopropanol in DCM (w/1% acetic acid) over 10 minutes. The pure fractions were combined and concentrated to provide 220 mg of pure 22 as a pale yellow solid.

Compound 22 (50 mg) was dissolved in dichloromethane/dimethylformamide (5 mL, 1:1) and 2 equivalents of TFP was added followed by 1.5 equivalents of EDCI. The reaction was stirred overnight at 22 °C and the crude reaction was concentrated to dryness. The product was purified via flash chromatography on a 4-gram silica gel column eluted with 0-10% isopropanol over 10 minutes. Pure fractions were concentrated, and the residue was lyophilized from 30% acetonitrile in water to provide 21 mg of purified TFP ester 23 as a pale yellow solid. The molecular weight and purity were confirmed by LC/MS.

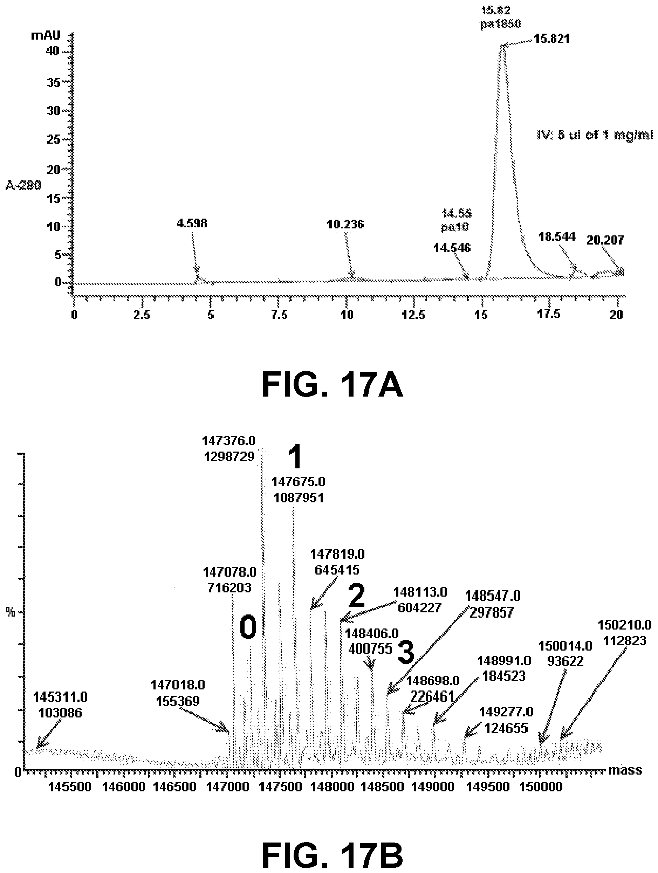

*Conjugation to antibody:* The TFP ester 23 was dissolved in dry DMSO to make a 20 mM stock solution and 6 molar equivalents (relative to the antibody) was added to 20 mg of an IgG antibody (specifically, the anti-CD20 antibody rituximab) (10 mg/mL in PBS). The conjugation reaction was incubated at 4 °C overnight. The resulting immunoconjugate BB9 was buffer exchanged into PBS (pH 7.2) to remove excess small molecular weight impurities. The final concentration was determined by measuring the absorbance at 280 nm on a THERMO SCIENTIFIC^{™} NANODROP^{™} 1000 spectrophotometer. The yield was 14 mg of BB9, or 70% based on recovered protein. Minimal aggregate (less than 1%) was detected by SEC analysis (see Figure 17A) and a DAR of 2.8 was determined via LC/MS analysis (see Figure 17B). The purified immunoconjugate was filtered through a 2 µM sterile filter and stored at -20 °C.

### Example 11. Synthesis of Immunoconjugate BB10 with a TFP Ester

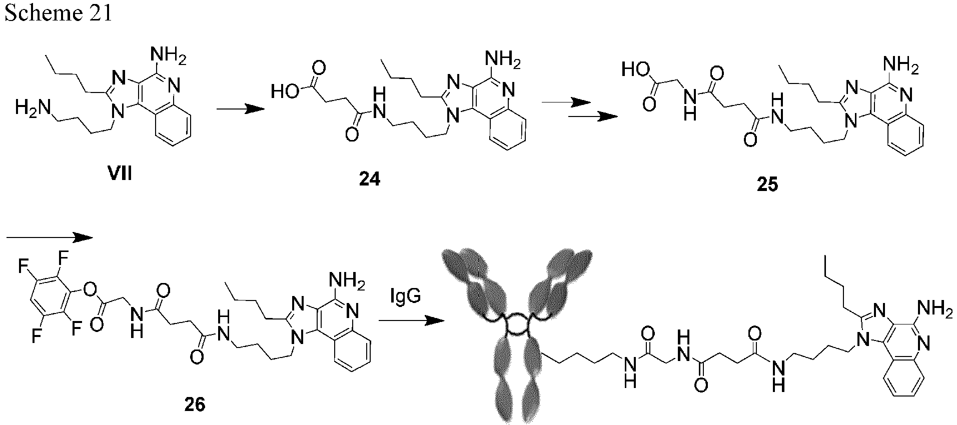

This example provides guidance on synthesis of an immunoconjugate with a different linker using the TFP ester method. Compound VII (150 mg) was dissolved in 20 mL THF and 10 mL of aqueous saturated sodium bicarbonate was added. Succinic anhydride (50 mg) was added in one portion and the mixture stirred for 1 hour at room temperature. 20 mL of IN HCl was added slowly and the mixture was extracted with 2X 50 mL of dichloromethane and the combined organic extracts were evaporated to dryness. The crude product 24 was purified on a 4 gram silica gel column eluted with 0-15% MeOH (1% acetic acid) over 15 minutes. Pure fractions were combined and evaporated to provide 180 mg of pure 24.

One hundred and fifty mg of 24 was dissolved in DMF (10 mL) and 1 equivalent of HATU was added followed by 2 equivalents of DIPEA. One and a half eq. of glycine-OtBu was added and stirred overnight. The DMF was evaporated and the residue treated with 5 mL of IN HCl in dioxane for 30 minutes with stirring. The solvent was evaporated, and the crude residue was flash purified on a 4 gram silica gel column eluted with 0-10% isopropanol over 15 minutes. Evaporation of pure fractions provided 110 mg of pure 25.

Compound 25 (50 mg) was dissolved in 10 mL DMF and 1.5 eq. of TFP was added followed by 1.2 eq. DCC and 2 mg of DMAP. The reaction was stirred overnight, concentrated to dryness and purified on silica gel (4g column) eluted with 0-10% IPA in DCM to provide 32 mg of pure TFP ester, compound 26, after lyophilization from 1:3 acetonitrile water.

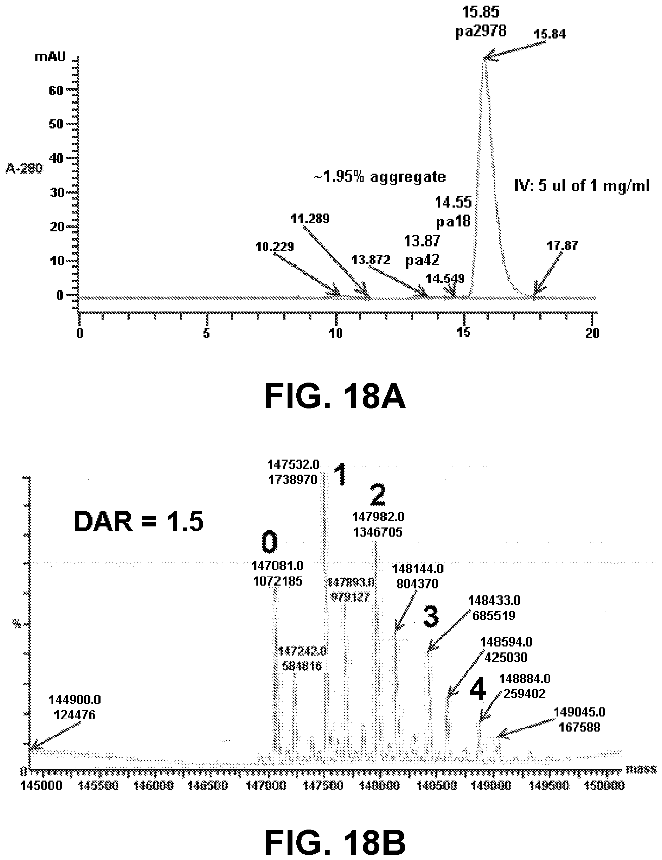

*Conjugation to antibody:* The TFP ester, compound 26, was dissolved in dry DMSO to make a 20 mM stock solution and 5 molar equivalents (relative to the antibody) was added to 20 mg antibody at 10 mg/mL in PBS. The conjugation reaction was incubated at 4 °C for 6 hours. The resulting immunoconjugate BB10 was buffer exchanged into PBS (pH 7.4) to remove excess small molecular weight impurities. The final protein concentration was determined by measuring the absorbance at 280 nm on a NANODROP^{™} 1000 spectrophotometer. The yield was 15 mg (75% based on recovered protein). SEC analysis detected minimal aggregate of less than 1% (see Figure 18A) and the DAR was determined to be 2.8 adjuvants per antibody via LC/MS analysis (see Figure 18B). The purified immunoconjugate was filtered through a 2 µM sterile filter and stored at -20 °C until needed.

### Example 12. Synthesis of Immunoconjugate BB11 with a TFP Ester

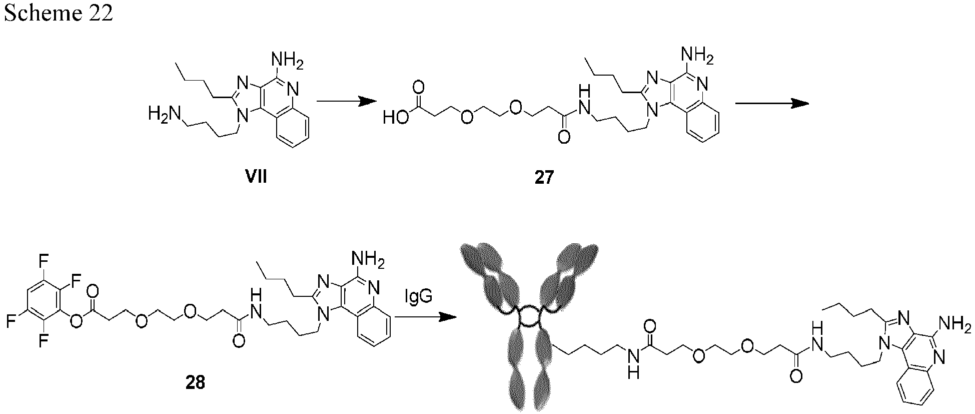

This example provides guidance on synthesis of an immunoconjugate with a different linker using the TFP method. Compound VII (155 mg, 0.5 mmol) was dissolved in 10 mL of DMF and 0.2 mL of DIPEA was added. In a separate container, 1.2 equivalents of PEG2-dicarboxylate mono methyl ester was dissolved in 5 mL of DMF and 2 equivalents DIPEA was added followed by HATU (1.2 equivalents). The mixture was added to VII and stirred 1 hour at room temperature. The reaction was concentrated to dryness under vacuum and the residue was dissolved in THF (5 mL). An equal volume of water was added followed by 2 mL of 1 M aqueous LiOH. The mixture was stirred overnight and then 10 mL of IN HCl was added. The acidified mixture was extracted 2x with dichloromethane, dried over sodium sulfate, concentrated to dryness and purified via silica gel chromatography. The product was eluted with 0-10% methanol over 10 minutes. The pure fractions were combined and concentrated to provide 110 mg of pure compound 27 as a pale yellow solid.

Compound 27 (50 mg) was dissolved in dichloromethane/dimethylformamide (5 mL, 1:1) and 2 equivalents of TFP was added followed by 1.5 equivalents of EDCI. The reaction was stirred overnight at ambient temperature and the reaction was concentrated to dryness. The crude TFP ester 28 was purified via flash chromatography on a 4-gram silica gel column eluted with 0-10% isopropanol over 10 minutes. Pure fractions were concentrated, and the residue was lyophilized from 30% acetonitrile in water to provide 41 mg of purified TFP ester 23 as a white solid. The molecular weight and purity were confirmed by LC/MS.

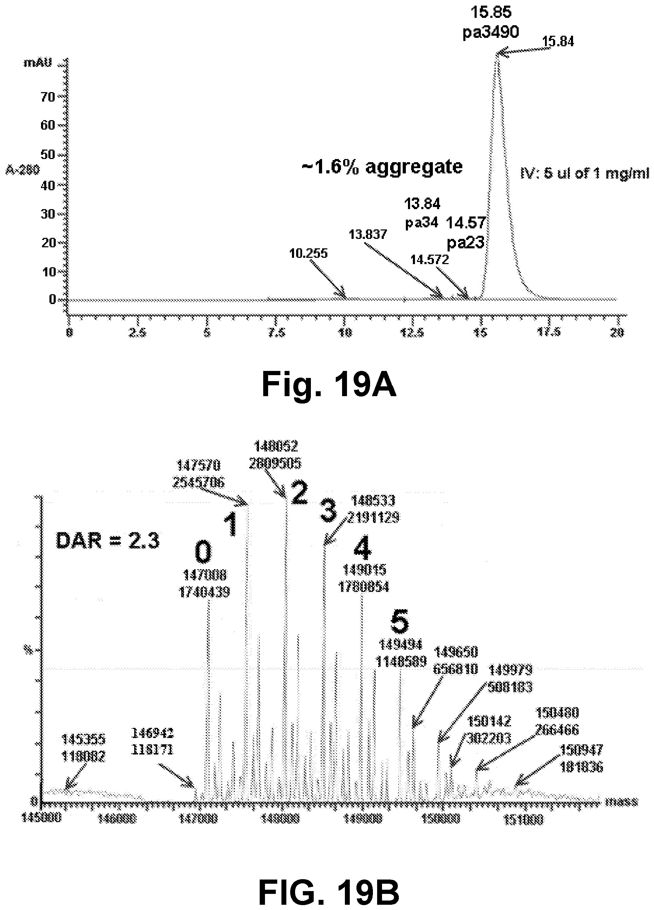

*Conjugation to antibody:* The TFP ester 28 was dissolved in dry DMSO to make a 20 mM stock solution and 8 molar equivalents (relative to the antibody) was added to 20 mL of an IgG antibody (specifically, the anti-CD20 antibody rituximab) (10 mg/mL in PBS). The conjugation reaction was incubated at 4 °C overnight. The resulting immunoconjugate BB11 was buffer exchanged into PBS (pH 7.2) to remove excess small molecular weight impurities. The final concentration was determined by measuring the absorbance at 280 nm on a Thermo Nanodrop 1000 spectrophotometer. The yield was 16 mg of conjugated immunoconjugate BB11, or 70% based on recovered protein. Minimal aggregate (less than 1%) was detected by SEC analysis (see Figure 19A) and a DAR of 2.3 was determined via LC/MS analysis (see Figure 19B). The purified immunoconjugate was filtered through a 2 µM sterile filter and stored at -20 °C.

### Example 13. Synthesis of Another TLR7/8 Adjuvant

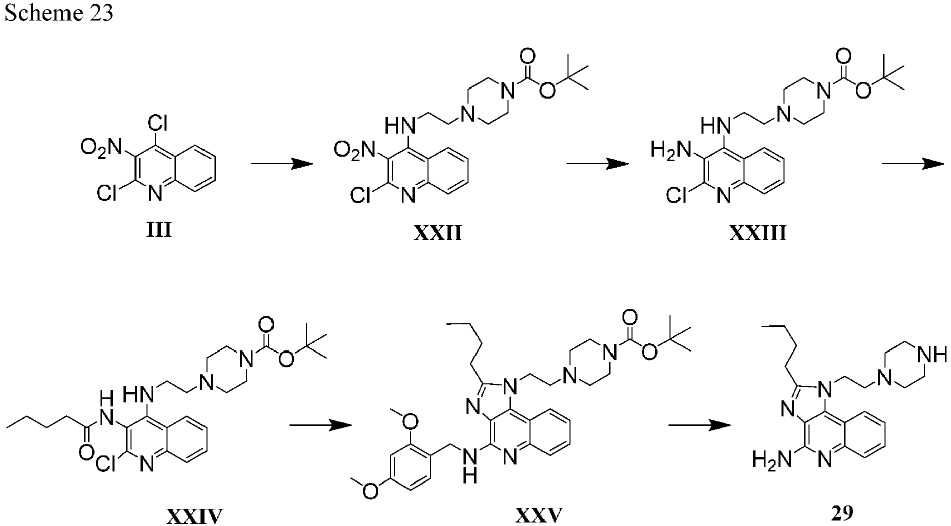

This example provides guidance on synthesis of another TLR agonist. Compound 29 is a compound VII analog that contains a piperazine side-chain for linker attachment. It was synthesized using methods previously described for the synthesis of the compound VII except that a Boc-protected piperazine analog was substituted for Boc-diaminobutane used in step 3 of the synthesis. The general synthetic route for compound 29 is outlined in Scheme 23. The addition of the piperazine side chain enables the synthesis of immunoconjugates that were previously inaccessible due to instability. Similar compound VII analogs containing succinate linkers are prone to cyclization upon TFP activation and the piperazine prevents cyclization. In addition, the tertiary amino group within the piperazine moiety maintains a positive charge after linker attachment and conjugation. Positive charges in this location are important for improved TLR8 potency. Compound 29 was subsequently used for synthesizing immunoconjugates as described below in Examples 14-16.

### Example 14. Synthesis of Immunoconjugate BB12 with a TFP Ester

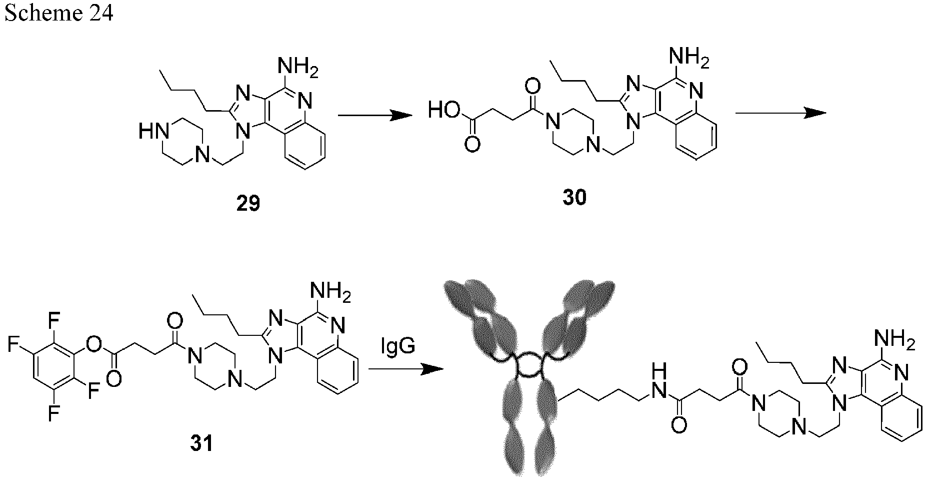

This example provides guidance on synthesis of an immunoconjugate with a different linker using the TFP ester method. Compound 29 (100 mg) was dissolved in 10 mL THF and 2 mL of aqueous saturated sodium bicarbonate was added followed by 10 mL of water. Succinic anhydride (50 mg) was added in one portion and the mixture was stirred at room temperature. After one hour, 20 mL of IN HCl was added slowly and the reaction mixture was extracted with 2X 50 mL of dichloromethane ("DCM"). The combined organic extracts were evaporated to dryness. The crude product 30 was purified on a 4 gram silica gel column eluted with 0-15% isopropanol in DCM (1% acetic acid) over 15 minutes. Pure fractions were combined and evaporated to dryness to provide 80 mg of pure acid 30.

Compound 30 (50 mg) was dissolved in dichloromethane/dimethylformamide (5 mL, 1:1) and 2 equivalents of TFP was added followed by 1.5 equivalents of EDCI. The reaction was stirred overnight at ambient temperature and the reaction was concentrated to dryness. The crude TFP ester 31 was purified via flash chromatography and eluted with 0-10% isopropanol over 10 minutes. Pure fractions were concentrated, and the residue was lyophilized from 30% acetonitrile in water to provide 41 mg of purified TFP ester 31 as a white solid. The molecular weight and purity were confirmed by LC/MS.

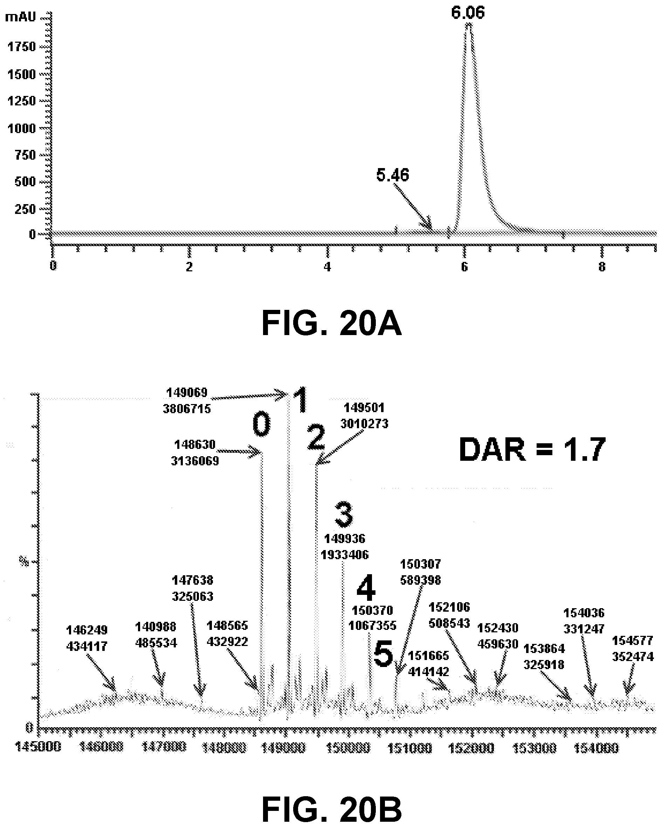

The TFP ester 31 was conjugated to an IgG1 antibody (specifically, the anti-CD20 antibody rituximab) as described previously for BB10 to provide BB12. SEC and LC/MS analysis of BB12 confirmed the molecular weight, a high monomeric purity with less than 2 % aggregate, and a DAR of 1.7 (see Figures 20A-B).

### Example 15. Synthesis of Immunoconjugates BB13 and BB14 with a TFP Ester

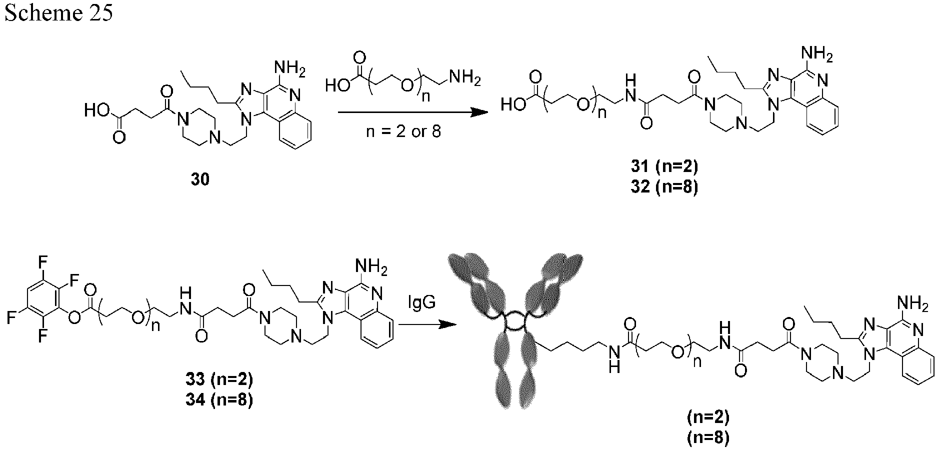

This example provides guidance on synthesis of immunoconjugates with different linkers using the TFP ester method. Compound 30 (Scheme 25) was coupled to polyethylene glycol (PEG) linkers containing 2 or 8 PEG units in order to extend the distance between the adjuvant and the antibody. Attachment of the PEG linker extensions was performed using previously described protocols for linker attachment and TFP activation. Briefly 100 mg of compound 30 was dissolved in 10 mL of DMF and 0.2 mL of DIPEA was added followed by HATU (1.2 equivalents). After 1 hour the appropriate amino PEG linker (n = 2 or 8) was added and stirred an additional 2 hours at room temperature. The reaction mixture was concentrated to dryness under vacuum and the residue was purified via preparative HPLC on a C-18 column eluted with 10-90% acetonitrile in water over 30 minutes. The pure fractions were combined and lyophilized to provide 65 mg and 45 mg of intermediates 31 or 32 as a clear glassy substance.

Compounds 31 and 32 were converted to the corresponding TFP esters 33 and 34 using previously described protocols. Briefly, the free acid 31 or 32 (50 mg) was dissolved in dichloromethane/dimethylformamide (5 mL, 1:1) and 2 equivalents of TFP was added followed by 1.5 equivalents of EDCI. The mixture was stirred overnight at room temperature and concentrated to dryness to provide crude TFP esters 33 and 34. The crude TFP esters were purified via flash chromatography on silica gel and eluted with 0-10% isopropanol over 10 minutes. Pure fractions were concentrated, and the residue was lyophilized from 30% acetonitrile in water to provide purified TFP esters 33 and 34 as clear solids. The molecular weight and purity of the pure compounds were confirmed by LC/MS.

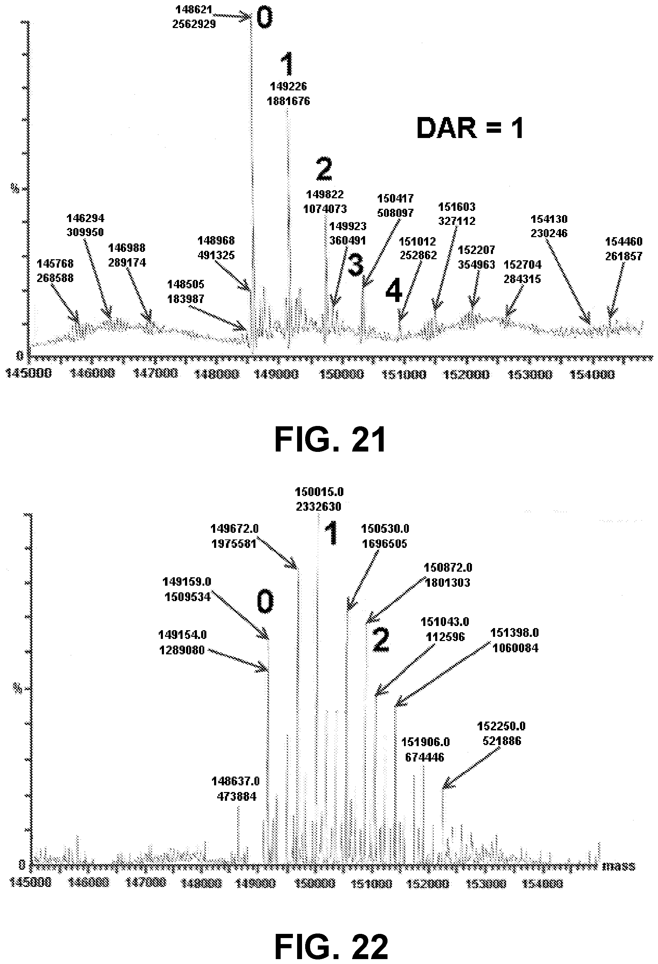

*Conjugation to antibody:* TFP esters 33 and 34 were conjugated to an IgG1 antibody (specifically, the anti-CD20 antibody rituximab) using previously described protocols. The TFP esters were dissolved in dry DMSO to make a 20 mM stock solution and 8 molar equivalents (relative to the antibody) was added to 20 mg of the IgG antibody at 10 mg/mL in PBS. The conjugation reaction was incubated at 4 °C for 12 hours. The resulting immunoconjugates, BB13 and BB14 were buffer exchanged into PBS (pH 7.4) to remove excess small molecular weight impurities. The final protein concentration was determined by measuring the absorbance at 280 nm on a NANODROP^{™} 1000 spectrophotometer. The yields were 75% based on recovered protein. SEC analysis detected minimal aggregate was present and the DARs of 1.0 and 1.7 adjuvants per antibody were determined via LC/MS analysis (see Figure 21 for BB13 and Figure 22 for BB14). The purified immunoconjugates were filtered through a 0.2 µM sterile filter and stored at -20 °C until needed.

### Example 16. Assessment of BB1, BB2, BB4, BB5, BB7, BB9, and BB10 Activity In Vitro

*Isolation of Human Antigen Presenting Cells.* Human antigen presenting cells (APCs) were negatively selected from human peripheral blood mononuclear cells obtained from healthy blood donors (Stanford Blood Center) by density gradient centrifugation using a RosetteSep Human Monocyte Enrichment Cocktail (Stem Cell Technologies) containing monoclonal antibodies against CD14, CD16, CD40, CD86, CD123, and HLA-DR. Immature APCs were subsequently purified to >97% purity via negative selection using an EasySep Human Monocyte Enrichment Kit without CD16 depletion containing monoclonal antibodies against CD14, CD16, CD40, CD86, CD123, and HLA-DR.

*Preparation of Tumor Cells.* Tumor cells were resuspended in PBS with 0.1% fetal bovine serum (FBS) at 1 to 10 × 10⁶ cells/mL. Cells were subsequently incubated with 2 µM CFSE to yield a final concentration of 1 µM. The reaction was ended after 2 minutes via the addition of 10 mL complete medium with 10% FBS and washed once with complete medium. Cells were either fixed in 2% paraformaldehyde and washed three times with PBS or left unfixed prior to freezing the cells in 10% DMSO, 20% FBS and 70% medium.

*APC-Tumor Co-cultures.* 2 × 10⁵ APCs were incubated with or without 6.5 x 10⁵ allogeneic CFSE-labeled tumor cells in 96-well plates (Corning) containing IMDM medium (Gibco) supplemented with 10% fetal bovine serum, 100 U/mL penicillin, 100 µg/mL streptomycin, 2 mM L-glutamine, sodium pyruvate, non-essential amino acids and, where indicated, various concentrations of unconjugated CD20 antibody, BB1, BB2, BB4, BB5, BB7, BB9, or BB10 prepared according to the examples above. Cells and cell-free supernatants were analyzed after 18 hours via flow cytometry or ELISA.

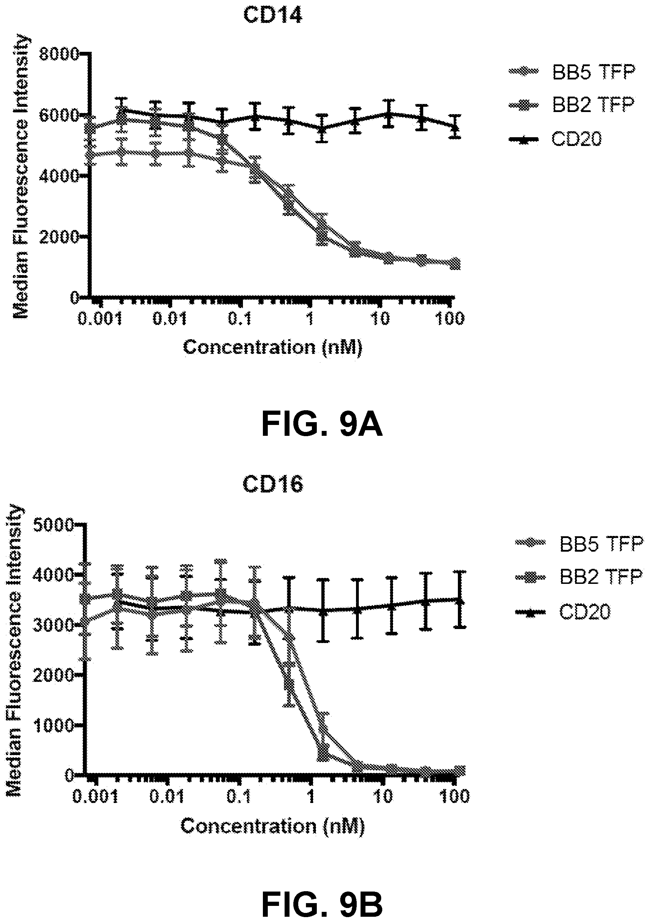

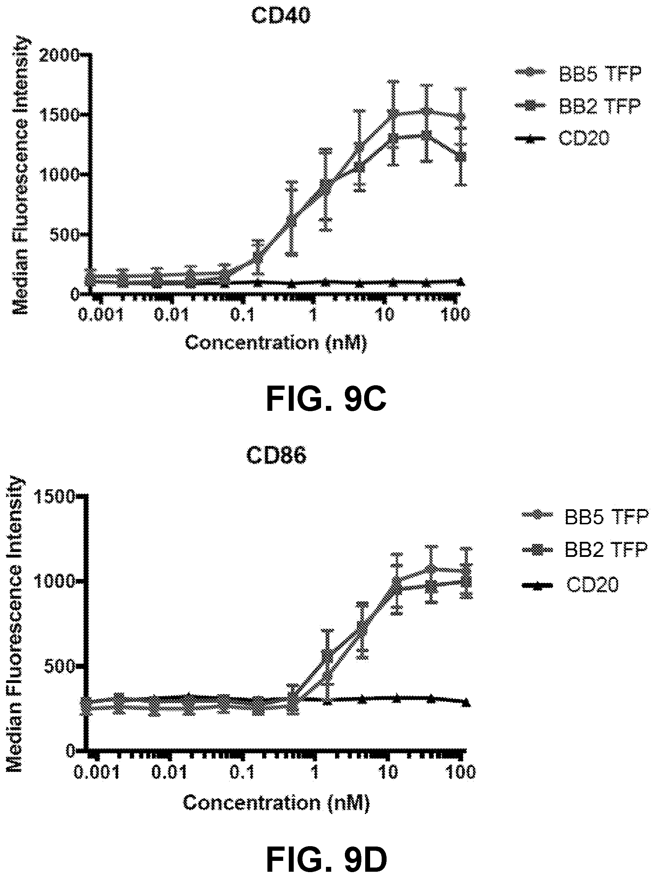

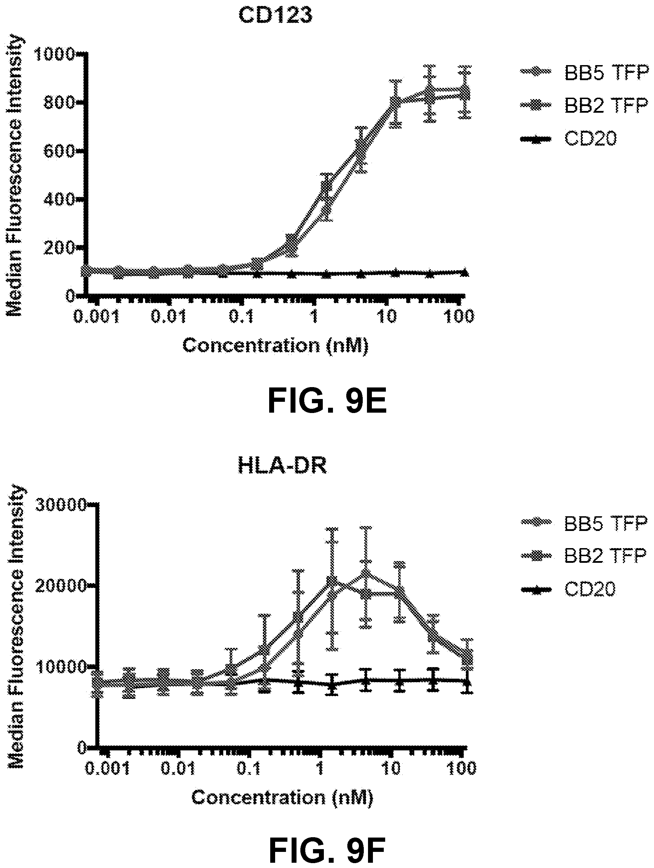

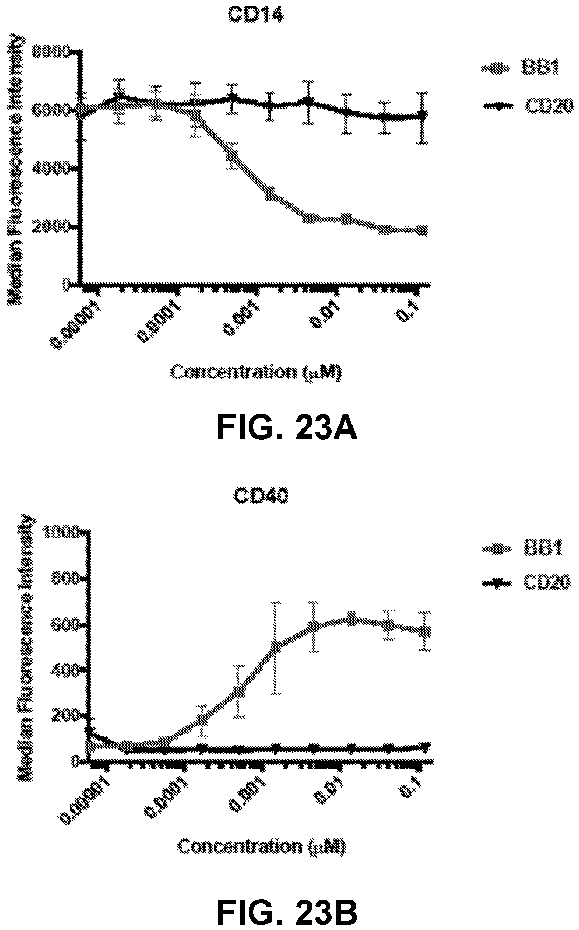

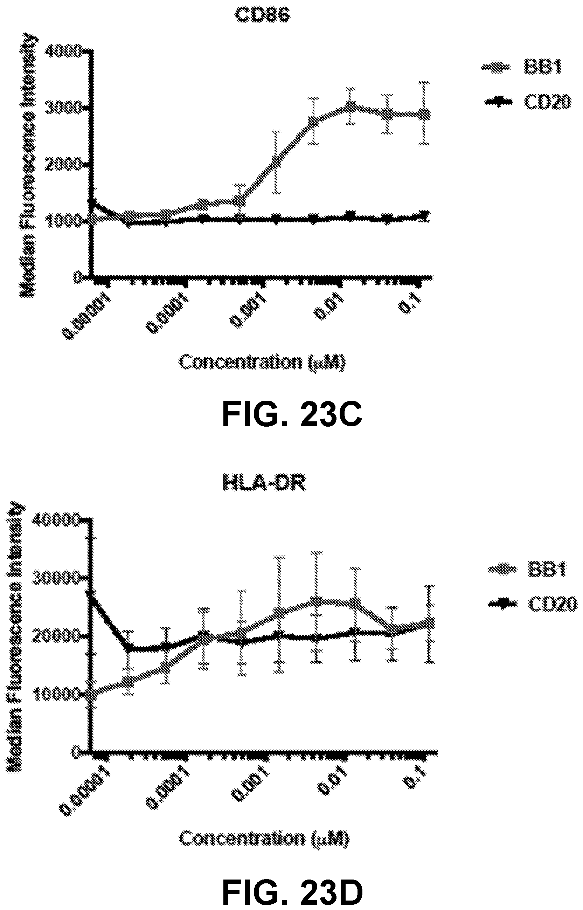

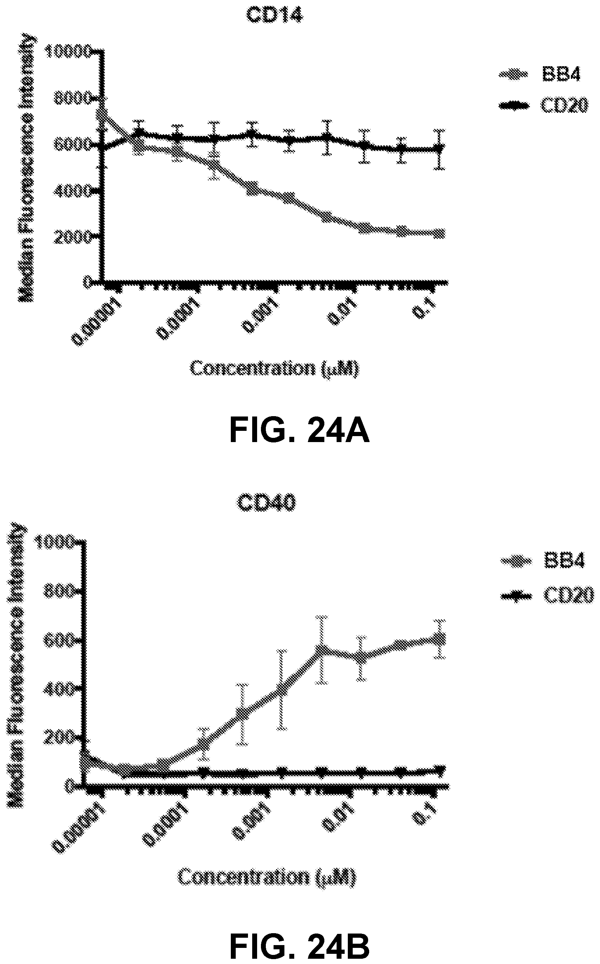

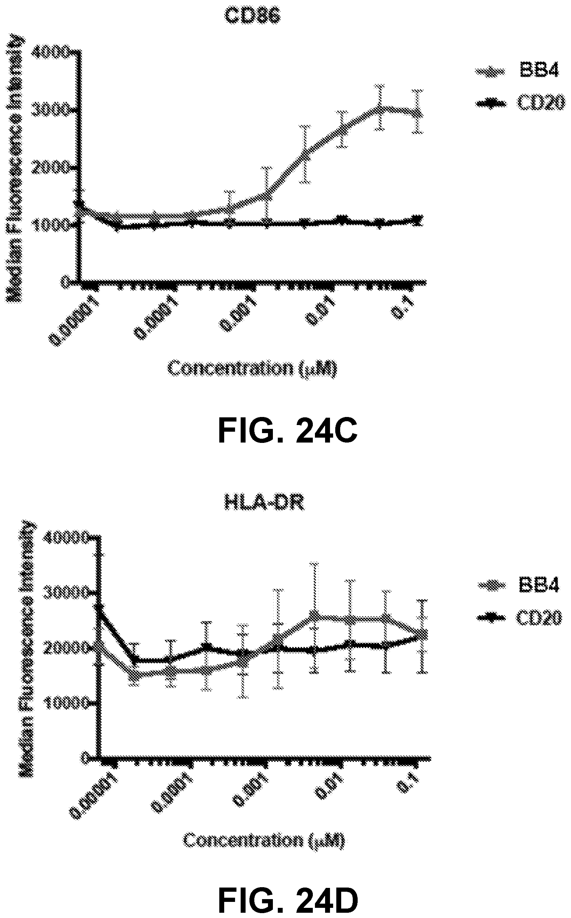

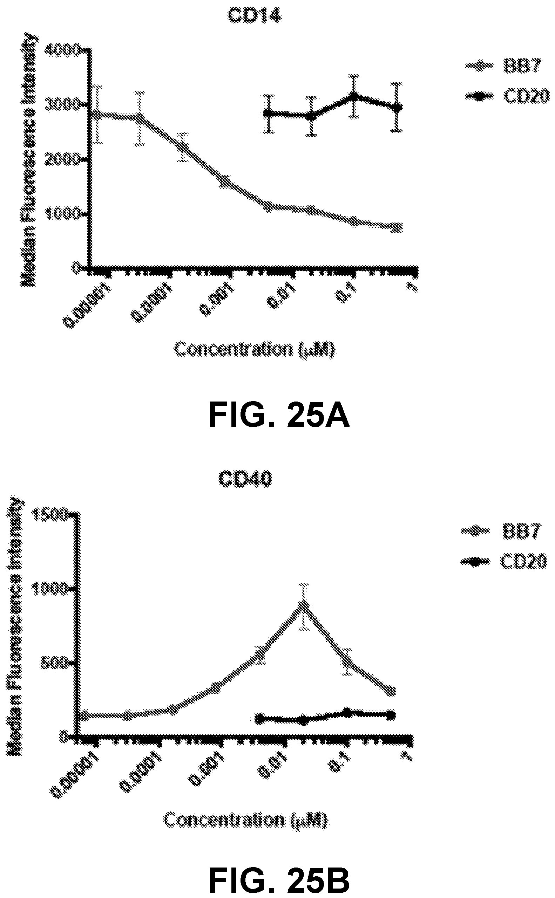

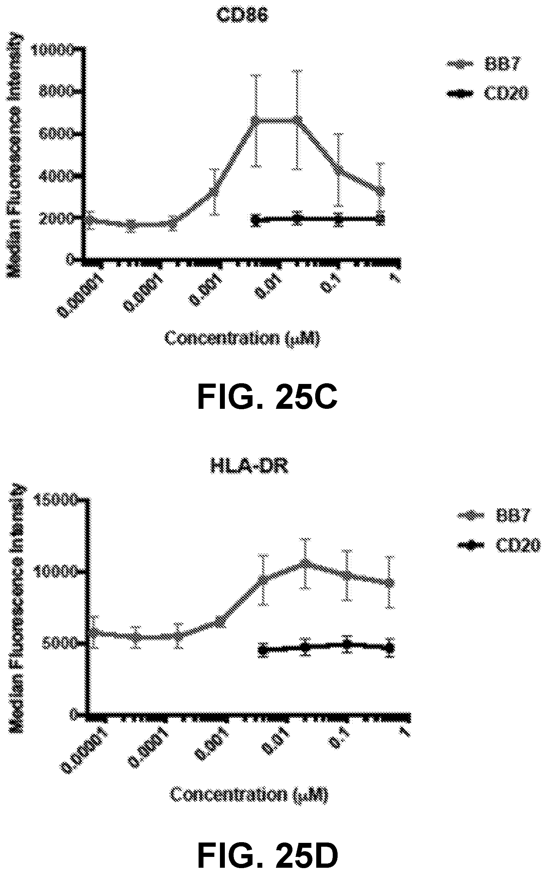

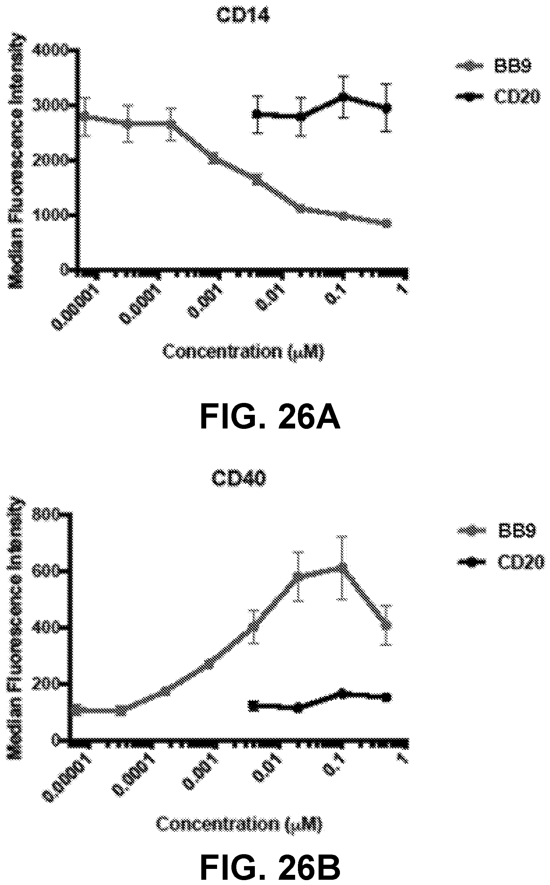

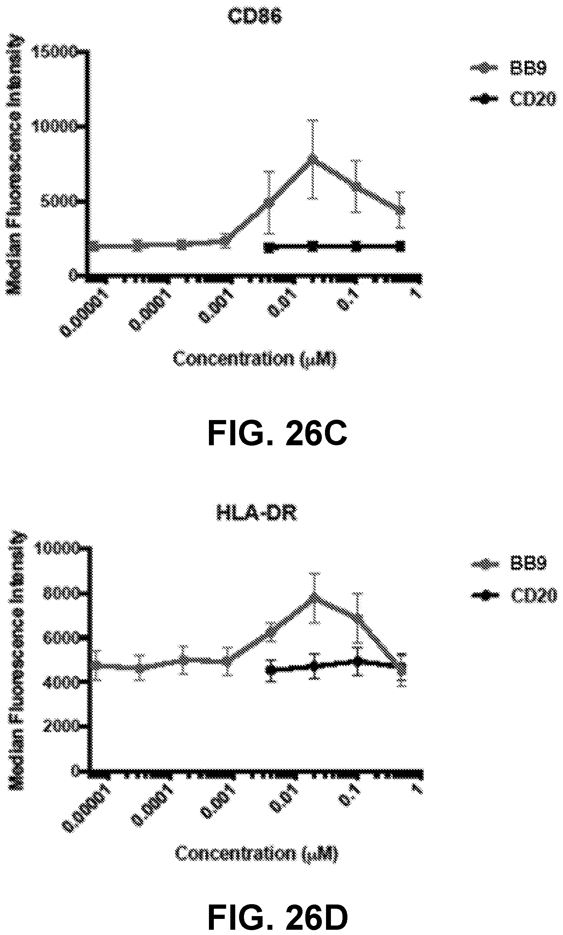

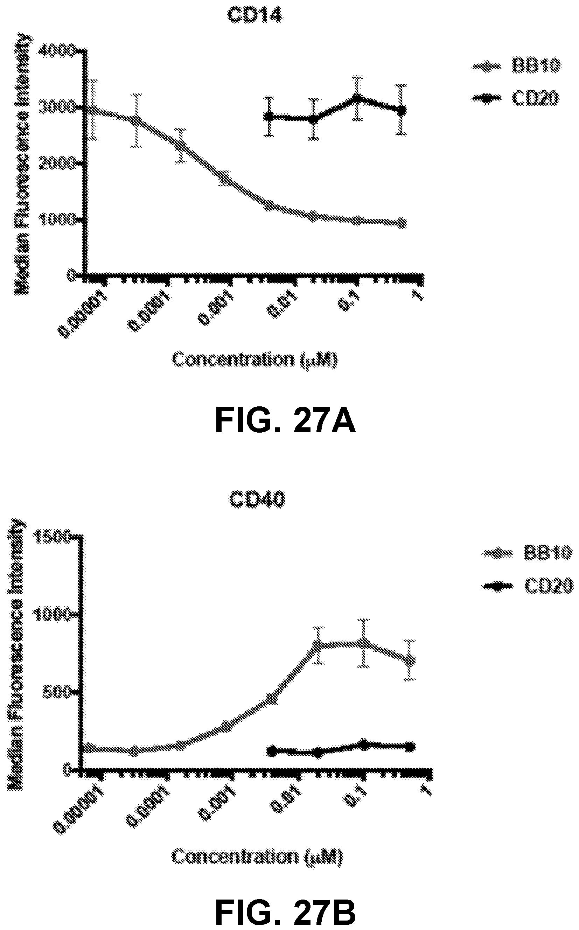

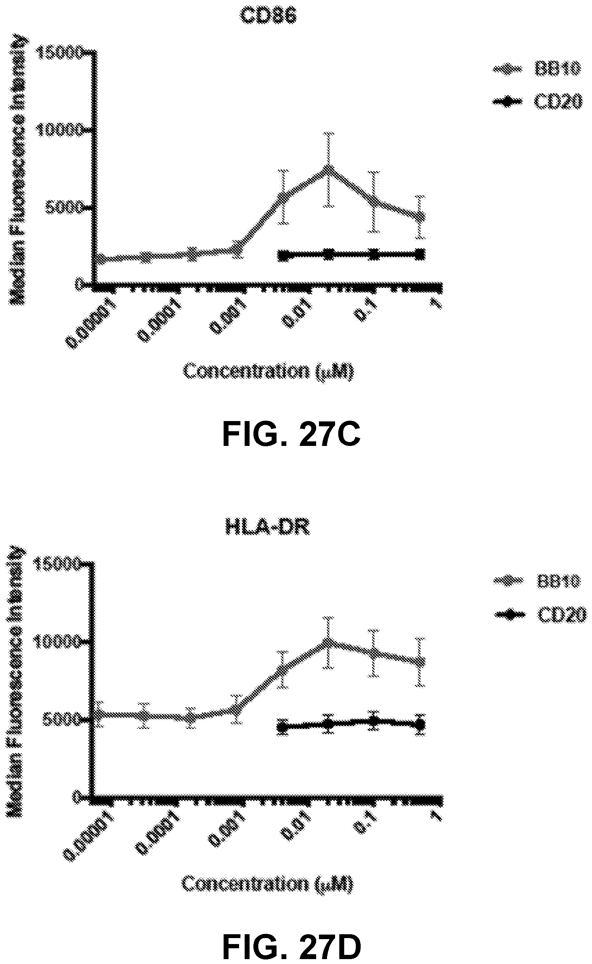

The results of this assay are shown in Figures 9A-9F for BB2 and BB5. Specifically, the graphs show that BB2 and BB5 prepared according to Schemes 11 and 14 elicits myeloid activation while the control, unconjugated CD20 antibody, does not. Further, Figures 23A-D show that BB1 elicits myeloid activation as indicated by CD14, CD20, CD86, and HLA-DR while the control does not. Figures 24A-D show that BB4 elicits myeloid activation as indicated by CD14, CD20, CD86, and HLA-DR while the control does not. Figures 25A-D show that BB7 elicits myeloid activation as indicated by CD14, CD20, CD86, and HLA-DR while the control does not. Figures 26A-D show that BB9 elicits myeloid activation as indicated by CD14, CD20, CD86, and HLA-DR while the control does not. Figures 27A-D show that BB10 elicits myeloid activation as indicated by CD14, CD20, CD86, and HLA-DR while the control does not.

### Example 17. Comparison of BB5 to Comparative Conjugate IRM1 and Comparative Conjugate IRM2

This example shows that immunoconjugates produced by the embodiments of the invention are superior to the immunoconjugates produced by the '528 synthesis methods. BB5 was synthesized according to Scheme 11. Comparative Conjugates IRM1 and IRM2 were prepared using the adjuvants described in the '528 patent as adjuvants IRM1 and IRM2. Specifically, IRM1 and IRM2 were conjugated to an IgG antibody (specifically, the anti-CD20 antibody rituximab) with an amide linker.

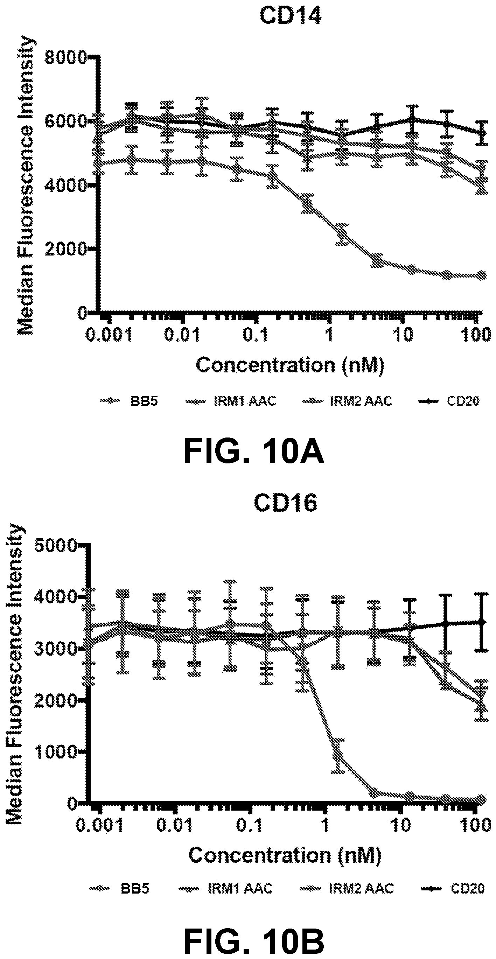

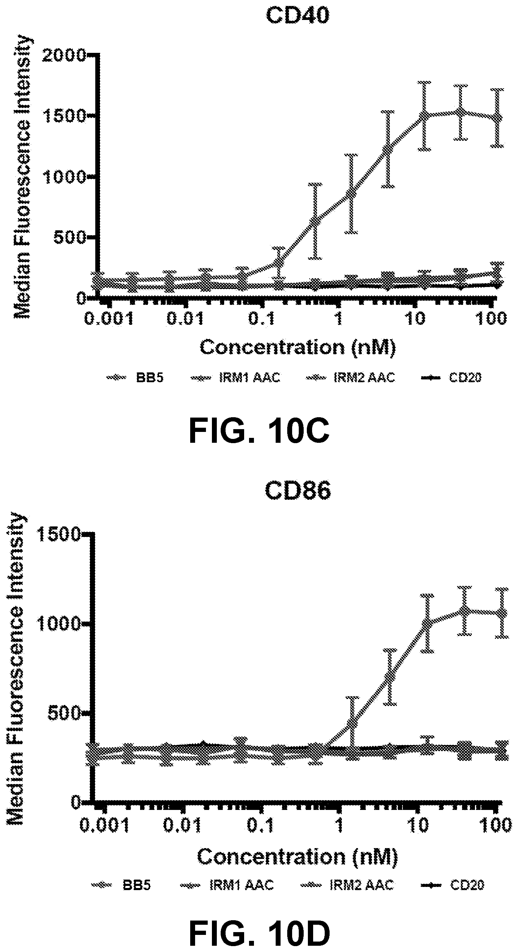

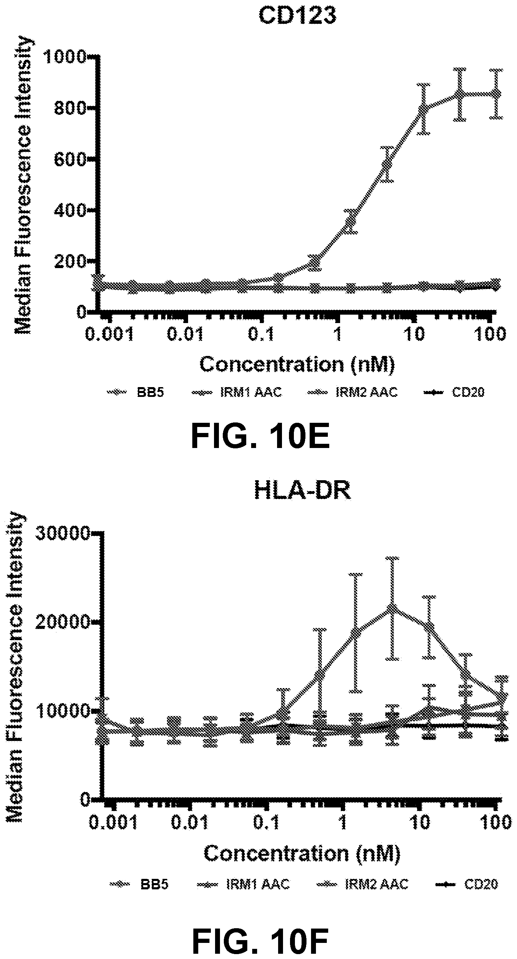

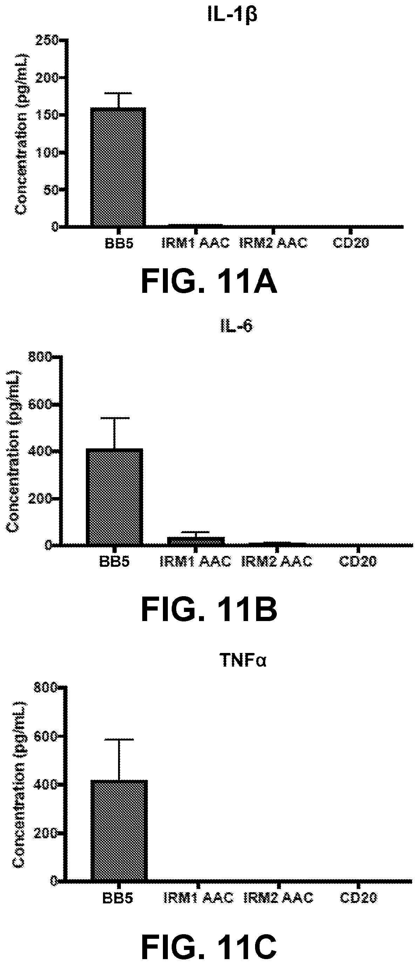

BB5 and Comparative Conjugates IRM1 and IRM2 were analyzed using the assay of Example 4. The results are shown in Figures 10A-10F and 11A-11C. Specifically, Figures 10A-10F show that BB5 prepared according to Scheme 11 elicits myeloid activation while Comparative Conjugates IRM1 and IRM2, and the control, unconjugated CD20 antibody, do not. Further, Figures 11A-11C show that BB5 prepared according to Scheme 11 elicits cytokine secretion while Comparative Conjugates IRM1 and IRM2, and the control, unconjugated CD20 antibody, do not.

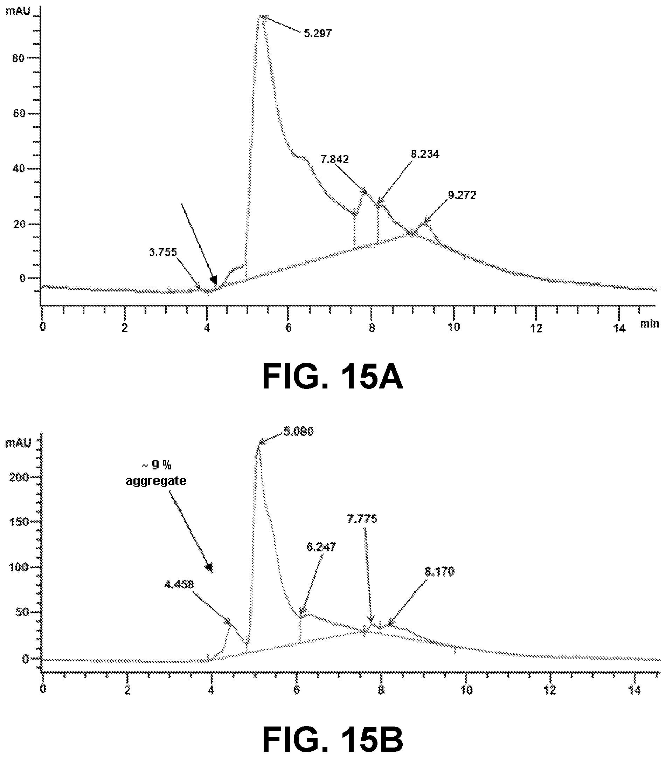

The Comparative Conjugates IRM1 and IRM2 had excessive aggregation as determined by LC/MS. Figures 15A-C shows the results of size exclusion chromatography following filtration with a 0.2µM filter. Comparative Conjugate IRM1 had 4 % aggregation and indicated by the first peak at 4.5 min (see Figure 15A). Comparative Conjugate IRM2 had 9.5 % aggregation and indicated by the first peak at 4.5 min (see Figure 15B). In contrast, BB5 had a small amount of aggregation (see Figure 15C). This difference is due in part to the thiolated intermediate that IRM1 and IRM2 have which is not present or necessary in the methods of the invention.

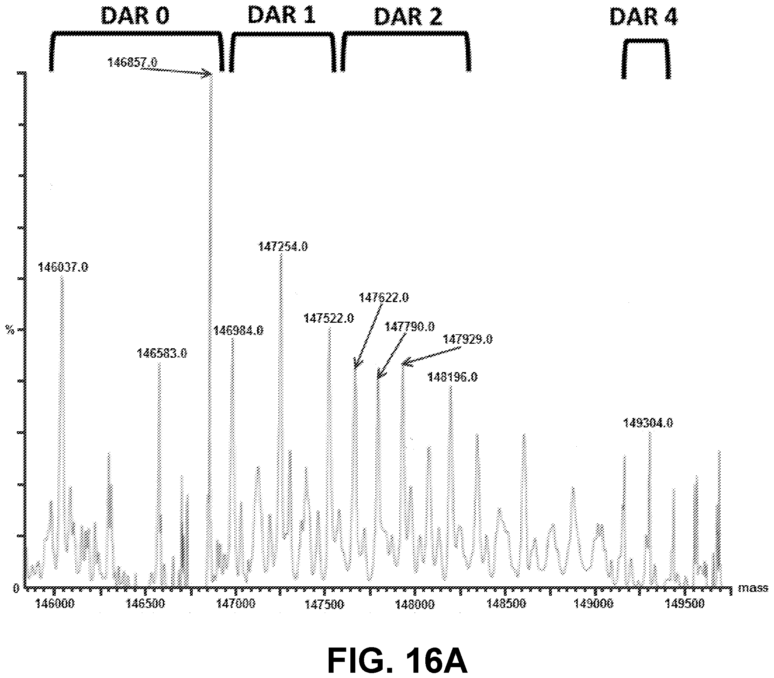

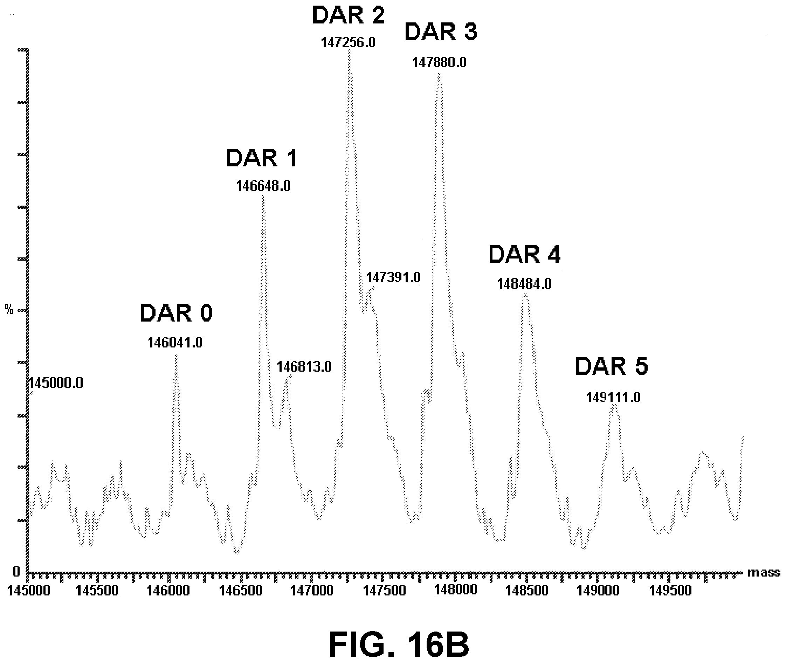

Figures 16A and 16B further illustrate the advantages of the methods of the invention. Compare Figure 16A, which shows Comparative Conjugate IRM1 following overnight deglycosylation with PNGase F and analyzed via LC/MS, to Figure 16B which shows BB5 following the same treatment.

BB5 and Comparative Conjugates IRM1 and IRM2 were also tested for storage stability. After synthesis, the conjugates were stored in 15 mL conical tubes for several hours. After storage, the tube containing the Comparative Conjugate IRM2 had a large white solid aggregate at the bottom of the tube. The tubes containing BB5 and Comparative Conjugate IRM1 contained clear fluid only and did not have any sediment.

### Example 18. Additional Immunoconjugates

The following immunoconjugates were prepared according to the methods of the invention. The immunoconjugates were tested in accordance with Example 16 and all were found to elicit myeloid activation. 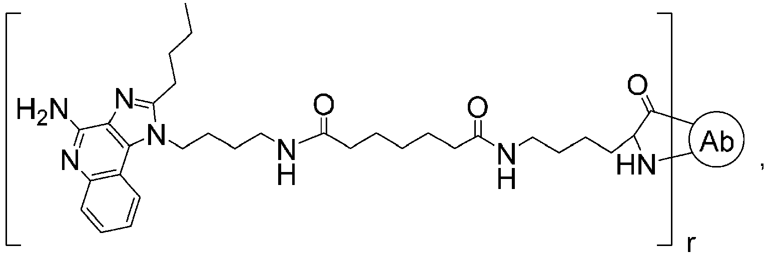 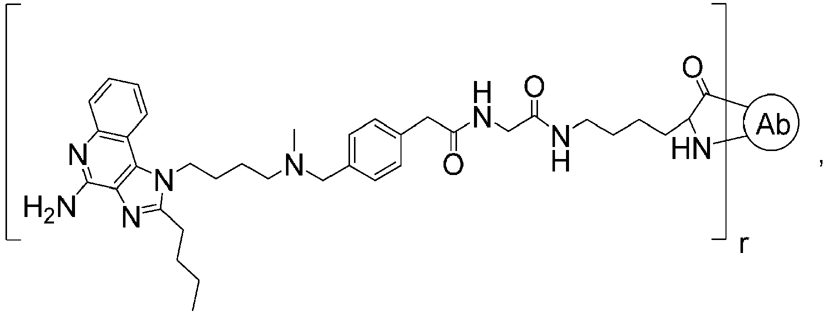 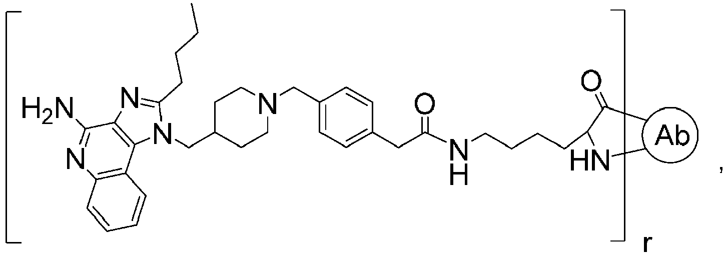 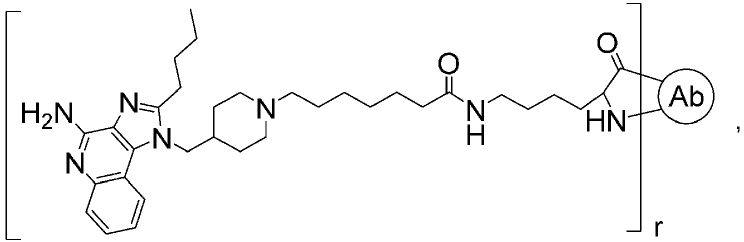 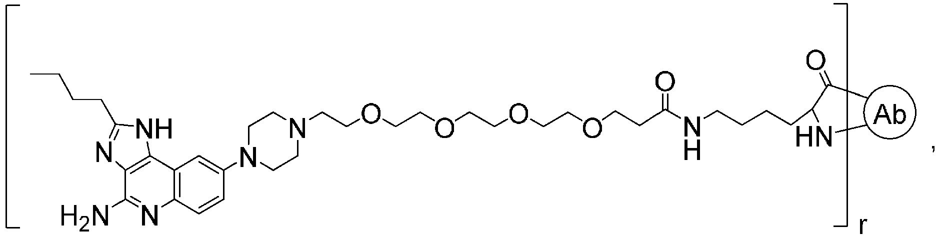 or

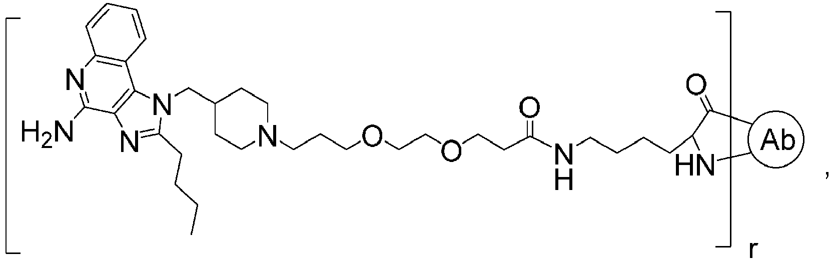

### Example 19. Synthesis of Immunoconjugate BB11 at pH 8.3

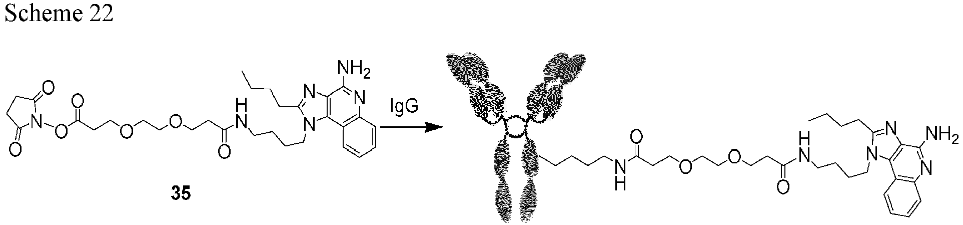

This example provides guidance on synthesis of an immunoconjugate with an aqueous solution buffered at a pH of 8.3. An IgG antibody (specifically, the anti-Her2 antibody trastuzumab) (10 mg/mL in PBS) was buffer exchanged into borate buffered saline ("BBS") pH 8.3 (50 mM boric acid pH 8.3, 125 mM NaCl) to provide the IgG antibody (specifically, the anti-Her2 antibody trastuzumab) as a 10 mg/mL in BBS.

Compound 27 (50 mg) was dissolved in dichloromethane/dimethylformamide (5 mL, 1:1) and 2 equivalents of *N*-hydroxysuccinimide ("NHS") was added followed by 1.5 equivalents of EDCI. The reaction was stirred overnight at ambient temperature and the reaction was concentrated to dryness. The crude ester 35 was purified via flash chromatography on a 4-gram silica gel column eluted with 0-10% isopropanol over 10 minutes. Pure fractions were concentrated, and the residue was lyophilized from 30% acetonitrile in water to provide 41 mg of purified ester 35. The molecular weight and purity were confirmed by LC/MS.
*Conjugation to antibody:* The ester 35 was dissolved in dry DMSO to make a 20 mM stock solution and 4, 8, or 12 molar equivalents (relative to the antibody) was added to 20 mL of an IgG antibody (the anti-HER2 antibody trastuzumab) (10 mg/mL in BBS). The conjugation reaction was incubated at 4 °C or 30 °C overnight (about 6 to about 15 hours). The resulting immunoconjugate BB11 was buffer exchanged into PBS (pH 7.2) using a Sephadex-G25 column to remove excess small molecular weight impurities. The final concentration was determined by measuring the absorbance at 280 nm on a NANODROP^{™} 1000 spectrophotometer. Minimal aggregate (less than 1%) was detected by SEC analysis and a DAR was determined via LC/MS analysis. The purified immunoconjugate was filtered through a 2 µM sterile filter and stored at 4 °C.

### Example 20. pH Dependent Synthesis of Immunoconjugates

This example demonstrates the conversion efficiency of immunoconjugates synthesized at a pH of 6.5 (citrate buffered saline "CBS"), 7.4 (phosphate buffered saline "PBS"), and 8.3 (borate buffered saline "BBS"). Immunoconjugates were prepared according to the procedure set forth in Example 19, using 8 and 12 molar equivalents (relative to the antibody) of the TFP or NHS ester, CBS, PBS, and BBS as the buffer for the antibody, and at a temperature of 4 °C or 30 °C.

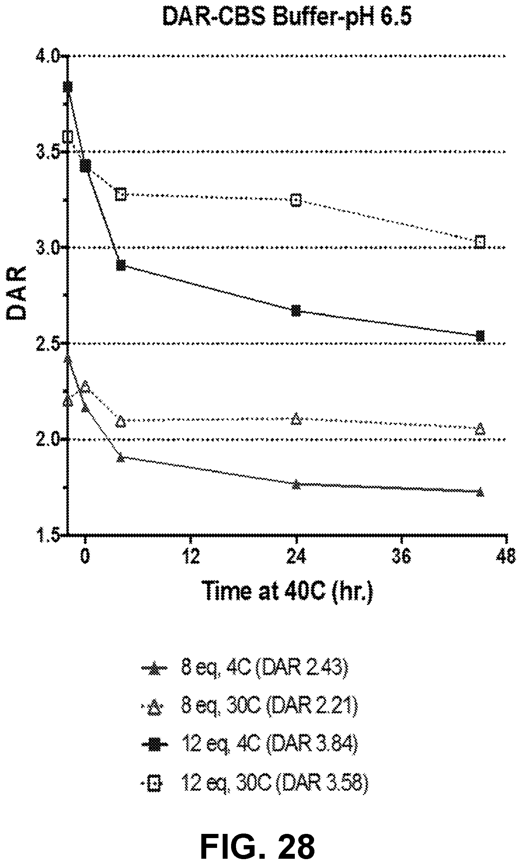

The resulting immunoconjugates were buffer exchanged into PBS (pH 7.2) using a Sephadex-G25 column to remove excess small molecular weight impurities, and the DAR was measured at 40 °C, as a function of time. The results for the CBS, PBS, and BBS buffers are set forth in FIG. 28, FIG. 29, and FIG. 30, respectively.

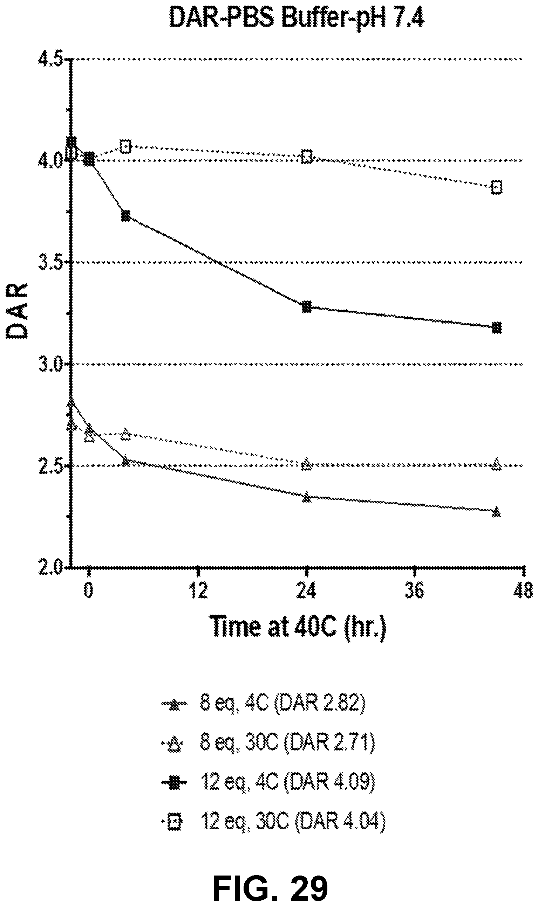

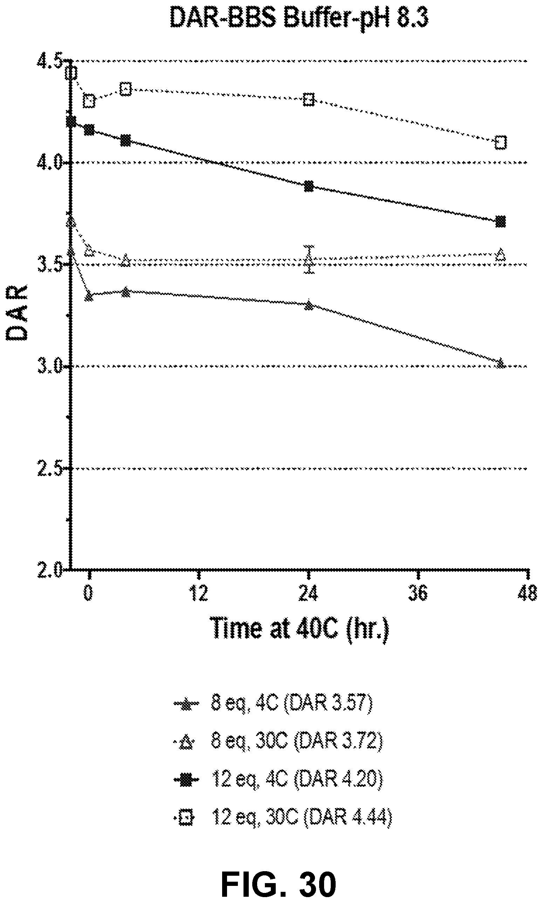

As demonstrated by FIGs. 28-30, the immunoconjugates synthesized using BBS (pH 8.3) resulted in higher DAR ratios at all temperatures and equivalents of the ester, as compared to immunoconjugates synthesized using CBS (pH 6.5) or PBS (pH 7.4). These results show that BBS buffer (pH 8.3) produces a higher conjugation efficiency to the antibody than CBS (pH 6.5) and PBS (pH 7.4). In addition, FIGs. 28-30 show that after incubation at 40 °C for 48 hours, the immunoconjugates synthesized using BBS (pH 8.3) maintained higher DAR ratios at all temperatures and equivalents of the ester, as compared to immunoconjugates synthesized using CBS (pH 6.5) or PBS (pH 7.4). These results show that BBS buffer (pH 8.3) produces a higher percentage of lysine conjugated adjuvants, as demonstrated by the higher DAR after 48 hours. It is believed that the tyrosine conjugated (i.e., hydrolysis labile conjugation) adjuvants are slowly hydrolyzed during incubation to produce a DAR corresponding only to the lysine conjugated immunoconjugates. FIG. 30 also demonstrates that conjugation using BBS (pH 8.3) resulted in at least 33% lysine conjugated immunoconjugates for all conditions except 8 equivalents of the ester and 4 °C, as demonstrated by the DAR after 48 hours of incubation at 40 °C. However, FIGs. 28 and 29 resulted in less than 33% lysine conjugated immunoconjugates for all conditions, as demonstrated by the DAR after 48 hours of incubation at 40 °C. In addition, FIG. 30 shows that conjugation at 30 °C is more efficient than conjugation 4 °C, as demonstrated by a higher DAR at all time points.

### Example 21. pH Dependent Synthesis of Immunoconjugates

This example demonstrates the stability of immunoconjugates synthesized at a pH of 6.5 (citrate buffered saline "CBS"), 7.4 (phosphate buffered saline "PBS"), and 8.3 (borate buffered saline "BBS"). Immunoconjugates were prepared according to the procedure set forth in Example 19, using 4, 8, and 12 molar equivalents (relative to the antibody) of the TFP or NHS ester, CBS, PBS, and BBS as the buffer for the antibody, and at a temperature of 4 °C or 30 °C.

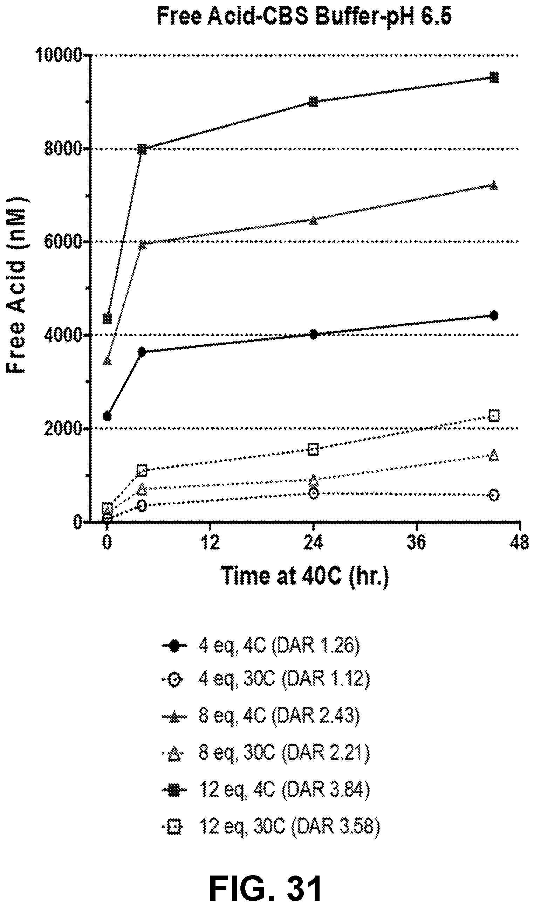

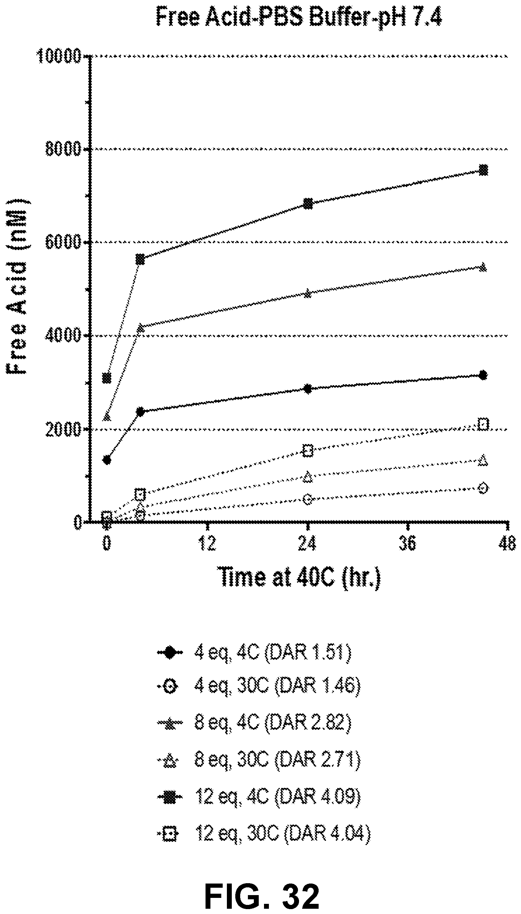

The resulting immunoconjugates were buffer exchanged into PBS (pH 7.2) using a Sephadex-G25 column to remove excess small molecular weight impurities, and the free acid (formed from the hydrolysis of the linker/adjuvant from the antibody) concentration was measured at 40 °C, as a function of time. The results for the CBS, PBS, and BBS buffers are set forth in FIG. 31, FIG. 32, and FIG. 33, respectively.

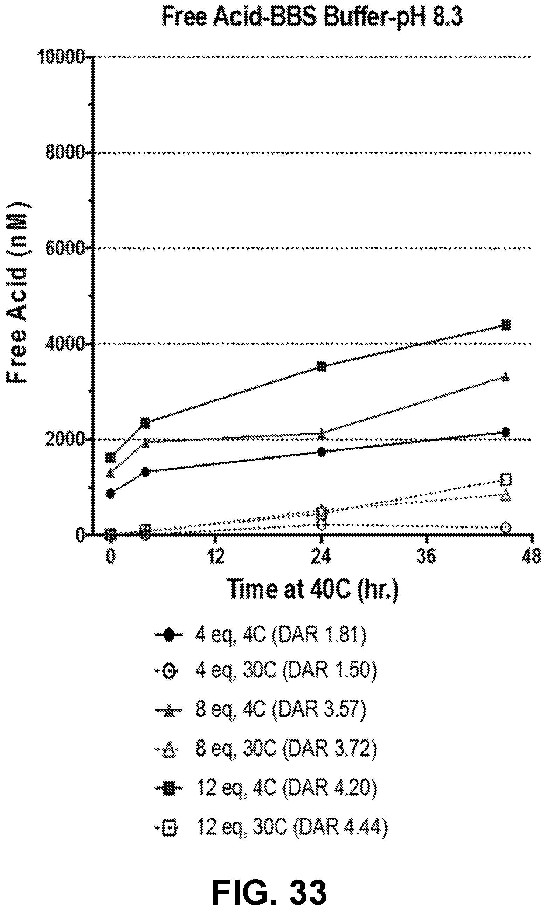

As demonstrated by FIGs. 31-33, the immunoconjugates synthesized using BBS (pH 8.3) resulted in free acid concentrations at all temperatures and equivalents of the ester, as compared to immunoconjugates synthesized using CBS (pH 6.5) or PBS (pH 7.4). These results show that BBS buffer (pH 8.3) produces less tyrosine conjugated (i.e., hydrolysis labile conjugation) adjuvants than CBS (pH 6.5) and PBS (pH 7.4). In addition, FIG. 33 shows that conjugation at 30 °C is more efficient at forming lysine conjugate immunoconjugates than conjugation 4 °C, as demonstrated by a higher DAR at all time points.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A method for producing an immunoconjugate, the method comprising combining one or more compounds of Formula I: 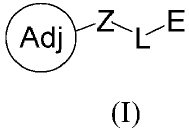 and an antibody of Formula II: 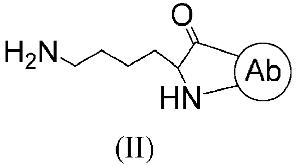 wherein Formula II is an antibody with residue 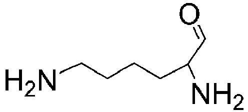 representing one or more lysine residues of the antibody,
in an aqueous solution buffered at a pH of 7.5 to 9 until at least 33 mol% of the one or more compounds of Formula I is conjugated to the antibody of Formula II to provide the immunoconjugate of Formula III: 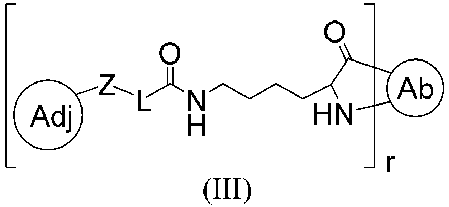 wherein
Adj is an adjuvant,
Z is -CH₂-, -C(O)NH-, -C(O)O-, or -C(O)-,
L is a linker,
E is an ester, and
r is the average number of adjuvants attached to the antibody and is a positive number up to 8,
in a first buffered aqueous solution.

2. The method of claim 1, wherein the aqueous solution is buffered at a pH of 8 to 9, preferably the aqueous solution is buffered at a pH of 8 to 8.6, or more preferably the aqueous solution is buffered at a pH of 8 to 8.3.

3. The method of claim 1 or 2, wherein the aqueous solution is buffered with borate buffered saline.

4. The method of any one of claims 1-3, wherein the aqueous solution is at a temperature of 0 °C to 50 °C, preferably the aqueous solution is at a temperature of 25 °C to 35 °C, or more preferably the aqueous solution is at a temperature of 30 °C.

5. The method of any one of claims 1-4, wherein the method further comprises performing a buffer exchange on the first buffered aqueous solution of the immunoconjugate of Formula III to provide a second buffered aqueous solution buffered at a pH of 6 to 7.5, preferably the second buffered aqueous solution is buffered at a pH of 7 to 7.5, or more preferably the second buffered aqueous solution is buffered at a pH of 7.2 to 7.4.

6. The method of claim 5, wherein the second buffered aqueous solution is buffered with phosphate buffered saline.

7. A method of any one of claims 1-6, wherein the ester is an N-hydroxysuccinimide (NHS) ester of the formula: 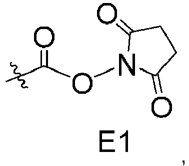 wherein the wavy line (" 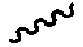 ") represents the point of attachment to the linker ("L").

8. The method of any one of claims 1-6, wherein the ester is a sulfo-N-hydroxysuccinimide ester of the formula: 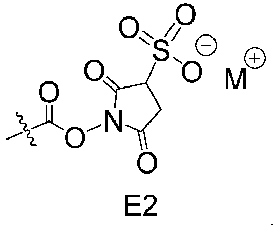 wherein M is any cation and the wavy line (" 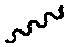 ") represents the point of attachment to the linker ("L").

9. The method of any one of claims 1-6, wherein the ester is a phenol ester of the formula: 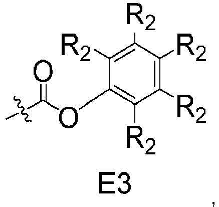 wherein each R₂ is independently selected from hydrogen, iodine, bromine, chlorine, or fluorine and the wavy line (" 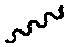 ") represents the point of attachment to the linker ("L").

10. The method of claim 9, wherein the ester is a phenol ester of the formula: 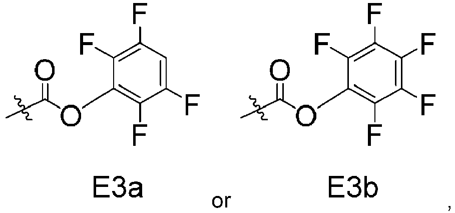 wherein the wavy line (" 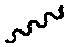 ") represents the point of attachment to the linker ("L").

11. The method of any one of claims 1-10, wherein the adjuvant is a TLR agonist, preferably selected from the group consisting of a TLR2 agonist, a TLR3 agonist, a TLR4 agonist, a TLR7 agonist, a TLR8 agonist, a TLR7/TLR8 agonist, and a TLR9 agonist, or more preferably selected from the group consisting of a TLR7 agonist, a TLR8 agonist, and a TLR7/TLR8 agonist.

12. The method of any one of claims 1-11, wherein the antibody binds to an antigen of a cancer cell, preferably the antibody is a monoclonal antibody, or more preferably the antibody is selected from the group consisting of an anti-HER2 antibody, an anti-PDL1 antibody, an anti-CEA antibody, and an anti-EGFR antibody.

13. The method of any one of claims 1-12, comprising combining the one or more compounds of Formula I and the antibody of Formula II in the aqueous solution until at least 40 mol% or preferably until at least 50 mol% of the one or more compounds of Formula I is conjugated to the antibody of Formula II to provide the immunoconjugate of Formula III.

14. The method of any one of claims 1-13, wherein the method results in more than a 5% reduction, preferably more than a 15% reduction, or more preferably more than a 25% reduction of detectable impurities in the immunoconjugate of Formula III in the first buffered aqueous solution relative to an immunoconjugate of Formula III in a first buffered aqueous solution prepared by combining the one or more compounds of Formula I and the antibody of Formula II in an aqueous solution buffered at a pH of less than 7.5 using phosphate buffered saline, wherein all reaction conditions are identical except for the buffer.

## Patentansprüche

1. Ein Verfahren zur Herstellung eines Immunkonjugats, das Verfahren umfassend das Kombinieren einer oder mehrerer Verbindungen der Formel I: 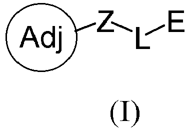 und einen Antikörper der Formel II: 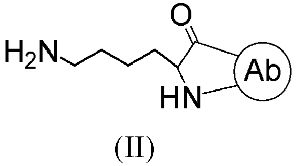 wobei Formel II ein Antikörper ist, dessen Rest 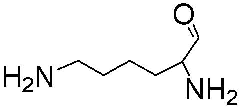 einen oder mehrere Lysinreste des Antikörpers darstellt,
in einer bei einem pH-Wert von 7,5 bis 9 gepufferten wässrigen Lösung, bis mindestens 33 Mol-% der einen oder mehreren Verbindungen der Formel I mit dem Antikörper der Formel II konjugiert sind, um das Immunkonjugat der Formel III zu erhalten: 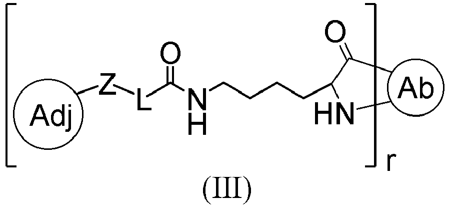 wobei
Adj ist ein Adjuvans,
Z ist -CH₂-, -C(O)NH-, -C(O)O-, oder -C(O)-,
L ist ein Linker,
E ist ein Ester, und
r ist die durchschnittliche Anzahl der an den Antikörper gebundenen Adjuvantien und ist eine positive Zahl bis zu 8,
in einer ersten gepufferten wässrigen Lösung.

2. Das Verfahren nach Anspruch 1, wobei die wässrige Lösung bei einem pH-Wert von 8 bis 9, vorzugsweise bei einem pH-Wert von 8 bis 8,6, oder noch bevorzugter bei einem pH-Wert von 8 bis 8,3, gepuffert ist.

3. Das Verfahren nach Anspruch 1 oder 2, wobei die wässrige Lösung mit Borat gepufferter Salzlösung gepuffert ist.

4. Das Verfahren nach einem der Ansprüche 1 bis 3, wobei die wässrige Lösung bei einer Temperatur von 0 °C bis 50 °C, vorzugsweise bei einer Temperatur von 25 °C bis 35 °C oder noch bevorzugter bei einer Temperatur von 30 °C, vorliegt.

5. Das Verfahren nach einem der Ansprüche 1 bis 4, wobei das Verfahren ferner die Durchführung eines Pufferaustauschs mit der ersten gepufferten wässrigen Lösung des Immunkonjugats der Formel III umfasst, um eine zweite gepufferte wässrige Lösung bereitzustellen, die bei einem pH-Wert von 6 bis 7,5 gepuffert ist, wobei die zweite gepufferte wässrige Lösung vorzugsweise bei einem pH-Wert von 7 bis 7,5 gepuffert ist, oder noch bevorzugter die zweite gepufferte wässrige Lösung bei einem pH-Wert von 7,2 bis 7,4 gepuffert ist.

6. Das Verfahren nach Anspruch 5, wobei die zweite gepufferte wässrige Lösung mit Phosphat gepufferter Salzlösung gepuffert ist.

7. Ein Verfahren nach einem der Ansprüche 1 bis 6, wobei der Ester ein N-Hydroxysuccinimid-(NHS)-Ester der Formel ist: 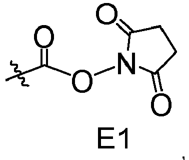 wobei die Wellenlinie (" 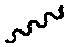 ") den Punkt der Bindung an den Linker ("L") darstellt.

8. Das Verfahren nach einem der Ansprüche 1 bis 6, wobei der Ester ein Sulfo-N-hydroxysuccinimid-Ester der Formel ist: 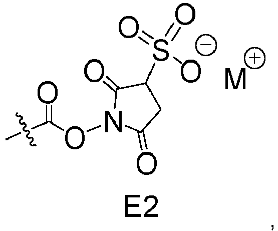 wobei M ein beliebiges Kation ist und die Wellenlinie (" 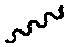 ") den Punkt der Bindung an den Linker ("L") darstellt.

9. Das Verfahren nach einem der Ansprüche 1 bis 6, wobei der Ester ein Phenolester der Formel ist: 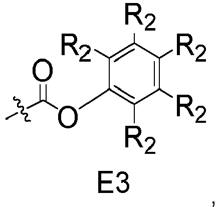 wobei jedes R₂ unabhängig voneinander aus Wasserstoff, Jod, Brom, Chlor oder Fluor ausgewählt ist und die Wellenlinie (" 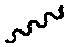 ") den Punkt der Bindung an den Linker ("L") darstellt.

10. Das Verfahren nach Anspruch 9, wobei der Ester ein Phenolester der Formel ist: 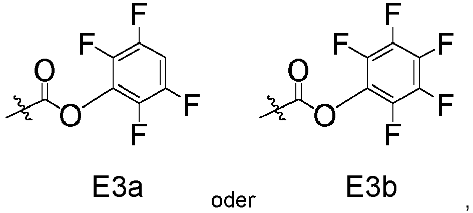 wobei die Wellenlinie (" 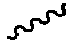 ") den Punkt der Bindung an den Linker ("L") darstellt.

11. Das Verfahren nach einem der Ansprüche 1 bis 10, wobei das Adjuvans ein TLR-Agonist ist, vorzugsweise ausgewählt aus der Gruppe bestehend aus einem TLR2-Agonisten, einem TLR3-Agonisten, einem TLR4-Agonisten, einem TLR7-Agonisten, einem TLR8-Agonisten, einem TLR7/TLR8-Agonisten und einem TLR9-Agonisten, oder noch bevorzugter ausgewählt aus der Gruppe bestehend aus einem TLR7-Agonisten, einem TLR8-Agonisten und einem TLR7/TLR8-Agonisten.

12. Das Verfahren nach einem der Ansprüche 1 bis 11, wobei der Antikörper an ein Antigen einer Krebszelle bindet, vorzugsweise ist der Antikörper ein monoklonaler Antikörper, oder noch bevorzugter ist der Antikörper ausgewählt aus der Gruppe bestehend aus einem Anti-HER2-Antikörper, einem Anti-PDL1-Antikörper, einem Anti-CEA-Antikörper und einem Anti-EG FR-Antikörper.

13. Das Verfahren nach einem der Ansprüche 1 bis 12, umfassend das Kombinieren der einen oder mehreren Verbindungen der Formel I und des Antikörpers der Formel II in der wässrigen Lösung, bis mindestens 40 Mol-% oder vorzugsweise bis mindestens 50 Mol-% der einen oder mehreren Verbindungen der Formel I mit dem Antikörper der Formel II konjugiert sind, um das Immunkonjugat der Formel III bereitzustellen.

14. Das Verfahren nach einem der Ansprüche 1 bis 13, wobei das Verfahren zu einer Verringerung von mehr als 5 %, vorzugsweise von mehr als 15 % oder besonders bevorzugt von mehr als 25 %, der nachweisbaren Verunreinigungen im Immunkonjugat der Formel III in der ersten gepufferten wässrigen Lösung führt im Vergleich zu einem Immunkonjugat der Formel III in einer ersten gepufferten wässrigen Lösung, die durch Kombinieren der einen oder mehreren Verbindungen der Formel I und des Antikörpers der Formel II in einer wässrigen Lösung hergestellt wird, die unter Verwendung von Phosphat gepufferter Salzlösung bei einem pH-Wert von weniger als 7,5 gepuffert ist, wobei alle Reaktionsbedingungen bis auf den Puffer identisch sind.

## Revendications

1. Procédé de production d'un immunoconjugué, le procédé comprenant la combinaison d'un ou de plusieurs composés de Formule I : 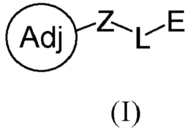 et d'un anticorps de Formule II : 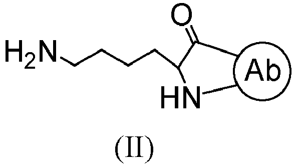 sachant que la Formule II est un anticorps avec un résidu 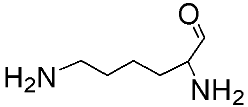 représentant un ou plusieurs résidus de lysine de l'anticorps,
dans une solution aqueuse tamponnée à un pH de 7,5 à 9 jusqu'à ce qu'au moins 33 %mol de l'un ou des plusieurs composés de Formule I soient conjugués à l'anticorps de Formule Il pour fournir l'immunoconjugué de Formule III : 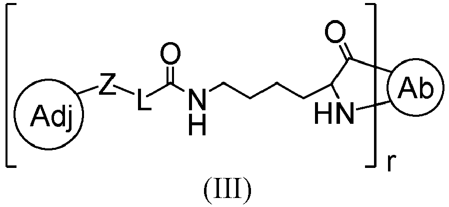 sachant que
Adj est un adjuvant,
Z is -CH₂-, -C(O)NH-, -C(O)O-, or -C(O)-,
L est un lieur,
E est un ester, et
r est le nombre moyen d'adjuvants attachés à l'anticorps et est un nombre positif allant jusqu'à 8,
dans une première solution aqueuse tamponnée.

2. Le procédé de la revendication 1, sachant que la solution aqueuse est tamponnée à un pH de 8 à 9, de préférence la solution aqueuse est tamponnée à un pH de 8 à 8,6, ou de manière plus préférentielle la solution aqueuse est tamponnée à un pH de 8 à 8,3.

3. Le procédé de la revendication 1 ou 2, sachant que la solution aqueuse est tamponnée avec de la saline tamponnée au borate.

4. Le procédé de l'une quelconque des revendications 1 à 3, sachant que la solution aqueuse est à une température de 0 °C à 50 °C, de préférence la solution aqueuse est à une température de 25 °C à 35 °C, ou de manière plus préférentielle la solution aqueuse est à une température de 30 °C.

5. Le procédé de l'une quelconque des revendications 1 à 4, sachant que le procédé comprend en outre l'exécution d'un échange de tampon sur la première solution aqueuse tamponnée de l'immunoconjugué de Formule III pour fournir une deuxième solution aqueuse tamponnée qui est tamponnée à un pH de 7 à 7,5, de préférence la deuxième solution aqueuse tamponnée est tamponnée à un pH de 7 à 7,5, ou de manière plus préférentielle la deuxième solution aqueuse tamponnée est tamponnée à un pH de 7,2 à 7,4.

6. Le procédé de la revendication 5, sachant que la deuxième solution aqueuse tamponnée est tamponnée avec de la saline tamponnée au phosphate.

7. Procédé de l'une quelconque des revendications 1 à 6, sachant que l'ester est un ester de N-hydroxysuccinimide (NHS) de la formule : 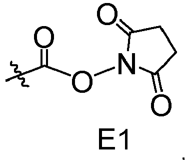 sachant que la ligne ondulée (« 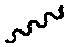 ») représente le point d'attache au lieur (« L »).

8. Le procédé de l'une quelconque des revendications 1 à 6, sachant que l'ester est un ester de sulfo-N-hydroxysuccinimide ester de la formule : 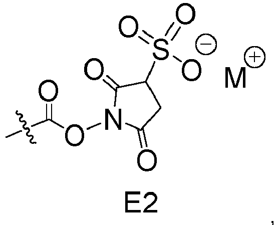 sachant que M est n'importe quel cation et que la ligne ondulée (« 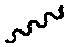 ») représente le point d'attache au lieur (« L »).

9. Le procédé de l'une quelconque des revendications 1 à 6, sachant que l'ester est un ester de phénol de la formule : 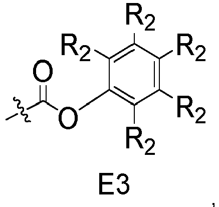 sachant que chaque R₂ est sélectionné indépendamment parmi l'hydrogène, l'iodine, la bromine, la chlorine, ou la fluorine et la ligne ondulée (« 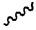 ») représente le point d'attache au lieur (« L »).

10. Le procédé de la revendication 9, sachant que l'ester est un ester de phénol de la formule : 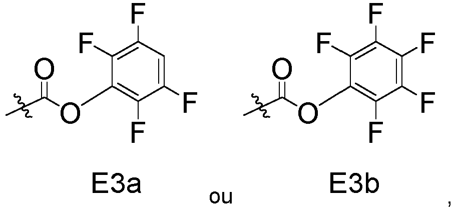 sachant que la ligne ondulée (« 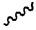 ») représente le point d'attache au lieur (« L »).

11. Le procédé de l'une quelconque des revendications 1 à 10, sachant que l'adjuvant est un agoniste de TLR, de préférence sélectionné dans le groupe constitué par un agoniste de TLR2, un agoniste de TLR3, un agoniste de TLR4, un agoniste de TLR7, un agoniste de TLR8, un agoniste de TLR7/TLR8, et un agoniste de TLR9, et de manière plus préférentielle sélectionné dans le groupe constitué par un agoniste de TLR7, un agoniste de TLR8, et un agoniste de TLR7/TLR8.

12. Le procédé de l'une quelconque des revendications 1 à 11, sachant que l'anticorps se lie à un antigène d'une cellule cancéreuse, de préférence l'anticorps est un anticorps monoclonal, ou de manière plus préférentielle l'anticorps est sélectionné dans le groupe constitué par un anticorps anti-HER2, un anticorps anti-PDL1, un anticorps anti-CEA, et un anticorps anti-EGFR.

13. Le procédé de l'une quelconque des revendications 1 à 12, comprenant la combinaison de l'un ou des plusieurs composés de Formule I et de l'anticorps de Formule II dans la solution aqueuse jusqu'à ce qu'au moins 40 %mol ou de préférence jusqu'à ce qu'au moins 50 %mol de l'un ou des plusieurs composés de Formule I soient conjugués à l'anticorps de Formule II pour fournir l'immunoconjugué de Formule III.

14. Le procédé de l'une quelconque des revendications 1 à 13, sachant que le procédé aboutit à une réduction de plus de 5 %, de préférence à une réduction de plus de 15 %, ou de manière plus préférentielle à une réduction de plus de 25 % d'impuretés détectables dans l'immunoconjugué de Formule III dans la première solution aqueuse tamponnée par rapport à un immunoconjugué de Formule III dans une première solution aqueuse tamponnée préparée en combinant l'un ou les plusieurs composés de Formule I et l'anticorps de Formule II dans une solution aqueuse tamponnée à un pH de moins de 7,5 moyennant de la saline tamponnée au phosphate, sachant que toutes les conditions de réaction sont identiques à l'exception du tampon.
